# EUROPEAN PATENT SPECIFICATION

(11) **EP 2 314 581 B1**
(45) Date of publication and mention of the grant of the patent: **25.07.2012**
(21) Application number: 09173953.2
(22) Date of filing: 23.10.2009
(51) Int. Cl.: C07D 333/56

(54) **A process for preparing benzo[b]thiophene derivatives**
Verfahren zur Herstellung von Benzo[b]thiophenderivaten
Procédé de préparation de dérivés de benzo[b]thiophène

(43) Date of publication of application: 27.04.2011
(73) Proprietor: Hexal AG, 83607 Holzkirchen (DE)
(72) Inventor: Schickaneder, Christian, 91207 Lauf a. d. Pegnitz (DE); Schäfer, Jürgen, 01445 Radebeul (DE)
(74) Representative: Prüfer & Partner GbR European Patent Attorneys

(56) References cited:
- US-A- 5 731 327
- US-A- 6 162 930
- US-B1- 6 756 388
- PETER G. M. WUTS, THEODORA W. GREENE: "Greene's Protective Groups in Organic Synthesis (Fourth Edition)" 2006, JOHN WILEY & SONS, INC. , USA , XP002557093 Protection for Phenols and Catechols, p. 367-382

## Description

### Field of the invention

The present invention relates in general to the field of organic chemistry, and in particular to the preparation of benzo[b]thiophene derivatives. These benzo[b]thiophene derivatives can be used as intermediates in the synthesis of pharmaceutically active agents such as raloxifene or derivatives thereof.

### Background of the invention

Compounds comprising a benzo[b]thiophene moiety are important intermediates for the preparation of active pharmaceutical active agents. For example, raloxifene (6-hydroxy-2-(4-hydroxyphenyl)-3-[4-(2-piperidinoethoxybenzoyl]-benzo[b]thiophene) is a selective estrogen receptor modulator possessing estrogen agonist-like actions on bone tissues and serum lipids, while displaying efficient estrogen antagonist properties in breast and uterus.

US 4,133,814 and US 4,358,593 disclose processes for preparing benzo[b]thiophene derivatives wherein an amine function comprising moiety is introduced at the 3-position of the thiophene ring by Friedel-Crafts acylation.

WO 98/48792 discloses a process for preparing substituted benzo[b]thiophenes, wherein 2-(4-hydroxyphenyl)-3-(4-hydroxybenzoyl)-6-hydroxybenzo[b]thiophene as key intermediate is O-alkylated at its 3-(4-hydroxybenzoyl)-substituent.

US 2002/0173645 A1 and US 5,731,327 disclose processes for preparing crystalline solvate forms of hydrochlorid salts of raloxifene, in particular a 1,2-dichloroethane solvate, a methylene chloride solvate, a chlorobenzene solvate, a 1,2,3-trichloropropane solvate and a chloroform solvate.

US 6,162,930 A and US 6,756,388 B1 respectively disclose a process for preparing benzo[b]thiophene compounds wherein Friedel-Crafts acylation is carried out in the presence of hyperstoichiometric amounts of Lewis acid catalyst.

T. W. Greene et. al., "Greene's protective groups in organic synthesis", fourth edition, 2006, John Wiley & Sons, Inc., cites reaction conditions for selectively removing a methoxy group located at an aryl group in the presence of an ethoxy group located at an aryl group and vice versa.

The object of the present invention is to provide an improved process for preparing benzo[b]thiophene derivatives representing valuable key intermediates for the preparation of pharmaceutically active agents such as raloxifene or derivatives thereof.

### Summary of the invention

Various aspects, advantageous features and preferred embodiments of the present invention as summarized in the following items, respectively alone or in combination, contribute to solving the object of the invention:
(1) A process for preparing a compound of formula VII' 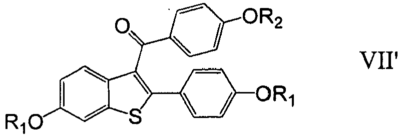 wherein R₁ and R₂ represent different hydroxy protecting groups which are independently from each other selectively removable, with the proviso that both -OR₁ an d -OR₂ are substantially not cleavable in the presence of a Lewis acid at Friedel-Crafts acylation conditions,
wherein R₁ is selected from the group consisting of linear or branched alkyl, linear or branched alkylaryl and linear or branched alkenyl; preferably R₁ is linear or branched alkyl; and R₂ is selected from the group consisting of linear or branched alkylsulfonyl, arylsulfonyl and linear or branched alkylarylsulfonyl ; preferably R₂ is arylsulfonyl, is prepared from a compound of formula VI' 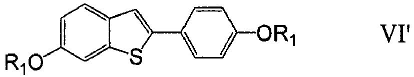 which is reacted with a compound of formula V' 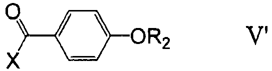 wherein R₁ and R₂ are defined as above, and
X represents Cl, Br, I or 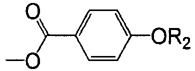 wherein R₂ is defined as above,
in the presence of a Lewis acid and a solvent.
The term "hydroxy protecting group" as used herein means any group known in the art which can be used for protecting a hydroxy group, with the proviso that the cleavage conditions for selectively removing said hydroxy protecting group will not adversely affect the structure of compounds of formula VII, VII', VIII or X.
The term "alkyl" as used herein means straight, branched or cyclic hydrocarbons of 1 to 12 carbon atoms, preferably 1 to 8 carbon atoms and more preferably 1 to 6 carbon atoms.
The term "aryl" as used herein means hydrocarbon aryls, preferably single or condensed six-membered rings, more preferably phenyl or naphthyl, in particular phenyl.
The term "substituted" as employed herein means that the aforementioned alkyl, aryl and alkylaryl groups have one, two or three linear or branched C₁-C₆ alkyl substituents. The term "substantially not cleavable in the presence of a Lewis acid at Friedel-Crafts acylation conditions" as used herein means that there is no or substantially no deprotection of -OR₁ and -OR₂ at Friedel-Crafts acylation conditions. In a "Friedel-Crafts acylation condition" suitable for testing absence of cleavage, a Lewis acid, that is an electron acceptor, which acts as a catalyst, may be used for example in about 1 molar amount to about 2 molar amount relative to the compound to be acylated, wherein the temperature of the reaction mixture is typically within a range of about 0°C to ambient temperature.
The procedural concept according to this aspect of the invention provides an orthogonal protecting group strategy for the present process for acylation. The term "orthogonal protecting group strategy" as used herein means that two different protecting groups R₁ and R₂ are introduced in a compound, wherein said two different protecting groups can be independently from each other selectively deprotected. By suitably selecting the type of hydroxy protecting group R₁ and R₂ and/or the positions of -OR₁ and -OR₂, the present orthogonal protecting group strategy surprisingly provides for protecting groups which are not cleavable at Friedel-Crafts acylation conditions and/or conditions of subsequent reaction steps, while selective and complete removal of R₁ and R₂ can be carried out in subsequent reactions steps at mild conditions.
In particular, according to this preferred aspect of the invention, the starting materials compound of formula VI' and compound of formula V' are suitably selected in order to obtain highly desirable products of formula VII'. Compound of formula VII' represents a useful intermediate for preparing pharmaceutically active agents such as raloxifene or derivatives thereof, since the introduction of the two different protecting groups R₁ and R₂ provides for an advantageous selective derivatisation of compound of formula VII' in subsequent reaction steps.
Furthermore, by suitably selecting R₁ and R₂, the possibly different cleavability/bond strength of O-R₁ and O-R₂ for a certain protecting group at the 2- and 6-positions compared to the 3-position of the benzo[b]thiophene moiety can be taken into account.
(2) The process according to item (1), wherein one of the hydroxy protecting groups R₁ and R₂ is stable under basic conditions and cleavable under acidic conditions but substantially not cleavable in the presence of a Lewis acid at Friedel-Crafts acylation conditions, while the other hydroxy protecting group is cleavable under basic conditions and stable under acidic conditions; preferably, R₁ is stable under basic conditions and cleavable under acidic conditions but substantially not cleavable in the presence of a Lewis acid at Friedel-Crafts acylation conditions, and R₂ is cleavable under basic conditions and stable under acidic conditions.
The term "basic conditions" as used herein means conditions wherein Bronsted bases, that is proton acceptors, are applied and added.
The term "acidic conditions" as used herein means conditions wherein Brønsted acids, that is proton donators, or Lewis acids, that is electron acceptors, are applied and added.
Besides the type of acid or base used, the reaction conditions of said "basic conditions" and "acidic conditions" have to be suitably selected such that there is no undesired cleavage due to harsh reaction conditions like large excess of acid or base, high reaction temperatures and/or long reaction times. For Lewis acids for example, cleavage of the protecting group(s) due to the presence of Lewis acid is undesired in the acylation process according to item (1), while cleavage by a Lewis acid may be carried out under suitable reaction conditions in a reaction step subsequent to acylation.
This measure provides for an efficient and economical deprotection of R₁ and R₂ respectively, since Brønsted bases and acids as well as Lewis acids are readily available and economic reactants for cleavage.
(3) The process according to item (1) or (2), wherein R₁ is selected from the group consisting of methyl, ethyl, tert-butyl, benzyl, p-methoxybenzyl (PMB), trityl (Tr), allyl; preferably methyl, ethyl, tert-butyl, more preferably methyl;
and R₂ is selected from the group consisting of mesyl, tosyl and benzene sulfonyl; preferably R₂ is tosyl and benzene sulfonyl; more preferably R₂ is benzene sulfonyl.
(4) The process according to any one of the preceding items, wherein R₁ is methyl and R₂ is benzene sulfonyl.
(5) The process according to any one of the preceding items, wherein X is Cl.
(6) The process according to any one of the preceding items, wherein the Lewis acid is used in substoichiometric amounts relative to compound of formula VI and VI' respectively, preferably in about 0.004 to 0.07 times molar amounts, more preferably in about 0.008 to 0.05 times molar amounts, and in particular in an amount of 0.01 to 0.02 relative to compound of formula VI and VI' respectively.
According to this beneficial embodiment of the invention, the amount of Lewis acid as a catalyst of the Friedel-Crafts acylation can be significantly reduced compared to conventional Friedel-Crafts conditions, especially those conventionally applied to similar reactants, wherein an excess of Lewis acid relative to the compound to be acylated is used. Thereby, acylation of compound of formula VI or VI' can be carried out under mild reaction conditions, which in turn provides for a stable and reliably process, since cleavage of the OR₁ moieties is avoided owing to the significantly reduced amount of Friedel-Crafts catalyst. Thus, this embodiment provides for high yields and less require efforts for purifying the product, while said process is also advantageous from economical view due to the substantial savings of Lewis acid catalyst.
(7) The process according to any one of the preceding items, wherein the Lewis acid comprises divalent or trivalent metal, preferably divalent metal, more preferably divalent metal generated in situ by comproportionation of elemental metal and salt comprising trivalent metal.
In this way, the Lewis acid catalyst is suitably selected in view of the activity of the catalyst. That is, the above defined Lewis acids provide for both fast reaction rates and high conversion rates. By suitably selecting the valency of the metal comprised in the Lewis acid, activity of the Lewis acid catalyst can be improved. Furthermore, activity of the Lewis acid catalyst may be improved by generating the metal comprised in the catalyst in situ.
(8) The process according to item (7), wherein the Lewis acid is FeCl₃, FeCl₂, FeBr₃, FeBr₂, FeSO₄, Fe₂(SO₄)₃, ZinCl₂, ZnBr₂, ZnSO₄; preferably FeCl₂, FeSO₄, ZnCl₂; more preferably FeSO₄, ZnCl₂; even more preferably FeSO₄ generated in situ by comproportionation of elemental Fe and Fe₂(SO₄)₃, and in particular FeSO₄ generated in situ by comproportionation of elemental Fe and Fe₂(SO₄)₃·5H₂O.
According to this embodiment of the invention, both metal cation and counter anion are suitably selected with the proviso that the Lewis acid catalyst has an improved activity.
(9) The process according to any one of items (1) to (8), wherein chlorobenzene, dichlorobenzene or toluene is used as the solvent, preferably chlorobenzene or toluene, more preferably toluene.
This embodiment of the invention provides for improvement of environmental friendliness of the process, while there is also an improvement in view of the working conditions. Under conventional Friedel-Crafts acylation conditions, halocarbons such as dichloroethane or methylene chloride are used as the solvent, wherein said halocarbons are hazardous to health and exhibit low boiling points. In contrast to that, toluene has a high boiling point and is much less hazardous to health. Toluene can not be used as a solvent in conventional Friedel-Crafts acylations, since toluene would be acylated, too. In contrast to that, under the process conditions defined in items (6) to (8) above, there is substantially no acylation of toluene as the solvent.
(10) The process according to any one of items (1) to (9), wherein a compound of formula VIII 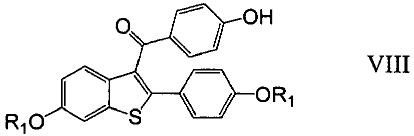 is prepared from the compound of formula VII' 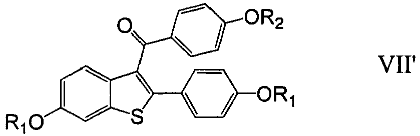 wherein R₁ is selected from the group consisting of linear or branched alkyl, linear or branched alkylaryl and linear or branched alkenyl; preferably R₁ is linear or branched alkyl; and R₂ is selected from the group consisting of linear or branched alkylsulfonyl, arylsulfonyl and linear or branched alkylarylsulfonyl; preferably R₂ is arylsulfonyl,
the process comprising selective deprotection of the -OR₂ group.
(11) The process according to item (10), wherein deprotection of the -OR₂ group is carried out in at least one organic solvent in the presence of an inorganic base optionally in the presence of water, preferably said deprotection is carried out in the presence of a phase transfer catalyst.
(12) The process according to item (11), wherein the phase transfer catalyst is selected from the group consisting of tetraalkyl ammonium salts and tetraalkyl phosphonium salts; preferably tetraalkyl ammonium salts.
(13) The process according to item (11) or (12), wherein the phase transfer catalyst is selected from the group consisting of trihexylmethylammonium chloride, benzyltriethylammonium bromide, benzyltriethylammonium hydroxide, cetyltrimethylammonium bromide, tetra-n-butylammonium bromide (TBAB), hexadecyltributylphosphonium bromide; preferably trihexylmethylammonium chloride, benzyltriethylammonium bromide, benzyltriethylammonium hydroxide, tetra-n-butylammonium bromide (TBAB); more preferably tetra-n-butylammonium bromide (TBAB).
(14) The process according to any one of items (11) to (13), wherein the inorganic base is K₂CO₃, NaOH or KOH, preferably NaOH or KOH, more preferably NaOH.
(15) The process according to any one of items (11) to (14), wherein the solvent is toluene.
(16) The process according to any one of items (10) to (15), wherein a compound of formula X 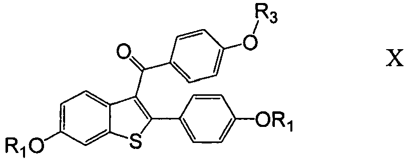 wherein R₁ is selected from the group consisting of linear or branched alkyl, linear or branched alkylaryl and linear or branched alkenyl; and
wherein R₃ is substituted or unsubstituted alkyl, substituted or unsubstituted aryl, substituted or unsubstituted alkylaryl, with the proviso that -OR₃ is substantially not cleavable at deprotection conditions for removal of R₁,
is prepared from the compound of formula VIII 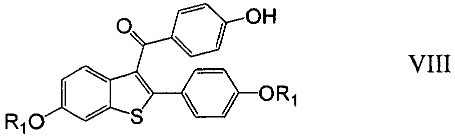 by O-alkylation with a compound of formula IX

Y-R₃ IX

wherein R₃ is defined as above
and Y is Cl, Br, I.
The term "substantially not cleavable at deprotection conditions for removal of R₁" as used herein means that there is no or substantially no cleavage of -OR₃ at conventional conditions for deprotecting -OR₁.
(17) The process according to item (16), wherein R₃ is preferably substituted with at least one amine moiety, preferably a tertiary amine moiety.
(18) The process according to item (16) or (17), wherein Y is Cl.
(19) The process according to any one of items (16) to (18), wherein R₃ is 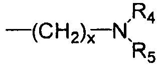 , wherein x = 1 to 20, preferably 1 to 15, more preferably 2 to 10, and in particular 2, and R₄ and R₅ independently from each other represent substituted or unsubstituted alkyl, substituted or unsubstituted aryl, substituted or unsubstituted arylalkyl, or R₄ and R₅ cooperatively with the N-atom form a part of a 5- to 10-membered ring, preferably a 5- to 7-membered ring, more preferably a 5- or 6-membered ring, in particular a 6-membered ring.
In this way, an advantageous introduction of an amine moiety into a compound of formula VIII is provided. Since the amine moiety is introduced subsequent to acylation reaction, undesired side reactions of amine moieties during acylation, e.g. ring opening of heterocyclic amine moieties such as piperidino or pyrrolidino, are effectively avoided.
(20) The process according to any one of items (16) to (19), wherein O-alkylation is carried out under basic conditions, preferably by using aqueous KOH or NaOH as a base, more preferably by using aqueous NaOH as a base.
(21) The process according to item (16) to (20), wherein the compound of formula X comprises at least one amine moiety in R₃, wherein said compound is converted into its respective salt by contacting compound of formula X with an acid, preferably said acid is selected from the group of organic or inorganic acids, more preferably from the group consisting of oxalic acid, succinic acid, lactic acid, malonic acid, HCI, HBr and H₂SO₄, even more preferably HCl, HBr and H₂SO₄. yet even more preferably HCl and HBr, and in particular HBr.
(22) The process according to any one of items (16) to (21), wherein the compound of formula X is recrystallized.
(23) The process according to item (22), wherein compound of formula X in form of its salt is recrystallized using methanol as the solvent.
(24) The process according to any one of items (16) to (23), wherein a compound of formula XI or a salt thereof 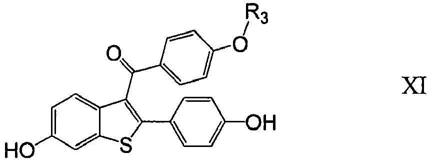 is prepared from the compound of formula X 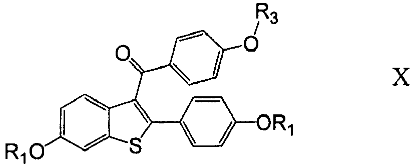 wherein R₁ is selected from the group consisting of linear or branched alkyl, linear or branched alkylaryl and linear or branched alkenyl; and
wherein R₃ is 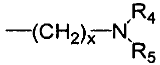 , wherein x = 1 to 20, preferably 1 to 15, more preferably 2 to 10, and in particular 2, and R₄ and R₅ independently from each other represent substituted or unsubstituted alkyl, substituted or unsubstituted aryl, substituted or unsubstituted arylalkyl, or R₄ and R₅ cooperatively with the N-atom form a part of a 5- to 10-membered ring, preferably a 5- to 7-membered ring, more preferably a 5- or 6-membered ring, in particular a 6-membered ring.
by deprotection of the -OR₁ groups.
(25) The process according to item (24), wherein R₁ of compound of formula X is alkyl, preferably methyl, ethyl or tert-butyl, more preferably methyl or ethyl, in particular methyl, and deprotection of the -OR₁ groups is carried out with a Lewis acid, preferably said Lewis acid is BCl₃ or BBr₃, more preferably said Lewis acid is BCl₃.
(26) The process according to item (25), wherein said Lewis acid is used in 2 to 7 times molar amounts, preferably 2.4 to 6 times molar amounts, more preferably 2.6 to 5 times molar amounts and in particular 2.9 to 4.5 times molar amounts relative to compound of formula X.
(27) The process according to item (25) or (26), wherein compound of formula XI comprising an amine moiety is converted into its respective salt by contacting compound of formula XI with an acid, preferably said acid is selected from the group of organic or inorganic acids, more preferably from the group consisting of oxalic acid, succinic acid, lactic acid, malonic acid, HCI, HBr and H₂SO₄, even more preferably HCl, HBr and H₂SO₄, yet even more preferably HCl and HBr, and in particular HBr.
(28) The process according to any one of items (25) to (27), wherein compound of formula XI is recrystallized.
(29) The process according to item (27) or (28), wherein compound of formula XI in form of its salt is recrystallized using a mixture of methanol/water.
(30) A compound of formula VII" 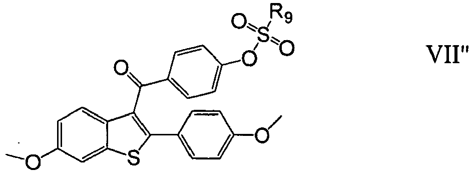 wherein R₉ is methyl, para-toluene or phenyl, preferably para-toluene or phenyl, more preferably phenyl.
(31) The compound according to item (30), wherein the compound of formula VII" is in crystalline form, preferably a compound of formula VII" wherein R₉ is phenyl and whose X-ray diffraction (XRD) pattern has at least signals at the following 2θ values (±0.5° respectively):

| | | | | | | | | | | |
|---|---|---|---|---|---|---|---|---|---|---|
| **2theta[°]** | 12.93 | 13.77 | 14.42 | 16.16 | 18.73 | 21.46 | 22.26 | 23.11 | 23.87 | 24.88 |

, more preferably said compound of formula VII" wherein R₉ is phenyl exhibits a X-ray diffraction (XRD) pattern having at least signals at the following 2θ values (±0.5° respectively):

| | | | | | | | | | | |
|---|---|---|---|---|---|---|---|---|---|---|
| **2theta[°]** | 8.81 | 11.15 | 12.93 | 13.77 | 14.42 | 14.84 | 16.16 | 17.07 | 18.73 | 19.51 |
| | 19.63 | 20.09 | 20.22 | 21.46 | 22.26 | 23.11 | 23.87 | 24.29 | 24.61 | 24.88 |
| | 25.76 | 26.03 | 26.19 | 26.74 | 27.67 | 27.79 | 28.09 | 29.11 | 30.39 | 34.17 |

; or a compound of formula VII" wherein R₉ is methyl and whose X-ray diffraction (XRD) pattern has at least signals at the following 2θ values (±0.5° respectively):

| | | | | | | | | | | |
|---|---|---|---|---|---|---|---|---|---|---|
| **2theta[°]** | 17.86 | 18.77 | 18.93 | 19.07 | 19.77 | 23 | 24.64 | 25.44 | 26.11 | 28.26 |

, preferably said compound of formula VII" wherein R₉ is methyl exhibits a X-ray diffraction (XRD) pattern having at least signals at the following 2θ values (±0.5° respectively):

| | | | | | | | | | | |
|---|---|---|---|---|---|---|---|---|---|---|
| **2theta[°]** | 6.21 | 11.44 | 12.25 | 12.97 | 14.51 | 15.31 | 16.31 | 17.86 | 18.77 | 18.93 |
| | 19.07 | 19.23 | 19.39 | 19.77 | 21.25 | 22.21 | 23 | 23.84 | 24.64 | 25.44 |
| | 25.57 | 26.11 | 28.26 | 29.76 | 29.92 | 30.3 | 39.06 | | | |

; or a compound of formula VII" wherein R₉ is p-toluene and whose X-ray diffraction (XRD) pattern has at least signals at the following 2θ values (±0.5° respectively):

| | | | | | | | | | | |
|---|---|---|---|---|---|---|---|---|---|---|
| **2theta[°]** | 12.09 | 16.05 | 17.59 | 17.86 | 18.97 | 20.53 | 21.05 | 22.57 | 23.03 | 26.44 |

, preferably said compound of formula VII" wherein R₉ is methyl exhibits a X-ray diffraction (XRD) pattern having at least signals at the following 2θ values (±0.5° respectively):

| | | | | | | | | | | |
|---|---|---|---|---|---|---|---|---|---|---|
| **2theta[°]** | 6.04 | 7.54 | 10.84 | 12.09 | 13.05 | 14.38 | 14.81 | 16.05 | 17.59 | 17.86 |
| | 18.01 | 18.97 | 19.18 | 19.41 | 20.4 | 20.53 | 21.05 | 21.3 | 22.57 | 23.03 |
| | 24.17 | 24.37 | 25.3 | 26.08 | 26.44 | 26.85 | 27.97 | 29.07 | 31.1 | 32.46 |

In this preferred embodiment, 28 values may be exact or substantially correspond with a tolerance of ±0.2° or even ±0.5° with the given 2θ values.
(32) A process for preparing raloxifene with the formula 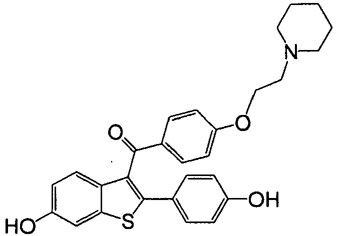 or a pharmaceutically acceptable salt thereof,
comprising the steps of:
a) providing a compound of formula VII' by a process according to any one of items (1) to (9),
b) converting said compound of formula VII' to compound of formula VIII by a process according to any one of items (10) to (15),
c) converting said compound of formula VIII to compound of formula X or optionally a salt thereof, wherein R₃ is 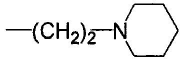 by a process according to any one of items (16) to (20), and
d) converting said compound of formula X or its salt to compound of formula XI according to any one of items (24) to (26) in order to obtain raloxifene or a pharmaceutically acceptable salt thereof,
e) optionally mixing said raloxifene or a pharmaceutically acceptable salt thereof with at least one pharmaceutically acceptable excipient.

The term "pharmaceutically acceptable excipient" as used herein means any physiologically inert, pharmacologically inactive material known in the art being compatible with the physical and chemical characteristics of the active agent.
(33) The process according to item (32), wherein compound of formula VIII is converted into its salt according to item (21) prior to step c), and preferably said salt is recrystallized according to item (23).
(34) The process according to item (32) or (33), wherein compound of formula XI is converted into its salt according to item (27) subsequent to step d), and preferably said salt is recrystallized according to item (29).
(35) The process according to any one of items (32) to (34), wherein raloxifene or its pharmaceutically acceptable salt obtained by said process have a purity > 98% measured by HPLC, more preferably > 99%, even more preferably > 99.2%, yet even more preferably > 99.4%, and in particular > 99.6%.
(36) Use of a compound of formula VII" 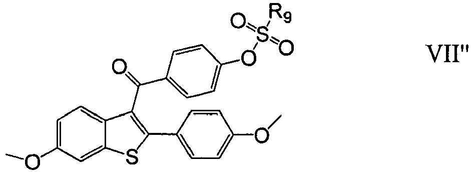 wherein R₉ is methyl, para-toluene or phenyl, preferably para-toluene or phenyl, more preferably phenyl,
in a process for preparing raloxifene or a pharmaceutically acceptable salt thereof.
Preferably, said compound of formula VII' is used in crystalline form.
(37) A process for preparing a crystalline solvate form of a salt of a compound of formula XI 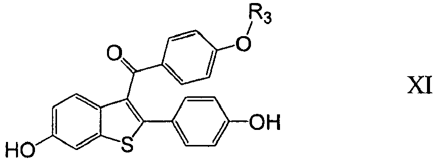 wherein R₃ is 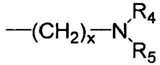 , wherein x = 1 to 20, preferably 1 to 15, more preferably 2 to 10, and in particular 2, and R₄ and R₅ independently from each other represent substituted or unsubstituted alkyl, substituted or unsubstituted aryl, substituted or unsubstituted arylalkyl, or R₄ and R₅ cooperatively with the N-atom form a part of a 5- to 10-membered ring, preferably a 5- to 7-membered ring, more preferably a 5- or 6-membered ring, in particular a 6-membered ring,
wherein a first solvent and a second solvent different from said first solvent are incorporated in said solvate form of the salt of the compound of formula XI with the proviso that a common solvate is formed,
wherein said process comprises the steps of:
a) carrying out a process according to any one of items (24) to (29) wherein a free base or a salt of a compound of formula XI is provided, preferably a salt thereof, more preferably a pharmaceutically acceptable salt thereof,
b) dissolving said compound provided in step a) in a mixture of said first solvent and said second solvent, preferably by heating, more preferably by heating under reflux conditions,
c) optionally distilling off a part of the volume of the mixture of first solvent and second solvent, preferably under reduced pressure in order to obtain a suspension, preferably distilling off is carried out by azeotropic distillation, more preferably distilling off is carried out under reduced pressure,
d) optionally seeding the mixture resulting from step b) or c) in order to induce crystallisation,
e) allowing a solvate form of a salt of a compound of formula XI to precipitate, preferably to crystallize, optionally under agitation, and
f) separating the crystalline solvate form of a salt of a compound of formula XI, preferably separation is carried out by filtration.

As to the meaning of the terms "alkyl", "aryl", "arylalkyl" and "substituted", reference is made to the explanations under item (1).
The term "solvate form" as used herein means a crystalline solid adduct wherein a stoichiometric or nonstoichiometric amount of a first solvent and a second solvent is incorporated in said crystalline solid.
The terms "first solvent"/"second solvent" as used herein denote the order in which said solvents are indicated within the chemical nomenclature used herein.
The term "seeding" as used herein means any conditions known in the art which may assist in the induction of crystallisation, such as adding a seed crystal, scratching the glass containing the mixture resulting from step b) or c) in order to promote the formation of seed crystals and/or cooling the mixture resulting from step b) or c) to or below ambient temperature. Preferably, seeding is effected by adding a seed crystal.
The procedural concept according to this aspect of the invention provides for an unconventional pathway for obtaining a pharmaceutical acceptable salt of compound of formula XI in form of solvates which may represent polymorphs. Polymorph means that a salt is obtained in different crystal structures. Said solvates represent highly valuable intermediates for the preparation of pharmaceutically active agents such as raloxifene or derivatives thereof, since the formation of said solvates provides for a highly effective purification step in the synthetic pathway for preparing a compound of formula XI, while incorporating a first and a second solvent into the crystalline solid of the compound of compound of formula XI. additionally provides for an advantageous setting of the properties of said solvate. Furthermore, the properties of said solvate can be suitably set by selecting an appropriate anion for the salt form of compound of formula XI. For example, properties such as melting point, chemical reactivity, density and solubility can be suitably set by incorporating an appropriate anion and/or appropriate solvents into a crystalline solvate form of a salt of a compound of formula XI, wherein setting the aforementioned properties in turn provides for a suitable setting of procedural conditions in a process for preparing pharmaceutically active agents such as raloxifene or derivatives thereof.
(38) The process according to item (37), wherein a free base of a compound of formula XI provided in step a) is converted into its respective salt by contacting compound of formula XI with an acid prior to step c), preferably said acid is selected from the group of organic or inorganic acids, more preferably from the group consisting of oxalic acid, succinic acid, lactic acid, malonic acid, HCl, HBr and H₂SO₄, even more preferably HCl, HBr and H₂SO₄, yet even more preferably HCl and HBr, and in particular HBr.
(39) A process for preparing a crystalline solvate form of a salt of a compound of formula XI 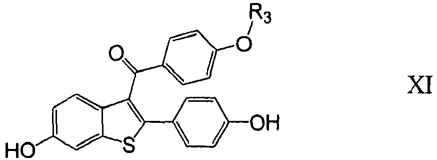 wherein R₃ is 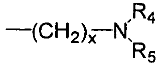 , wherein x = 1 to 20, preferably 1 to 15, more preferably 2 to 10, and in particular 2, and R₄ and R₅ independently from each other represent substituted or unsubstituted alkyl, substituted or unsubstituted aryl, substituted or unsubstituted arylalkyl, or R₄ and R₅ cooperatively with the N-atom form a part of a 5- to 10-membered ring, preferably a 5- to 7-membered ring, more preferably a 5- or 6-membered ring, in particular a 6-membered ring,
wherein a first solvent and a second solvent different from the first solvent is incorporated in said solvate form of the salt of the compound of formula XI with the proviso that a common solvate is formed,
wherein said process comprises the steps of:
i) carrying out a process according to any one of items (16) to (23) wherein via said process, a salt or a free base of a compound of formula X 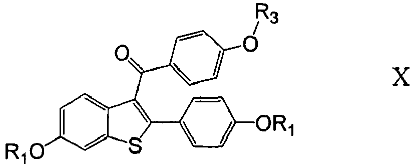 is provided,
   wherein R₁ is selected from the group consisting of linear or branched alkyl, linear or branched alkylaryl and linear or branched alkenyl; and wherein R₃ is defined as above,
ii) deprotecting the -OR₁ groups of said salt of a compound of formula X in the presence of a second solvent in order to convert compound of formula X into compound of formula XI
iii) adding a first solvent to a reaction mixture resulting from step ii), preferably said first solvent is added dropwise within a predetermined time interval, and
iv) heating the reaction mixture resulting from step iii), preferably heating to reflux, and
v) cooling and optionally agitating the reaction mixture resulting from step iv), preferably said reaction mixture is cooled to a temperature of -20 to 20 °C, more preferably -10 to 10 °C, and
vi) separating the solvate form of a salt of a compound of formula XI, preferably separation is carried out by filtration.

As to the meanings of the terms "hydroxy protecting group", "alkyl", "aryl", "arylalkyl", "substituted", "substantially not cleavable in the presence of a Lewis acid at Friedel-Crafts acylation conditions", reference is made to the explanations under item (1).
The term "free base of a compound of formula XI" as employed herein means a compound of formula XI wherein the nitrogen of R₃ is in its free basic form, that is, said nitrogen is not protonated and thus distinct from the nitrogen atom from R₃ of a salt of a compound of formula XI.
This beneficial aspect of the present invention provides for a particularly advantageous reaction pathway for obtaining a solvate form of a salt of compound of formula XI from a salt or a free base of a compound of formula X, wherein a step for isolation of the intermediate salt of a compound of formula X forming in step ii) can be omitted, while the crystalline solvate form of a salt of a compound of formula XI is obtained in both high yield and purity. Furthermore, changing the crystal structure of the pharmaceutical acceptable salt of compound of formula XI by solvating said two solvents therein additionally provides for an advantageous setting of the properties of said solvate. For example, properties such as melting point, chemical reactivity, density and solubility can be suitably set by incorporating appropriate solvents into a crystalline solvate form of a salt of a compound of formula XI, wherein setting the aforementioned properties in turn provides for suitable setting procedural conditions in a process for preparing pharmaceutically active agents such as raloxifene or derivatives thereof.
(40) The process according to item (39), wherein in step i), the compound of formula X is provided in crude form or in isolated form, preferably in isolated form, more preferably in an isolated form wherein the compound of formula X has a purity of at least 90% measured by HPLC, even more preferably at least 95%, yet even more preferably at least 97% and in particular at least 98%.
The term "crude form" as used herein means that the compound of formula X is provided in form of the reaction mixture wherein the reaction for preparing compound of formula X is carried out, that is *in situ*.
The term "isolated form" as used herein means that the compound of formula X is separated from the reaction mixture wherein the reaction for preparing compound of formula X is carried out by appropriate means such as filtration. Preferably, the separated compound is subsequently subjected to further purification step(s).
According to this embodiment of the invention, compound of formula X is provided in a suitable form. Applying a compound of formula X in crude form as the starting material provides for reducing both work-up and purification efforts in a process for preparing a compound of formula XI. On the other hand, applying a compound of formula X in isolated form as the starting material provides for a surprisingly high purity of compound of formula XI.
(41) The process according to item (39) or (40), wherein in step i) a compound of formula X' is provided 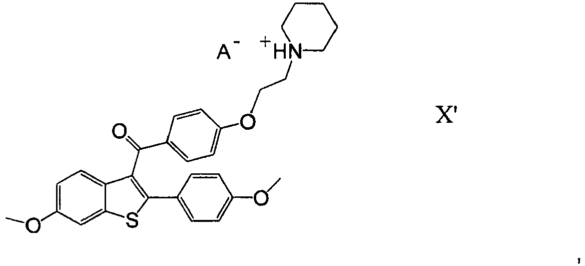 wherein A⁻ is Cl⁻ or Br⁻, preferably Br⁻.
(42) The process according to item (40), wherein the compound of formula X' is in crystalline form, preferably a compound of formula X' wherein A⁻ is Cl⁻ and whose X-ray diffraction (XRD) pattern has at least signals at the following 2θ values (±0.5° respectively):

| | | | | | | | | | | |
|---|---|---|---|---|---|---|---|---|---|---|
| **2theta[°]** | 11.41 | 13.07 | 16.74 | 19.27 | 19.39 | 21.56 | 23.42 | 24 | 24.63 | 26.05 |

, more preferably said compound of formula X' wherein A⁻ is Cl⁻ exhibits a X-ray diffraction (XRD) pattern having at least signals at the following 2θ values (±0.5° respectively):

| | | | | | | | | | | |
|---|---|---|---|---|---|---|---|---|---|---|
| **2theta[°]** | 6.41 | 11.41 | 12.56 | 13.07 | 14.82 | 15.45 | 15.86 | 16.74 | 17.1 | 18.33 |
| | 18.95 | 19.27 | 19.39 | 19.79 | 20.09 | 21.56 | 22.32 | 22.92 | 23.42 | 24 |
| | 24.63 | 24.77 | 25.53 | 25.89 | 26.05 | 26.83 | 29.35 | 29.49 | 30.45 | 42.03 |

; or a compound of formula X' wherein A⁻ is Br⁻ and whose X-ray diffraction (XRD) pattern has at least signals at the following 2θ values (±0.5° respectively):

| | | | | | | | | | | |
|---|---|---|---|---|---|---|---|---|---|---|
| **2theta[°]** | 7.41 | 13.05 | 16.64 | 19.07 | 19.22 | 21.45 | 23.31 | 24.34 | 25.81 | 26.62 |

, preferably said compound of formula X' wherein A⁻ is Br⁻ exhibits a X-ray diffraction (XRD) pattern having at least signals at the following 2θ values (±0.5° respectively):

| | | | | | | | | | | |
|---|---|---|---|---|---|---|---|---|---|---|
| **2theta[°]** | 7.41 | 11.38 | 13.05 | 14.57 | 15.28 | 16.64 | 17.1 | 19.07 | 19.22 | 19.56 |
| | 19.71 | 20.04 | 21.45 | 22.35 | 22.84 | 23.31 | 23.81 | 24.34 | 25.45 | 25.81 |
| | 25.96 | 26.12 | 26.43 | 26.62 | 27.3 | 29.3 | 30.33 | 33.65 | 33.95 | 41.36 |

In this preferred embodiment, 26 values may be exact or substantially correspond with a tolerance of ±0.2° or even ±0.5° with the given 2θ values.
(43) The process according to any one of items (39) to (42), wherein in step ii), deprotection is carried out by applying a process according to any one of items (25) to (29).
(44) The process according to any one of items (37) to (43), wherein the crystalline solvate form of a salt of a compound of formula XI is based on a pharmaceutically acceptable salt of a compound of formula XI, preferably the acid forming said pharmaceutically acceptable salt of a compound of formula XI whereupon said solvate form is based is selected from the group of organic or inorganic acids, preferably from the group consisting of oxalic acid, succinic acid, lactic acid, malonic acid, HCl, HBr and H₂SO₄, more preferably HCl, HBr and H₂SO₄, even more preferably HCl and HBr, and in particular HBr.
(45) The process according to any one of items (27) to (44), wherein the anion of a salt of a compound of formula XI obtained in step ii) is exchanged by another anion prior to step iii), preferably by an anion of an acid forming a pharmaceutically acceptable salt as defined in item (44).
This embodiment of the invention provides for a particularly advantageous conversion of a salt of a compound of formula XI obtained in step ii) to a pharmaceutically acceptable salt of a compound of formula XI by exchange of the anion. Thereby, a solvate form of a pharmaceutically acceptable salt of a compound of formula XI is obtained in step vi) which provides for valuable intermediates for preparing a non-solvated pharmaceutically acceptable salt of a compound of formula XI. Exchange of the anion may e.g. be performed by converting the salt of a compound of formula XI obtained in step ii) into its free base by adding an (aqueous) base. The resulting free base of compound of formula XI may then be separated by suitable means such as extraction (from an aqueous phase) with an appropriate organic solvent. Then, an appropriate acid is added to the free base of compound of formula XI in order to form a pharmaceutically acceptable salt of a compound of formula XI.
(46) The process according to any one of items (37) to (45), wherein said first solvent is incorporated into the salt of compound of formula XI in an amount of percent by weight which is lower than the amount of percent by weight of the second solvent
(47) The process according to any one of items (37) to (46), wherein the crystalline solvate form of a salt of compound of formula XI comprises said first solvent in an amount of about 0.1 to 8 percent by weight and said second solvent in an amount of about 3 to 15 percent by weight relative to the solvate form of a salt of compound of formula XI, preferably compound of formula XI provided in step a) comprises said first solvent in an amount of about 1 to 5 percent by weight and said second solvent in an amount of about 7.7 to 10 percent by weight relative to the solvate form of a salt of compound of formula XI.
(48) The process according to any one of items (37) to (47), wherein said first solvent is an alcohol and said second solvent is a halocarbon, preferably said first solvent is a C₁-C₄-alcohol and said second solvent is a C₁-C₁₀-halocarbon optionally comprising a benzene ring, more preferably said first solvent is methanol or ethanol and said second solvent is methylene chloride, chloroform or chlorobenzene, in particular said first solvent is methanol and said second solvent is methylene chloride.
(49) The process according to any one of items (39) to (48), wherein the solvate form of a salt of a compound of formula XI is a crystalline solvate form of a compound of formula XI' 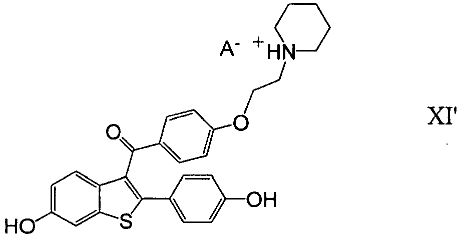 wherein A⁻ is the deprotonated form of an organic or inorganic acid,
said crystalline solvate form comprising a first solvent and a second solvent.
(50) A crystalline solvate form according to item (49), wherein said crystalline solvate form comprises said first solvent in an amount of about 0.1 to 8 percent by weight and said second solvent in an amount of about 3 to 15 percent by weight relative to the solvate form of a salt of compound of formula XI, preferably compound of formula XI provided in step a) comprises said first solvent in an amount of about 1 to 5 percent by weight and said second solvent in an amount of about 7.7 to 10 percent by weight relative to the solvate form of a the salt of compound of formula XI.
(51) The crystalline solvate form according to item (49) or (50), wherein A⁻ is selected from the group consisting of oxalate, succinate, lactate, malonate, Cl⁻, Br and SO₄²⁻, preferably Cl⁻, Br⁻ and SO₄²⁻ more preferably Cl⁻, Br⁻, and even more preferably Br⁻.
(52) The crystalline solvate form according to any one of items (49) to (51), wherein said first solvent is an alcohol and said second solvent is a hydrocarbon or halocarbon, preferably said first organic solvent is a C₁-C₄-alcohol and said second organic solvent is a C₆-C₁₀-hydrocarbon comprising a benzene moiety or a C₁-C₁₀-halocarbon optionally comprising a benzene moiety, more preferably said first solvent is methanol or ethanol and said second solvent is toluene, methylene chloride, chloroform or chlorobenzene, in particular, said first solvent is methanol and said second solvent is methylene chloride.
(53) The crystalline solvate form according to any one of items (49) to (52), wherein a compound of formula XI' wherein A⁻ is Cl⁻ and wherein the first solvent is methanol and the second solvent is methylene chloride exhibits a X-ray diffraction (XRD) pattern having at least signals at the following 2θ values (±0.5° respectively):

| | | | | | | | | | | |
|---|---|---|---|---|---|---|---|---|---|---|
| **2theta[°]** | 13.46 | 16.45 | 17.56 | 18.76 | 19.1 | 19.53 | 19.76 | 20.75 | 21.26 | 23.57 |

preferably said compound of formula XI' wherein A⁻ is Cl⁻ and wherein the first solvent is methanol and the second solvent is methylene chloride exhibits a X-ray diffraction (XRD) pattern having at least signals at the following 2θ values (±0.5° respectively):

| | | | | | | | | | | |
|---|---|---|---|---|---|---|---|---|---|---|
| **2theta[°]** | 11.01 | 13.31 | 13.46 | 15.88 | 16.45 | 17.56 | 18.76 | 18.9 | 19.1 | 19.53 |
| | 19.76 | 20.75 | 20.98 | 21.26 | 21.61 | 23.4 | 23.57 | 24.4 | 24.76 | 25.32 |
| | 25.42 | 25.84 | 25.96 | 28.69 | 28.73 | 28.78 | 29.39 | 31.1 | 31.17 | 32.74 |

; or a compound of formula XI' wherein A⁻ is Cl⁻ and wherein the first solvent is methanol and the second solvent is toluene exhibiting a X-ray diffraction (XRD) pattern having at least signals at the following 2θ values (±0.5° respectively):

| | | | | | | | | | | |
|---|---|---|---|---|---|---|---|---|---|---|
| **2theta[°]** | 13.45 | 14.43 | 16.38 | 18.34 | 19.11 | 19.74 | 20.38 | 21.18 | 22.66 | 23.72 |

preferably said compound of formula XI' wherein A⁻ is Cl⁻ and wherein the first solvent is methanol and the second solvent is toluene exhibits a X-ray diffraction (XRD) pattern having at least signals at the following 2θ values (±0.5° respectively):

| | | | | | | | | | | |
|---|---|---|---|---|---|---|---|---|---|---|
| **2theta[°]** | 11.12 | 13.45 | 14.43 | 15.75 | 16.38 | 17.52 | 18.34 | 18.61 | 19.11 | 19.4 |
| | 19.74 | 20.38 | 20.64 | 20.95 | 21.18 | 21.37 | 22.66 | 23 | 23.47 | 23.72 |
| | 24.1 | 24.17 | 24.36 | 25.08 | 25.51 | 25.64 | 25.89 | 28 | 29.02 | 29.28 |

; or a compound of formula XI' wherein A⁻ is Br⁻ and wherein the first solvent is methanol and the second solvent is methylene chloride exhibiting a X-ray diffraction (XRD) having at least signals at the following 2θ values (±0.5° respectively):

| | | | | | | | | | | |
|---|---|---|---|---|---|---|---|---|---|---|
| **2theta[°]** | 16.43 | 17.57 | 18.76 | 18.96 | 19.06 | 20.98 | 21.18 | 23.39 | 23.54 | 25.28 |

preferably said compound of formula XI' wherein A⁻ is Br⁻ and wherein the first solvent is methanol and the second solvent is methylene chloride exhibits a X-ray diffraction (XRD) pattern having at least signals at the following 2θ values (±0.5° respectively):

| | | | | | | | | | | |
|---|---|---|---|---|---|---|---|---|---|---|
| **2theta[°]** | 10.82 | 13.47 | 14.11 | 15.92 | 16.43 | 17.11 | 17.57 | 18.76 | 18.96 | 19.06 |
| | 19.52 | 19.75 | 20 | 20.73 | 20.98 | 21.18 | 23.06 | 23.39 | 23.54 | 24.37 |
| | 24.53 | 25.28 | 25.88 | 28.55 | 28.64 | 28.74 | 29.4 | 31.07 | 31.71 | 32.68 |

In this preferred embodiment, 2θ values may be exact or substantially correspond with a tolerance of ±0.2° or even ±0.5° with the given 2θ values.
(54) A process for preparing a non-solvated pharmaceutically acceptable salt of a compound of formula XI 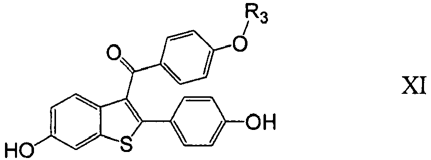 wherein R₃ is 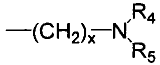 , wherein x = 1 to 20, preferably 1 to 15, more preferably 2 to 10, and in particular 2, and R₄ and R₅ independently from each other represent substituted or unsubstituted alkyl, substituted or unsubstituted aryl, substituted or unsubstituted arylalkyl, or R₄ and R₅ cooperatively with the N-atom form a part of a 5- to 10-membered ring, preferably a 5- to 7-membered ring, more preferably a 5- or 6-membered ring, in particular a 6-membered ring,
comprising the steps of:
i) carrying out a process according to any one of items (39) to (48) to provide a crystalline solvate form of a pharmaceutically acceptable salt of a compound of formula XI 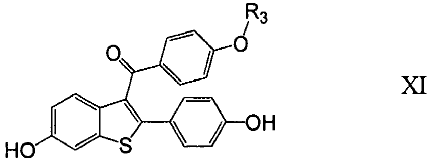 wherein R₃ is 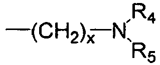 , wherein x = 1 to 20, preferably 1 to 15, more preferably 2 to 10, and in particular 2,
   and R₄ and R₅ independently from each other represent substituted or unsubstituted alkyl, substituted or unsubstituted aryl, substituted or unsubstituted arylalkyl, or R₄ and R₅ cooperatively with the N-atom form a part of a 5- to 10-membered ring, preferably a 5- to 7-membered ring, more preferably a 5- or 6-membered ring, in particular a 6-membered ring,
ii) dissolving said crystalline solvate form provided in step i) in an organic solvent, wherein said organic solvent forms an azeotrope with at least one solvent incorporated in said solvate form,
iii) optionally seeding the mixture resulting from step ii) in order to induce crystallisation,
iv) allowing the pharmaceutically acceptable salt of compound of formula XI to precipitate, preferably to crystallize, optionally under agitation, and
v) separating the pharmaceutically acceptable salt of a compound of formula XI, preferably separation is carried out by filtration.

The term "crystalline solvate form" as used herein means a crystalline solid adduct wherein a stoichiometric or nonstoichiometric amount of at least one organic solvent is incorporated in said crystalline solid. In a particular embodiment, a first solvent and a second solvent is incorporated in said crystalline solid.
The term "azeotrope" as used herein means a mixture of two (or more) fluid components, in this case at least one organic solvent incorporated in the crystalline solvate form provided in step i) / organic solvent applied in step ii), wherein said two (or more) fluid components can not be separated by simple distillation, that is distillation without rectification step.
According to this preferred aspect of the invention, a process for preparing a non-solvated pharmaceutically acceptable salt of a compound of formula XI is provided, wherein the crystalline solvate form of a pharmaceutically acceptable salt of a compound of formula XI is converted to the non-solvated form of the respective pharmaceutically acceptable salt in both high yields and high purity, while the obtained non-solvate form of the pharmaceutically acceptable salt of a compound of formula XI is substantially free of organic solvent(s) incorporated into the starting material in solvate form.
(55) The process according to item (54), wherein the crystalline solvate form of a pharmaceutically acceptable salt of a compound of formula XI comprises a first solvent and a second solvent different from the first solvent, wherein said first solvent and said second solvent are incorporated in the pharmaceutically acceptable salt of a compound of formula XI.
According to this preferred embodiment of the invention, a particularly suitable starting material for the process for preparing a non-solvated pharmaceutically acceptable salt of a compound of formula XI is provided. Surprisingly, even though said crystalline form comprises two organic solvents, said crystalline solvate form provides for a non-solvated form of the respective pharmaceutically acceptable salt in both particularly high yields and exceptional high purity, while the obtained non-solvate form of the pharmaceutically acceptable salt of a compound of formula XI is substantially free of said first solvent and said second solvent incorporated into the starting material in solvate form.
(56) The process according to item (54) or (55), wherein said first solvent is incorporated into the salt of compound of formula XI in an amount of percent by weight which is lower than the amount of percent by weight of the second solvent
(57) The process according to any one of items (54) to (56), wherein the crystalline solvate form of a salt of compound of formula XI comprises said first solvent in an amount of about 0.1 to 8 percent by weight and said second solvent in an amount of about 3 to 15 percent by weight relative to the solvate form of a salt of compound of formula XI, preferably compound of formula XI provided in step a) comprises said first solvent in an amount of about 1 to 5 percent by weight and said second solvent in an amount of about 7.7 to 10 percent by weight relative to the solvate form of a salt of compound of formula XI.
(58) The process according to any one of items (54) to (57), wherein said first solvent is an alcohol and said second solvent is a hydrocarbon or halocarbon, preferably said first organic solvent is a C₁-C₄-alcohol and said second organic solvent is a C₆-C₁₀-hydrocarbon comprising a benzene moiety or a C₁-C₁₀-halocarbon optionally comprising a benzene moiety, more preferably said first solvent is methanol or ethanol and said second solvent is toluene, methylene chloride, chloroform or chlorobenzene, even more preferably said first solvent is methanol and said second solvent is toluene, methylene chloride or chloroform, and in particular, said first solvent is methanol and said second solvent is methylene chloride.
(59) The process according to any one of items (54) to (58), wherein the solvate form of a salt of a compound of formula XI is a solvate as defined in any one of items (49) to (53).
(60) The process according to any one of items (54) to (59), wherein the azeotrope formed in step ii) forms under normal or reduced pressure, preferably under reduced pressure.
(61) The process according to any one of items (54) to (60), wherein solvent(s) incorporated into said solvate form of a pharmaceutically acceptable salt of a compound of formula XI is/are removed by azeotropic distillation of the mixture resulting from step ii), preferably said azeotropic distillation is carried out at reduced pressure.
(62) The process according to any one of item (54) to (61), wherein said organic solvent of step ii) is an alcohol, preferably a water-miscible alcohol, more preferably a C₁-C₄-alcohol, even more preferably methanol or ethanol, and in particular methanol.
(63) The process according to any one of items (54) to (62), wherein said organic solvent of step ii) comprises a predetermined amount of water, preferably said amount of water is predetermined by a volume ratio of water and organic solvent of 0 to 0.5, preferably 0.24 to 0.44, more preferably 0.27 to 0.40, and in particular 0.29 to 0.38.
(64) The process according to item (63), wherein said water used in step ii) is potable water, preferably deionized water, more preferably reverse osmosis water.
(65) The process according to any one of items (54) to (63), wherein in step ii), the resulting mixture is heated prior to step iii), preferably heating is under reflux conditions.
(66) The process according to any one of items (54) to (65), wherein the acid forming the pharmaceutically acceptable salt of a compound of formula XI whereupon said solvate form is based is selected from the group of organic or inorganic acids, preferably from the group consisting of oxalic acid, succinic acid, lactic acid, malonic acid, HCl, HBr and H₂SO₄, more preferably HCl, HBr and H₂SO₄, even more preferably HCl and HBr, and in particular HBr.
(67) The process according to any one of items (54) to (66), wherein activated charcoal is added in step ii) and said charcoal is separated from the mixture resulting from step ii) prior to step iii), preferably separation is carried out by filtration.
(68) The process according to any one of items (54) to (67), wherein said pharmaceutically acceptable salt of a compound of formula XI obtained in step v) has a purity of > 98% measured by HPLC, more preferably > 99%, even more preferably > 99.2%, yet even more preferably > 99.4%, and in particular > 99.6%.

### Detailed description of the invention

The present invention is now described in more detail by referring to further preferred and further advantageous embodiments and examples, which are however presented for illustrative purposes only and shall not be understood as limiting the scope of the present invention.

Reaction Scheme 1 illustrates a preferred embodiment of the process according to the present invention. The illustrative and preferred embodiment is shown here for preparing a compound of formula Xl

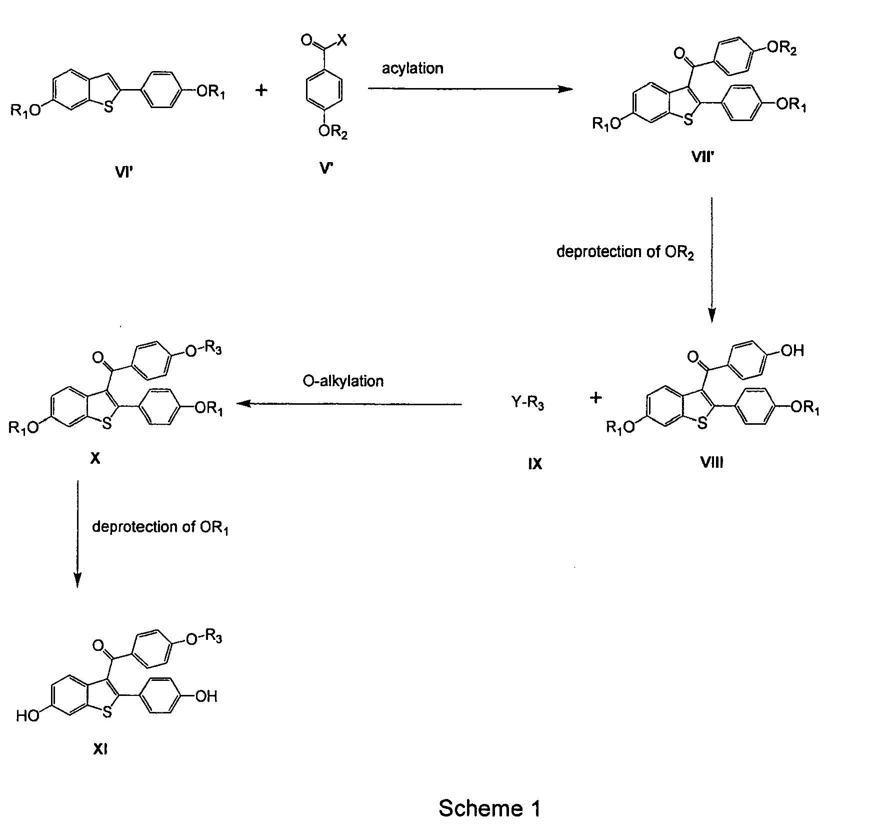

According to the preferred embodiment of Scheme 1 (wherein R₁, R₂, R₃, X and Y are defined as in the preceding items), a compound of formula Vll' is prepared by acylating a compound of formula Vl' with a compound of formula V' in the presence of a Lewis acid catalyst.

Compound of formula V' is preferably prepared by a reaction pathway as depicted in Scheme 2 below.

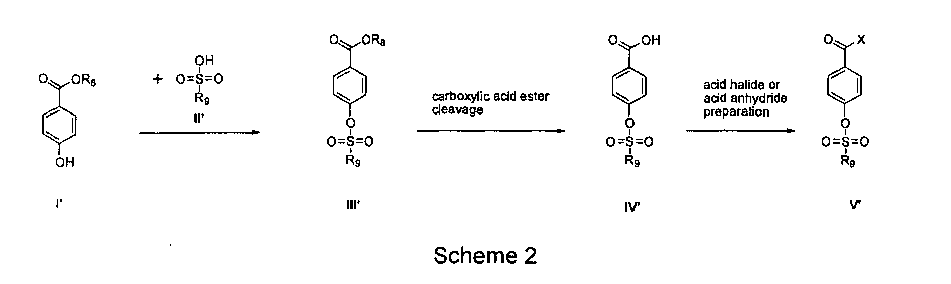

According to the preferred embodiment of Scheme 2 (wherein R₈ and R₉ are defined as in the preceding items), a compound of formula V' is prepared by esterification of a compound of formula I' with a compound of formula ll'. Next, compound of formula lll' is converted into compound of formula IV' by cleavage of the carboxylic acid ester moiety, and compound of formula lV' in turn is converted to a carboxylic acid halogenide of formula V'. For example, compound of formula V' may be obtained by using the following reactants: thionyl chloride or phosphorus pentachloride for obtaining carboxylic acid chlorides and phosphorus pentabromide for obtaining carboxylic acid bromides respectively.

Compound of formula Vl' is readily available. For example, it can be prepared by reacting m-methoxythiophenol with a-bromo-4-methoxyacetophenone as described in. US 4,133,814.

The advantage of introducing two different protecting groups R₁and R₂ into compound of formula Vll' is that an orthogonal protecting group strategy is provided, that is R₁ and R₂ can be independently from each other selectively removed. Protecting groups R₁ and R₂ have to be suitably selected with the proviso that -OR₁ and -OR₂ are substantially not cleavable in the presence of a Lewis acid at Friedel-Crafts acylation conditions.

Conventionally, Friedel-Crafts acylation is carried out with a Lewis acid as the catalyst in about 1 molar amount to about 2 molar amount relative to the compound to be acylated, wherein the temperature of the reaction mixture is typically within a range of about 0°C to ambient temperature, wherein elevated temperatures may be required for less reactive compounds to be acylated.

It was surprisingly found that Friedel-Crafts acylation can be carried out with Lewis acid catalyst used in substoichiometric amounts relative to the compound to be acylated, wherein the temperature of the reaction mixture is within a range of about 50°C to reflux temperature of the reaction mixture. Preferably, the Lewis acid catalyst is used in about 0.004 to 0.07 times molar amounts, more preferably in about 0.008 to 0.05 times molar amounts, and in particular in an amount of 0.01 to 0.02 relative to the compound to be acylated. Since the amount of Lewis acid as the catalyst can be significantly reduced compared to conventional Friedel-Crafts conditions, acylation of compound of formula Vl'can be carried out under mild reaction conditions. Thus, a stable and reliably process is provided, since cleavage of the OR₁ moieties is avoided owing to the significantly reduced amount of Friedel-Crafts catalyst.

Mostly, Lewis acids such as BCl₃, BF₃ and AlC1₃ are used as a catalyst for conventional Friedel-Crafts acylation. Surprisingly, Lewis acids comprising divalent metal represent effective catalysts for Friedel-Crafts acylation providing both fast reaction rates and high conversion rates. Preferably, a divalent metal generated in situ by comproportionation of elemental metal and salt comprising trivalent metal is used as the Lewis acid catalyst.

Furthermore, both metal cation and counter anion may be suitably selected in order to improve activity of the Lewis acid catalyst. Preferably, the Lewis acid is FeCl₃, FeCl₂, FeBr₃, FeBr₂, FeSO₄, Fe₂(SO₄)₃, ZnCl₂, ZnBr₂, ZnSO₄; more preferably FeCl₂, FeSO₄, ZnC1₂; even more preferably FeSO₄, ZnCl₂; in particular FeSO₄ generated in situ by comproportionation of elemental Fe and Fe₂(SO₄)₃, wherein Fe₂(SO₄)₃ is preferably applied in pentahydrate form. Surprisingly, Fe₂(SO₄)₃ in pentahydrate form represents an effective catalyst, since under conventional Friedel-Crafts acylation conditions, water disturbs or even prevents the acylation reaction.

Preferably, chlorobenzene, dichlorobenzene or toluene is used as the solvent in the acylation step, more preferably chlorobenzene or toluene, and in particular toluene. Conventionally, at Friedel-Crafts acylation conditions, halocarbons such as dichloroethane or methylene chloride are preferably used as the solvent. However, such halocarbons are hazardous to health. Furthermore, they exhibit low boiling points, that is they are relatively volatile. Therefore, it is very likely that maximum allowable concentration (MAC) values may be exceeded. The aromatic solvents chlorobenzene, dichlorobenzene or toluene provide for efficient reaction rates. Furthermore, the relatively high boiling aromatics chlorobenzene, dichlorobenzene and toluene are less hazardous than the aforementioned halocarbons. Surprisingly, chlorobenzene, dichlorobenzene and toluene provide for an efficient acylation, while further providing improvement of both environmental friendliness of the process and working conditions. Even more surprisingly, it was found that toluene represents a suitable solvent for Friedel-Crafts acylation, since toluene is acylated under conventional Friedel-Crafts conditions and can therefore not be used as a solvent under conventional Friedel-Crafts conditions.

Further according to the preferred embodiment illustrated by Scheme 1, a compound of Vlll is prepared by subjecting a compound of formula Vll' to a deprotection reaction, wherein -OR₂ is selectively cleaved. Said selective deprotection reaction is enabled by the beneficial present orthogonal protecting group strategy, i.e. introducing two different hydroxy protecting groups R₁ and R₂ in compound of formula Vll' by the aforementioned acylation step.

The hydroxy protecting groups R₁ and R₂ are stable under basic conditions and cleavable under acidic conditions, but substantially not cleavable in the presence of a Lewis acid at Friedel-Crafts acylation conditions, while the other hydroxy protecting group is cleavable under basic conditions and stable under acidic conditions. Preferably, R₁ is stable under basic conditions and cleavable under acidic conditions but substantially not cleavable in the presence of a Lewis acid at Friedel-Crafts acylation conditions, and R₂ is cleavable under basic conditions and stable under acidic conditions. More preferably R₁ is selected from the group consisting of linear or branched alkyl, linear or branched alkylaryl and linear or branched alkenyl; preferably R₁ is linear or branched alkyl, and R₂ is selected from the group consisting of linear or branched sulfonylalkyl, sulfonylaryl and linear or branched sulfonylalkylaryl; preferably R₂ is sulfonylaryl. Even more preferably, R₁ is selected from the group consisting of methyl, ethyl, tert-butyl, benzyl, p-methoxybenzyl (PMB), trityl (Tr), allyl; preferably methyl, ethyl, tert-butyl, more preferably methyl, and R₂ is selected from the group consisting of mesyl, tosyl and benzene sulfonyl; preferably R₂ is tosyl and benzene sulfonyl; more preferably R₂ is benzene sulfonyl. In particular, R₁ is methyl and R₂ is benzene sulfonyl.

By suitably selecting protecting groups R₁ and R₂, which are located at different positions of the benzo[b]thiophene moiety, complete deprotection of both -OR₁ and -OR₂ can be attained under mild conditions. It is known that cleavability of a certain protecting group, for example methyl, may differ at the different positions of the benzo[b]thiophene moiety. For example, WO 98/48792, which does not disclose an orthogonal protecting group strategy since all hydroxy protecting groups are methyl groups, describes that a methoxy group at the 3-benzoyl substituent of the benzo[b]thiophene moiety can not be cleaved under conventional cleavage conditions wherein a large excess of Lewis acid such as BCI₃ is applied as the cleaving agent, while methoxy groups at the 2- and 6-positions of benzo[b]thiophene are smoothly cleaved under the aforementioned conditions. For removing the methyl group from the methoxy group at the 3-benzoyl substituent of the benzo[b]thiophene moiety, harsh conditions like refluxing in pyridinium chloride had to be applied, nevertheless removal was not complete.

Preferably, the aforementioned deprotection step for cleavage of the -OR₂ group is carried out under phase transfer catalysis conditions, wherein the reaction is carried out in a mixture of organic solvent, such as toluene and water in the presence of an inorganic base such as NaOH or KOH and a phase transfer catalyst such as tetra-n-butylammonium bromide (TBAB).

Subsequent to deprotection of -OR₂, compound of formula Vlll is converted to compound of formula X by 0-alkylation using compound of formula lX as the alkylation reagent. Preferably, Y of the alkylation reagent is Cl. Furthermore, R₃ preferably has the following formula 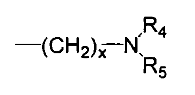 , wherein x = 1 to 20, preferably 1 to 15, more preferably 2 to 10, and in particular 2, and R₄ and R₅ independently from each other represent substituted or unsubstituted alkyl, substituted or unsubstituted aryl, substituted or unsubstituted alkylaryl, or R₄ and R₅ cooperatively with the N-atom form a part of a 5- to 10-membered ring, preferably a 5- to 7-membered ring, more preferably a 5- or 6-membered ring, in particular a 6-membered ring.

Further according to the preferred embodiment illustrated by Scheme 1, subsequent to O-alkylation, a deprotection reaction is carried out in order to convert compound of formula X to compound of formula Xl. For example, in case R₁ of compound of formula X is alkyl such as methyl, deprotection of the -OR₁ groups is carried out with a Lewis acid such as BC1₃ as the cleaving agent. Since -OR₁ is substantially not cleavable in the presence of a Lewis acid at Friedel-Crafts acylation conditions, deprotection conditions have to be suitably selected in order to achieve complete cleavage of -OR₁. Therefore, the Lewis acid as the cleavage agent is used in relatively large excess, preferably in 2 to 7 times molar amounts, preferably 2.4 to 6 times molar amounts, more preferably 2.6 to 5 times molar amounts and in particular 2.9 to 4.5 times molar amounts relative to compound of formula X.

Preferably, compound of formula Xl represents a pharmaceutically active agent such as raloxifene.

According to another preferred embodiment, compounds of formula lll', lV', Vll", X' and a crystalline solvate form of a compound of formula Xl are respectively used for preparing a pharmaceutically active agent, wherein said pharmaceutically active agent is optionally admixed with at least one pharmaceutically-acceptable excipient. Preferably, said pharmaceutically active agent is raloxifene or a pharmaceutically acceptable salt thereof.

Pharmaceutically-acceptable excipients include, but are not limited to, solvents, polymers, plasticizers, fillers, binders, buffer systems, surfactants, dyes or pigments, preservatives and flavouring substances.
Reaction Scheme 3 illustrates another preferred embodiment of the process according to the present invention. The illustrative and preferred embodiment is shown here for preparing a non-solvated pharmaceutically acceptable salt of a compound of formula Xl:

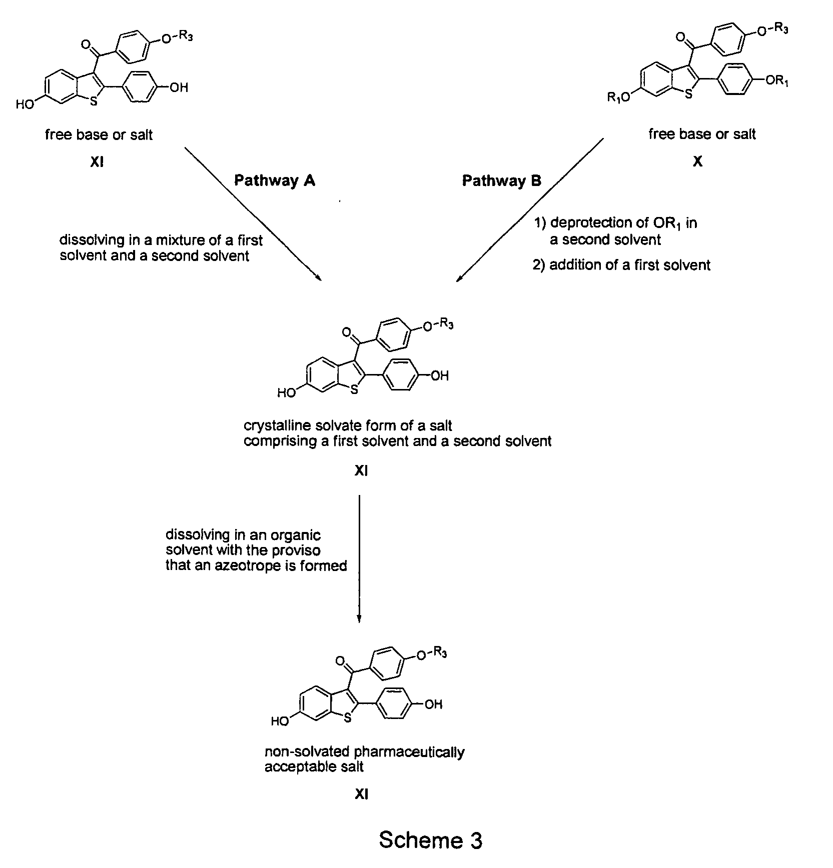

According to the preferred embodiment of Scheme 3 (wherein R_{1,} R₃, first solvent and second solvent are defined as in the preceding items), a crystalline solvate form of a compound of formula Xl is prepared either from a compound of formula Xl (pathway A) or from a compound of formula X (pathway B). Preferably, compounds of formula X and Xl are prepared according to the preferred embodiment of Scheme 1 as described above.

According to one aspect of the invention, a crystalline solvate form of a compound of formula Xl is prepared via pathway A comprising the steps of:
a) providing a free base or a salt of a compound of formula Xl, preferably a salt thereof, more preferably a pharmaceutically acceptable salt thereof,
b) dissolving said compound provided in step a) in a mixture of a first solvent and a second solvent, preferably by heating, more preferably by heating under reflux conditions,
c) optionally distilling off a part of the volume of the mixture of first solvent and second solvent, preferably under reduced pressure in order to obtain a suspension, preferably distilling off is carried out by azeotropic distillation, more preferably distilling off is carried out under reduced pressure,
d) optionally seeding the mixture resulting from step b) or c) in order to induce crystallisation,
e) allowing a solvate form of a salt of a compound of formula Xl to precipitate, preferably to crystallize, optionally under agitation, and
f) separating the crystalline solvate form of a salt of a compound of formula Xl , preferably separation is carried out by filtration.

It was surprisingly found that the above procedural concept provides crystalline solvate forms of a salt of a compound of formula Xl. Said crystalline solvate forms represents highly valuable intermediates for the preparation of pharmaceutically active agents such as raloxifene or derivatives thereof, since said solvate is obtained in exceptional high purity. Thus, a highly effective purification step in a synthetic pathway for preparing a compound of formula Xl is provided. Furthermore, incorporating a first and a second solvent into the crystalline solid of the compound of compound of formula Xl additionally provides for an advantageous setting of the properties of said solvate, wherein the properties of said solvate can be suitably set by selecting an appropriate anion for the salt form of compound of formula Xl. For example, properties such as melting point, chemical reactivity, density and solubility can furthermore be suitably set by incorporating an appropriate anion and/or appropriate solvents into a crystalline solvate form of a salt of a compound of formula Xl, wherein setting the aforementioned properties in turn provides for a suitable setting of procedural conditions in a process for preparing pharmaceutically active agents such as raloxifene or derivatives thereof.

In a preferred embodiment of pathway A, a free base of a compound of formula Xl provided in step a) is converted into its respective salt by contacting compound of formula Xl with an acid prior to step c), preferably said acid is selected from the group of organic or inorganic acids, more preferably from the group consisting of oxalic acid, succinic acid, lactic acid, malonic acid, HCl, HBr and H₂SO₄, even more preferably HCl, HBr and H₂SO₄, yet even more preferably HCl and HBr, and in particular HBr. In this way, the chemical and/or physical properties of the solvate obtained in step f) can be suitably set in view of procedural conditions in a process for preparing pharmaceutically active agents such as raloxifene or derivatives thereof.

According to another aspect of the invention, a crystalline solvate form of a compound of formula XI is prepared via pathway B comprising the steps of:
i) providing a salt or a free base of a compound of formula X 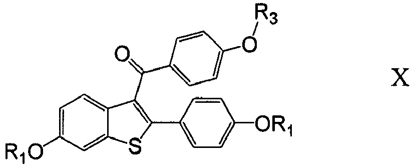 wherein R₁ is selected from the group consisting of linear or branched alkyl, linear or branched arylalkyl and linear or branched alkenyl,
   , and
   wherein R₃ is defined as above,
ii) deprotecting the -OR₁ groups of said salt of a compound of formula X in the presence of a second solvent in order to convert compound of formula X into compound of formula XI
iii) adding a first solvent to a reaction mixture resulting from step ii), preferably said first solvent is added dropwise within a predetermined time interval, and
iv) heating the reaction mixture resulting from step iii), preferably heating to reflux, and
v) cooling and optionally agitating the reaction mixture resulting from step iv), preferably said reaction mixture is cooled to a temperature of -20 to 20 °C, more preferably -10 to 10 °C, and
vi) separating the solvate form of a salt of a compound of formula XI, preferably separation is carried out by filtration.

Surprisingly, when using compound X as a starting compound, a solvate form of a salt of compound of formula XI is obtained in both high yield and purity, while a step for isolation of the intermediate salt of a compound of formula X forming in step ii) can be omitted. As to the advantages of said solvate form of a salt of compound of formula XI, it is referred to the explanations for pathway A.

In a preferred embodiment of pathway B, in step i), the compound of formula X is provided in crude form or in isolated form, preferably in isolated form, more preferably in an isolated form wherein the compound of formula X has a purity of at least 90% measured by HPLC, even more preferably at least 95%, yet even more preferably at least 97% and in particular at least 98%. Since the starting material compound of formula X may be applied in crude form or in isolated form having a suitably selected purity, there is free choice concerning the nature of compound of formula X provided in step i). While applying the crude form of compound of formula X as the starting material provides for reducing both work-up and purification efforts in a process for preparing a crystalline solvate form of a salt of compound of formula XI, applying a compound of formula X in isolated form as the starting material provides for a surprisingly high purity of a crystalline solvate form of a salt of compound of formula XI

Preferably, in step i) of pathway B, a compound of formula X' is provided 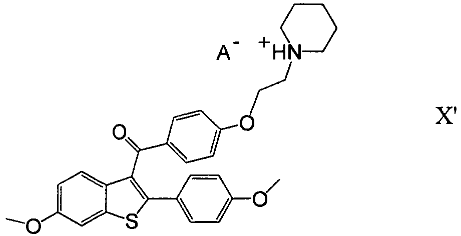 wherein A⁻ is Cl⁻ or Br⁻, preferably Br⁻. More preferably, the compound of formula X' is in crystalline form, even more preferably a compound of formula X' wherein A⁻ is Cl⁻ and whose X-ray diffraction (XRD) pattern has at least signals at the following 2θ values (±0.5° respectively):

| | | | | | | | | | | |
|---|---|---|---|---|---|---|---|---|---|---|
| **2theta[°]** | 11.41 | 13.07 | 16.74 | 19.27 | 19.39 | 21.56 | 23.42 | 24 | 24.63 | 26.05 |

; or a compound of formula X' wherein A⁻ is Br⁻ and whose X-ray diffraction (XRD) pattern has at least signals at the following 2θ values (±0.5° respectively):

| | | | | | | | | | | |
|---|---|---|---|---|---|---|---|---|---|---|
| **2theta[°]** | 7.41 | 13.05 | 16.64 | 19.07 | 19.22 | 21.45 | 23.31 | 24.34 | 25.81 | 26.62 |

In this way, particularly suitable starting materials are provided which further contribute to a exceptional high purity of the solvate obtained in step vi).

Preferably, in step ii) of pathway B, deprotection reaction for cleaving the -OR₁ groups is carried out as described above for the embodiment of Scheme 1.

According to another embodiment of pathway B, the anion of a salt of a compound of formula XI obtained in step ii) is exchanged by another anion prior to step iii), preferably by an anion of an acid forming a pharmaceutically acceptable salt as defined in item (47). In this way, a particularly advantageous conversion of a salt of a compound of formula XI obtained in step ii) to a pharmaceutically acceptable salt of a compound of formula XI by exchange of the anion is provided.

According to one embodiment of pathways A and B, the crystalline solvate form of a salt of a compound of formula XI is based on a pharmaceutically acceptable salt of a compound of formula XI, preferably the acid forming said pharmaceutically acceptable salt of a compound of formula XI whereupon said solvate form is based is selected from the group of organic or inorganic acids, preferably from the group consisting of oxalic acid, succinic acid, lactic acid, malonic acid, HCl, HBr and H₂SO₄, more preferably HCl, HBr and H₂SO₄, even more preferably HCl and HBr, and in particular HBr.

Preferably, in the process of pathways A and B, said first solvent is incorporated into the salt of compound of formula XI in an amount of percent by weight which is lower than the amount of percent by weight of the second solvent. More preferably, the crystalline solvate form of a salt of compound of formula XI comprises said first solvent in an amount of about 0.1 to 8 percent by weight and said second solvent in an amount of about 3 to 15 percent by weight relative to the solvate form of a salt of compound of formula XI, preferably compound of formula XI provided in step a) comprises said first solvent in an amount of about 1 to 5 percent by weight and said second solvent in an amount of about 7.7 to 10 percent by weight relative to the solvate form of a salt of compound of formula XI. Even more preferably, said first solvent is an alcohol and said second solvent is a halocarbon, preferably said first solvent is a C₁-C₄-alcohol and said second solvent is a C₁-C₁₀-halocarbon optionally comprising a benzene ring, more preferably said first solvent is methanol or ethanol and said second solvent is methylene chloride, chloroform or chlorobenzene, in particular said first solvent is methanol and said second solvent is methylene chloride. In this way, the chemical and/or physical properties of the crystalline solvate form of a compound of formula XI can be suitably set in view of procedural conditions in a process for preparing pharmaceutically active agents such as raloxifene or derivatives thereof.

Further according to the preferred embodiment illustrated by Scheme 3, a non-solvated pharmaceutically acceptable salt of a compound of formula XI is prepared by a process starting from a crystalline solvate form of a compound of formula XI. Said process comprises the steps of:
i) providing a crystalline solvate form of a pharmaceutically acceptable salt of a compound of formula XI 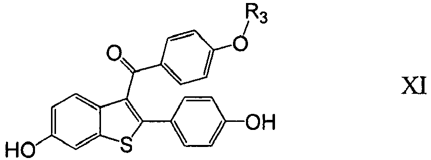 wherein R₃ is 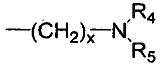 , wherein x = 1 to 20, preferably 1 to 15, more preferably 2 to 10, and in particular 2,
   and R₄ and R₅ independently from each other represent substituted or unsubstituted alkyl, substituted or unsubstituted aryl, substituted or unsubstituted arylalkyl, or R₄ and R₅ cooperatively with the N-atom form a part of a 5- to 10-membered ring, preferably a 5- to 7-membered ring, more preferably a 5- or 6-membered ring, in particular a 6-membered ring,
ii) dissolving said crystalline solvate form provided in step i) in an organic solvent, wherein said organic solvent forms an azeotrope with at least one solvent incorporated in said solvate form,
iii) optionally seeding the mixture resulting from step ii) in order to induce crystallisation,
iv) allowing the pharmaceutically acceptable salt of compound of formula XI to precipitate, preferably to crystallize, optionally under agitation, and
v) separating the pharmaceutically acceptable salt of a compound of formula XI, preferably separation is carried out by filtration.

The above mentioned aspect provides a process for preparing a non-solvated pharmaceutically acceptable salt of a compound of formula XI, wherein the crystalline solvate form of a pharmaceutically acceptable salt of a compound of formula XI is converted to the non-solvated form of the respective pharmaceutically acceptable salt in both high yields and high purity, while the obtained non-solvate form of the pharmaceutically acceptable salt of a compound of formula XI is substantially free of organic solvent(s) incorporated in the starting material in solvate form.

Preferably, the crystalline solvate form of a pharmaceutically acceptable salt of a compound of formula XI comprises a first solvent and a second solvent different from the first solvent, wherein said first solvent and said second solvent are incorporated in the pharmaceutically acceptable salt of a compound of formula XI. Surprisingly, even though said crystalline form comprises two organic solvents, said crystalline solvate form provides for a non-solvated form of the respective pharmaceutically acceptable salt in both particularly high yields and exceptional high purity, while the obtained non-solvate form of the pharmaceutically acceptable salt of a compound of formula XI is substantially free of both first solvent and second solvent incorporated into the starting material in solvate form.

More preferably, said first solvent is incorporated into the salt of compound of formula XI in an amount of percent by weight which is lower than the amount of percent by weight of the second solvent. Even more preferably, the crystalline solvate form of a salt of compound of formula XI comprises said first solvent and said second solvent in the following amounts (given in percent by weight relative to the solvate form of a salt of compound of formula XI): about 0.1 to 8 percent by weight of the first solvent and about 3 to 15 percent by weight of the second solvent, preferably about 1 to 5 percent by weight of the first solvent and about 7.7 to 10 percent by weight of the second solvent.

Furthermore, preferably said first solvent is an alcohol and said second solvent is a hydrocarbon or halocarbon. More preferably, said first organic solvent is a C₁-C₄-alcohol and said second organic solvent is a C₆-C₁₀-hydrocarbon comprising a benzene moiety or a C₁-C₁₀-halocarbon optionally comprising a benzene moiety, even more preferably said first solvent is methanol or ethanol and said second solvent is toluene, methylene chloride, chloroform or chlorobenzene, even more preferably said first solvent is methanol and said second solvent is toluene, methylene chloride or chloroform, and in particular, said first solvent is methanol and said second solvent is methylene chloride.

According to one embodiment, the azeotrope formed in step ii) forms under normal or reduced pressure, more preferably under reduced pressure. Even more preferably, solvent(s) incorporated into said solvate form of a pharmaceutically acceptable salt of a compound of formula XI is/are removed by azeotropic distillation of the mixture resulting from step ii), preferably said azeotropic distillation is carried out at reduced pressure.

The organic solvent applied in step ii) is preferably an alcohol, more preferably a water-miscible alcohol, even more preferably a C₁-C₄-alcohol, yet even more preferably methanol or ethanol, and in particular methanol.

According to a further embodiment, said organic solvent of step ii) comprises a predetermined amount of water, preferably said amount of water is predetermined by a volume ratio of water and organic solvent of 0 to 0.5, preferably 0.24 to 0.44, more preferably 0.27 to 0.40, and in particular 0.29 to 0.38. Preferably, said water is potable water, preferably deionized water, more preferably reverse osmosis water.

According to a further embodiment, in step ii), the resulting mixture is heated prior to step iii), preferably heating is under reflux conditions.

In another preferred embodiment, activated charcoal is added in step ii) and said charcoal is separated from the mixture resulting from step ii) prior to step iii), preferably separation is carried out by filtration.

Preferably, said pharmaceutically acceptable salt of a compound of formula XI obtained in step v) has a purity of > 98% measured by HPLC, more preferably > 99%, even more preferably > 99.2%, yet even more preferably > 99.4%, and in particular > 99.6%.

The following examples are merely illustrative of the present invention and they should not be considered as limiting the scope of the invention in any way. The examples and modifications or other equivalents thereof will become apparent to those versed in the art in the light of the present entire disclosure.

### Examples

### Methods

**Heavy metals.** According to USP (231, method II). **Sulphated ash.** According to Ph. Eur. (2.4.14). **IR**. FTIR-spectrometer Spectrum one (Perkin-Elmer); according to Ph. Eur. (2.2.24, KBr). DSC. DSC822 (Mettler), inert gas: nitrogen, temperature program from 30°C to 400°C /heating rate: 10°C/min. **Water content.** Titrando 835 (Metrohm) with double-Pt-electrode; Karl-Fisher-titration (abbreviated as KFT in the following Examples). **MP.** B545 (Büchi). **Residual solvents.** Gas chromatograph 6890 (Agilent) with head space sampler; head space gas chromatography, column DB-624: 30 m length, 0.32 mm ID, 1,8 µm film thickness, temperature program from 40°C to 250°C. **Titration**. Titrino 716 / Titrando 835 (Metrohm); with sodium hydroxide solution and solvotrode, with silver nitrate solution and silver titrode. **NMR**. Bruker AVANCE 300 spectrometer at 301 K. **XRPD**. transmission diffractometer STADI P (STOE & Cie., Darmstadt, Germany) using Ge-monochromated CuKa₁ radiation (1.5406 A) in Debye-Scherrer-geometry; the most intense 2θ-peaks of the obtained X-ray diffractograms were determined by the analysis software "Match! Version 1.9d, Crystal Impact, 2003-2009".

**Gas Chromatography**. The analysis was carried out by using an GC-system consisting of a split/splitless-injector, an autosampler, column oven and Fl-detector (Agilent), using a Rxi-5-MS column (25 m x 0.25 mm ID, 0.5 µm film thickness). Injection volume 1 µl. Heating rate 10 K/min.

| | |
|---|---|
| Hydrogen | 40 ml/min |
| Air | 450 ml/min |
| Make-up gas (N₂) | 25 ml/min |

**A) Compounds III', IV'**. A sample (50 mg) was dissolved in 1 mL of dimethylformamide and assayed by gas chromatography

| | |
|---|---|
| Carrier gas, flow rate | Nitrogen: 1.4 mL/min, constant flow |
| Split flow rate | 28 mL/min |
| Injector temperature | 300°C |
| Oven temperature program: | |
| Start temperature/holding time | 50°C / 0 min |
| Final temperature/holding time | 330°C / 20 min |
| Duration of analysis | 45 min |
| Detector / temperature | 300°C |

**B) Compounds V'**. A sample (50 mg) was dissolved in 1 mL of toluene and assayed by gas chromatography.

| | |
|---|---|
| Carrier gas, flow rate | Nitrogen: 1.4 mUm in, constant flow |
| Split flow rate | 28 mL/min |
| Injector temperature | 300°C |
| Oven temperature program: | |
| Start temperature/holding time | 50°C / 0 min |
| Final temperature/holding time | 330°C / 20 min |
| Duration of analysis | 45 min |
| Detector / temperature | 300°C |

**D) Compounds VII'**. A sample (50 mg) was dissolved in 1.5 mL of toluene and assayed by gas chromatography.

| | |
|---|---|
| Carrier gas, flow rate | Nitrogen: 1.0 mL/min, constant flow |
| Split flow rate | 20 mL/min |
| Injector temperature | 330°C |
| Oven temperature program: | |
| Start temperature/holding time | 50°C / 0 min |
| Final temperature/holding time | 330°C / 60 min |
| Duration of analysis | 88 min |
| Detector / temperature | 330°C |

**GC-MS.** The analysis was carried out by using an GC-MS-system consisting of a split/splitless-injector, an autosampler, column oven and MSD-detector (Agilent), using a Rxi-5-MS column (25 m x 0.25 mm ID, 0.5 µm film thickness). Carrier gas, flow rate helium: 1.9 mL/min, constant flow. Split flow rate 38 mL/min. Oven temperature program: Start temperature/holding time 50°C / 0 min. Heating rate 10 K/min. Injection volume 1 µl.

| | |
|---|---|
| AUX / temperature | 350°C |
| MS Source / temperature | 230°C |
| MS Quad / temperature | 150°C |
| Scan masses | from 15 to 550 |
| Threshold | 25 |

**A) Compounds III'.** A sample (50 mg) was dissolved in 1 mL of dimethylformamide and subjected to GC/MS analysis.

| | |
|---|---|
| Injector temperature | 330°C |
| Final temperature/holding time | 330°C / 50 min |
| Duration of analysis | 78 min |

**B) Compounds IV'**. A sample (50 mg) was dissolved in 1 mL of dimethylformamide and subjected to GC/MS analysis.

| | |
|---|---|
| Injector temperature | 300°C |
| Final temperature/holding time | 300°C / 20 min |
| Duration of analysis | 45 min |

**C) Compounds V'**. A sample (50 mg) was dissolved in 1 mL of toluene and subjected to GC/MS analysis.

| | |
|---|---|
| lnjector temperature | 300°C |
| Final temperature/holding time | 300°C / 20 min |
| Duration of analysis | 45 min |

**D) Compounds VII'**. A sample (50 mg) was dissolved in 1 mL of toluene and subjected to GC/MS analysis.

| | |
|---|---|
| lnjector temperature | 330°C |
| Final temperature/holding time | 330°C / 50 min |
| Duration of analysis | 78 min |

**High Performance Liquid Chromatography.** The analysis was carried out by using an HPLC-system consisting of a gradient pump, an autosampler, column thermostat and UV-detector (Agilent or Dionex).
**A) CompoundsIII',IV', V'**. A sample (25 mg) was weighed into a 50-mL volumetric flask, and dissolved in 25 mL acetonitrile. Water was added to 50.0 mL. An aliquot of this solution (10 µL) was assayed by high performance liquid chromatography, using a YMC Pack Pro C18 RS column (25 cm x 4.6 mm lD, 5 µm particle) maintained at 40°C and UV detection (220 nm). Flow rate: 1.0 ml/min. Gradient solvent system (A: 20 mM KH₂PO₄ buffer, pH 3.0; B: acetonitrile):

| time (min) | A (%) | B (%) |
|---|---|---|
| 0 | 90 | 10 |
| 20 | 10 | 90 |
| 22 | 10 | 90 |
| 23 | 90 | 10 |
| 30 | 90 | 10 |

**B) Compounds VII'**. A sample (25 mg) was weighed into a 50-mL volumetric flask, and dissolved in acetonitrile. An aliquot of this solution (10 µL) was assayed by high performance liquid chromatography, using a YMC Pack Pro C18 RS column (25 cm x 4.6 mm lD, 5 µm particle) maintained at 40°C and UV detection (220 nm). Flow rate: 1.0 ml/min. Gradient solvent system (A: 20 mM KH₂PO₄ buffer, pH 3.0; B: acetonitrile):

| time (min) | A (%) | B (%) |
|---|---|---|
| 0 | 50 | 50 |
| 20 | 5 | 95 |
| 25 | 5 | 95 |
| 25.3 | 50 | 50 |
| 30 | 50 | 50 |

**C) Compounds VIII', X'.** A sample (25 mg) was weighed into a 50-mL volumetric flask, and dissolved in 25 mL acetonitrile. Water was added to 50.0 mL. An aliquot of this solution (10 µL) was assayed by high performance liquid chromatography, using a YMC Pack Pro C18 RS column (25 cm x 4.6 mm lD, 5 µm particle) maintained at 40°C and UV detection (290 nm). Flow rate:1.0 ml/min. Gradient solvent system (A: 10 mM ammonium formate buffer, pH 3.0; B: acetonitrile):

| time (min) | A (%) | B (%) |
|---|---|---|
| 0 | 90 | 10 |
| 20 | 10 | 90 |
| 22 | 10 | 90 |
| 23 | 90 | 10 |
| 30 | 90 | 10 |

**LC-MS**. A sample (5 mg) was dissolved in 10 mL acetonitrile and diluted to 25.0 mL with acetonitrile / water (1 : 1). An aliquot of this solution (5 µL) was assayed by high performance liquid chromatography, using a Purospher STAR RP18 endcapped column (12.5 cm × 2 mm lD, 5 µm particle) maintained at 25°C and MS detection in ESl positive mode. Flow rate: 0.3 ml/min. Gradient solvent system (A: water with 0.05% formic acid; B: acetonitrile with 0.05% formic acid).

| time (min) | A (%) | B (%) |
|---|---|---|
| 0 | 80 | 20 |
| 20 | 0 | 100 |
| 25 | 0 | 100 |
| 26 | 80 | 20 |
| 35 | 80 | 20 |

**D) Compounds XI'.** A sample (25 mg) was weighed into a 50-mL volumetric flask, and dissolved in 25 mL acetonitrile and 20 mL of water. Water was added to 50.0 mL. An aliquot of this solution (10 µL) was assayed by high performance liquid chromatography, using a YMC Pack Pro C18 RS column (25 cm x 4.6 mm lD, 5 µm particle) maintained at 40°C and UV detection (290 nm). Flow rate: 1.0 ml/min. Gradient solvent system (A: water with 0.05% formic acid; B: acetonitrile).

| time (min) | A (%) | B (%) |
|---|---|---|
| 0 | 90 | 10 |
| 20 | 10 | 90 |
| 22 | 10 | 90 |
| 23 | 90 | 10 |
| 30 | 90 | 10 |

**(LC-MS)**. A sample (5 mg) was disssolved to 100.0 mL in a mixture of acetonitrile / water (1:1). An aliquot of this solution (5 µL) was assayed by high performance liquid chromatography, using a Purospher STAR RP18 endcapped column (12.5 cm x 2 mm lD, 5 µm particle) maintained at 25°C and MS detection in ESI positive mode (abbreviated as ESI(+) in the following Examples). Flow rate: 0.3 ml/min. (A: water with 0.05% formic acid; B: acetonitrile with 0.05% formic acid).

| time (min) | A (%) | B (%) |
|---|---|---|
| 0 | 80 | 20 |
| 20 | 0 | 100 |
| 25 | 0 | 100 |
| 26 | 80 | 20 |
| 35 | 80 | 20 |

### Examples 1-4: Preparation of compounds of formula III'

### Example 1

### 4-Benzenesulfonyloxy-benzoic acid ethyl ester

Aq. K₂CO₃ (2.5 M, 80.6 mL) was added at 13 - 18 °C to a mixture of 4-hydroxy-benzoic acid ethyl ester (332.5 g, 2 mol), benzenesulfonyl chloride (365.6 g, 2.07 mol), tetra-n-butylammonium hydrogen sulphate (5.0 g, 0.03 mol), ethyl acetate (500 mL), and water (100 mL) with stirring. The mixture was heated to 35 °C with stirring and treated with aq. K₂CO₃ (2.5 M, 367 mL) over a period of 1 h. The mixture was heated at reflux for 2 h and treated with water (200 mL) with stirring. The aqueous phase was separated and the organic phase was washed with water (150 mL). The organic phase was evaporated to dryness at 45 - 75 °C and the residue was dried under reduced pressure to obtain a colourless or yellowish liquid. Yield: 613.3 g (100 %). **Purity**: 100.0 % (HPLC). ¹**H NMR** (300 MHz, DMSO-d₆, ppm): δ 7.9 - 7.8 (m, 5 H, arom.), 7.7 (m, 2 H, arom.), 7.2 (d, 2 H, arom.), 4.3 (q, 2 H, R₂CH₂), 1.3 (t, 3 H, RCH₃); **¹³C-{H}-NMR** (75 MHz, DMSO-d₆, ppm): δ 164.6 (>C=O), 152.2 (R₂C-O-R, arom.), 135.2 (=CH-), 134.1 (=C<), 131.1 (=CH-), 129.8 (=CH-), 128.9 (=C<), 128.2 (=CH-), 122.3 (=CH-), 61.0 (>CH₂), 14.0 (-CH₃)). IR (KBr, cm⁻¹): 1718 (v_{c=o}), 1602, 1499, 1450, 1380, 1278, 1202, 1178, 1154, 1093, 1018, 871, 746, 712, 697, 583, 568. **GC/MS** (El, RT; 22.119 min): m/z = 306 [M]⁺.

### Example 2

### 4-Benzenesulfonyloxy-benzoic acid methyl ester

4-Benzenesulfonyloxy-benzoic acid methyl ester was obtained in a similar manner as 4-benzenesulfonyloxy-benzoic acid ethyl ester by employing 4-hydroxy-benzoic acid methyl ester as starting material. **Purity**: 99.61 % (HPLC). **¹H NMR** (300 MHz, DMSO-d₆, ppm): δ.01 - 7.92 (d, 2 H, arom.), 7.91 - 7.78 (m, 3 H, arom.), 7.74 - 7.61 (m, 2 H, arom.), 7.19 (d, 2 H, arom.), 3.83 (s, 3 H, -OCH₃)- **¹³C-{H}-NMR** (75 MHz, DMSO-d₆, ppm): δ65.0 (>C=O), 152.21 (R₂C-O-R, arom.), 135.16 (=CH-), 133.98 (=CH<), 131.15 (=CH-), 129.81 (=CH-), 128.62 (=CH<), 128.11 (=CH-), 122.29 (=CH-), 52.25 (-CH₃). IR (KBr, cm⁻¹): 3000, 2952, 1728 (v_{c=o}), 1607, 1500, 1542, 1436, 1415, 1373, 1309, 1281, 1203, 1182, 1161, 1092, 1017, 998, 962, 878, 856, 827, 773, 751, 712, 687, 646, 625, 613, 573. **GC/MS** (El, RT; 22.1 min): m/z = 292 [M]⁺.

### Example 3

### 4-Methanesulfonyloxy-benzoic acid ethyl ester

4-Methanesulfonyloxy-benzoic acid ethyl ester was obtained in a similar manner as 4-benzenesulfonyloxy-benzoic acid ethyl ester by employing methanesulfonyl chloride as starting material. **¹H NMR** (300 MHz, DMSO-d₆, ppm): δ 8.06 (d, 2 H, arom.), 7.49 (d, 2 H, arom.), 4.33 (q, 2 H, -CH₂CH₃), 3.45 (s, 3 H, -CH₃) 1.32 (t, 3 H, -CH₂CH₃). **¹³C-{H}-NMR** (75 MHz, DMSO-d₆, ppm): δ 164.71 (>C=O), 152.38 (R₂COSO₂CH₃), 131.21 (=CH-), 128.75 (=C<), 122.41 (=CH-), 60.96 (>CH₂), 37.71 (-CH₃), 14.04 (-CH₃). lR (KBr, cm¹): 2943, 1717 (v_{c=o}), 1675, 1603, 1500, 1415, 1373, 1334, 1280, 1205, 1171, 1156, 1112, 1096, 1019, 967, 879, 793, 774, 713, 698, 628, 547, 529, 521. **GC/MS** (El, RT; 17.9 min): m/z = 244 [M]⁺.

### Example 4

### 4-Toluene-4-sulfonyloxy-benzoic acid ethyl ester

4-Toluene-4-sulfonyloxy-benzoic acid ethyl ester was obtained in a similar manner as 4-benzenesulfonyloxy-benzoic acid ethyl ester by employing 4-methyl-benzenesulfonyl chloride as starting material. **Purity**: 97.14 % (HPLC). **Mp**.: 58.6 - 59.9 °C. DSC: Peak at 61.88 °C.**¹H NMR** (300 MHz, DMSO-d₆, ppm): δ 7.95 (d, 2 H, arom.), 7.75 (d, 2 H, arom.), 7.46 (d, 2 H, arom.), 7.18 (d, 2 H, arom.), 4.29 (q, 2 H, -OCH₂CH₃), 2.41 (s, 3 H, -CH₃), 1.29 (t, 3 H, -OCH₂CH₃). **¹³C-{H}-NMR** (75 MHz, DMSO-d₆, ppm): δ164.57 (>C=O), 152.27 (R₂COSO₂CH₃), 146.0 (=CH<), 131.09 (=CH-), 130.23 (=CH-),128.80 (=CH<), 128.14 (=CH-), 122.26 (=CH-), 60.94 (-OCH₂CH₃), 21.08 (-CH₃), 13.98 (-OCH₂CH₃). **lR** (KBr, cm⁻¹): 2981, 1720 (v_{c=o}), 1595, 1501, 1476, 1454, 1415, 1376, 1293, 1212, 1199, 1177, 1159, 1121, 1092, 1024, 875, .843, 818, 777, 738, 694, 671, 619, 573, 551, 510. **GC/MS** (El, RT; 23.7 min): m/z = 320 [M]⁺.

### Example 5-7: Preparation of compounds of formula IV'

### Example 5

### Benzenesulfonyloxy-benzoic acid

### a) Preparation by using formic acid and sulphuric acid as an acid for ester cleavage:

A stirred mixture of 4-benzenesulfonyloxy-benzoic acid ethyl ester (310.0 g, 1.0 mol), prepared as described in example 1, formic acid (85 %, 450 mL), and sulphuric acid (96 %, 6.0 mL) was heated to distillation. 23 mL solvents were distilled off over a period of approximately 1 h and formic acid (85 %, 23 mL) was added. This procedure was repeated 16 times. The reaction mixture was cooled and water (150 mL) was added at 90 °C. After stirring for 1 h at 20 °C the precipitate was filtered off, washed with water (188 mL) and ethyl acetate (125 mL) and dried under reduced pressure at 55 °C. Yield: 252.4 g (91 %).
For further, optional purification, 241.5 g of the product obtained above were suspended in ethyl acetate (1.69 L) and heated to reflux. The solution was filtered and the filter rinsed with ethyl acetate (20 mL). The combined clear filtrates were cooled to 3-5 °C with stirring and stirring was continued for further 3 h. The solid was filtered off, washed with ethyl acetate (2 x 80 mL), and dried under reduced pressure at 50 °C. Yield: 200.7 g (83 %).
**Purity**: 97.09 % (HPLC). **DSC**: peak at 173.5 °C.**¹H NMR** (300 MHz, DMSO-d₆, ppm): δ3.2 (s, 1 H, -OH), 8.0 - 7.8 (m, 5 H, arom.), 7.7 (m, 2 H, arom.), 7.2 (d, 2 H, arom.); **¹³C-{H}-NMR** (75 MHz, DMSO-d₆, ppm): δ 166.1 (>C=O), 152.0 (R₂C-O-R, arom.), 135.2 (=CH-), 134.1 (=C<), 131.3 (=CH-), 129.8 (=CH-), 128.1 (=C<), 128.1 (=CH-), 122.1 (=CH-). **lR** (KBr, cm-¹): 2552, 1684 (v_{c=o}), 1600, 1498, 1450, 1427, 1355, 1290, 1200, 1169, 1090, 1016, 953, 868, 848, 822, 806, 784, 764, 740, 720, 694, 685, 642, 588, 540. **GC/MS** (El, RT; 22.082 min): m/z = 278 [M]⁺. **XRPD:** the X-ray diffractogram exhibits signals at the following 2θ values:

| **2theta[°]** | **I/I₀** |
|---|---|
| 15.18 | 106.23 |
| 16.12 | 1000.00 |
| 16.31 | 98.84 |
| 16.36 | 104.02 |
| 16.45 | 67.93 |
| 16.63 | 343.92 |
| 18.39 | 76.71 |
| 19.71 | 65.40 |
| 20.67 | 215.13 |
| 21.27 | 245.18 |
| 25.24 | 427.99 |
| 26.78 | 116.27 |
| 27.15 | 222.73 |
| 27.68 | 393.76 |

### b) Preparation by using acetic acid as an acid for ester cleavage:

Benzenesulfonyloxy-benzoic acid was obtained in a similar manner as described above by employing acetic acid as solvent. Yield: 93 %. **Purity**: 99.99 % (HPLC). **DSC**: peak at 173.13 °C.).

### c) Preparation by using 4-benzenesulfonyloxy-benzoic acid methyl ester as starting material:

Benzenesulfonyloxy-benzoic acid was also obtained in a similar manner described above by employing 4-benzenesulfonyloxy-benzoic acid methyl ester as starting material. Yield: 93 %. **Purity**: 99.98 % (HPLC). **Mp**.: 174.8 °C.

### Example 6

### 4-Methanesulfonyloxy-benzoic acid

4-Methanesulfonyloxy-benzoic acid was obtained similar to example 5 item b, by employing 4-methanesulfonyloxy-benzoic acid ethyl ester as starting material. Yield: 74 %. **Purity**: 99.77 % (HPLC). **Mp**.: 226.1 °C. **DSC**: Peak at 225.58 °C. **¹H NMR** (300 MHz, DMSO-d₆, ppm): δ3.16 (bs, 1 H, -OH), 8.05 (d, 2 H, arom.), 7.47 (d, 2 H, arom.), 3.44 (s, 3 H, -CH₃).
**¹³C-{H}-NMR** (75 MHz, DMSO-d₆, ppm): δ 166.30 (>C=O), 152.21 (R₂COSO₂CH₃), 131.41 (=CH-), 129.73 (=C<), 122.27 (=CH-), 37.71 (-CH₃). **IR** (KBr, cm⁻¹): 3036, 1689 (v_{c=o}), 1602, 1502, 1429, 1331, 1290, 1197, 1169, 1150, 1129, 1099, 1017, 982, 938, 864, 830, 789, 772, 718, 691, 626, 543, 509. **GC/MS** (El, RT; 18.000 min): m/z = 216 [M]⁺. XRPD: the X-ray diffractogram exhibits signals at the following 2θ values:

| **2theta[°]** | **I/I₀** |
|---|---|
| 12.37 | 161.87 |
| 14.62 | 1000 |
| 16.52 | 105.99 |
| 17.22 | 413.93 |
| 18.29 | 671.88 |
| 19.71 | 93.91 |
| 21.39 | 515.14 |
| 22.02 | 351.87 |
| 22.11 | 432.26 |
| 23.29 | 384.7 |
| 23.47 | 75.14 |
| 24.3 | 111.61 |
| 24.62 | 146.86 |
| 24.94 | 48.67 |
| 25.3 | 112.52 |
| 25.75 | 205.97 |
| 26 | 356.88 |
| 26.71 | 55.31 |
| 27.62 | 103.63 |
| 27.73 | 90.94 |
| 29.53 | 132.11 |
| 30.17 | 49.55 |
| 31.35 | 91.22 |
| 31.89 | 113.83 |
| 33.41 | 44.25 |
| 34.83 | 87.83 |
| 35.66 | 52.49 |
| 37.19 | 84.97 |
| 41.73 | 47.42 |
| 44.99 | 44.61 |

### Example 7

### 4-Toluene-4-sulfonyloxy-benzoic acid

4-Toluene-4-sulfonyloxy-benzoic acid was obtained in a similar manner as described in example 5 item b, employing 4-toluene-4-sulfonyloxy-benzoic acid ethyl ester as starting material. **Purity:** 99.61 % (HPLC). **DSC**: peak at 173.91 °C.**¹H NMR** (300 MHz, DMSO-d₆, ppm): δ3.15 (bs, 1 H, -OH), 7.94 (d, 2 H, arom.), 7.75 (d, 2 H, arom.), 7.46 (d, 2 H, arom.), 7.15 (d, 2 H, arom.), 2.40 (s, 3 H, -CH₃).**¹³C-{H}-NMR** (75 MHz, DMSO-d₆, ppm): δ 166.16 (>C=O), 152.09 (R₂COSO₂R), 145.98 (=C<), 131.26 (=CH-), 131.21 (=C<), 130.24 (=CH-), 129.77 (=C<), 128.16 (=CH-), 122.10 (=CH-), 21.11 (-CH₃). **lR** (KBr, cm⁻¹): 2990, 1689 (v_{c=o}), 1602, 1502, 1434, 1367, 1293, 1203, 1174, 1155, 1093, 1017, 943, 860, 813, 778, 735, 692, 670, 645, 617, 569, 551. **GC/MS** (El, RT; 23.800 min): m/z = 292 [M]⁺. XRPD: the X-ray diffractogram exhibits signals at the following 2θ values:

| **2theta [°]** | **I/I**₀ |
|---|---|
| 6.87 | 133.88 |
| 13.22 | 523.25 |
| 13.47 | 165.35 |
| 13.81 | 103.42 |
| 16.37 | 64.61 |
| 17.4 | 55.61 |
| 17.61 | 198.3 |
| 17.87 | 1000 |
| 18.47 | 119.97 |
| 18.98 | 123.47 |
| 20.62 | 570.98 |
| 21.26 | 272.35 |
| 22.68 | 289.29 |
| 22.94 | 81.7 |
| 23.7 | 382.18 |
| 24.13 | 55.77 |
| 26.64 | 168.41 |
| 26.83 | 108.72 |
| 27.13 | 621.84 |
| 27.3 | 88.9 |
| 27.42 | 144.2 |
| 27.73 | 64.03 |
| 28.97 | 74.1 |
| 30.15 | 65.31 |
| 33.53 | 66.59 |

### Example 8: Preparation of compounds of formula V'

### Benzenesulfonic acid 4-chlorocarbonyl-phenyl ester

### a) Preparation by using DMF as a catalyst and toluene as the solvent:

A mixture of benzenesulfonyloxy-benzoic acid (100 g, 0.36 mol), obtained according to example 5 item a, toluene (300 mL), and DMF (0.028 mL) was heated to 63-68 °C with stirring. SOCl₂ (43.7 g) was added slowly (40 min) and stirring was continued for further 5 h at 65 - 70 °C. The solvent was distilled off under reduced pressure at 40-53 °C and the residue was dried in vacuum at 50 °C. Yield: 108.8 g. **Purity**: 98.57 % (HPLC). **DSC**: peak at 41.45 °C.**¹H NMR** (300 MHz, CDCl₃, ppm): δ.1 - 7.7 (m, 5 H, arom.), 7.6 (m, 2 H, arom.), 7.2 (d, 2 H, arom); **¹³C-{H}-NMR** (75 MHz, DMSO-d₆, ppm): δ67.1 (>C=O), 154.6 (R₂COSO₂R), 135.2 (=C<), 134.7 (=CH-), 133.1 (=CH-), 131.9 (=C<), 129.4 (=CH-), 128.4 (=CH-), 122.8 (=CH-)). **lR** (KBr, cm⁻¹): 1780 (v_{c=o}), 1748, 1597, 1496, 1450, 1411, 1299, 1200, 1183, 1157, 1093, 1015, 866, 754, 733, 707, 686, 629, 581, 518. **GC/MS** (El, RT; 21.360 min): m/z = 296 [M]⁺.

### b) Preparation by using DMF as a catalyst and chlorobenzene as the solvent:

Benzenesulfonic acid 4-chlorocarbonyl-phenyl ester was obtained in a similar manner as described in example 8 item a, employing chlorobenzene as the solvent.

### c) Preparation by using dimethylamino)pyridine (DMAP) as a catalyst and chlorobenzene as the solvent:

A flask was charged with benzenesulfonyloxy-benzoic acid (50.0 g, calcd. dry, 180 mmol) and chlorobenzene (150 mL). 50 mL of chlorobenzene were distilled off under reduced pressure at 70 - 90 °C and DMAP (0.22 g, 1.8 mmol) was added. The suspension was slowly treated with SOCl₂ (21.8 g, 183.6 mmol, 1.5 h) at 75 - 85 °C with stirring. After the addition was completed stirring was continued for further 2 h at 85-91 °C to complete the reaction. The reaction mixture was subsequently flushed with nitrogen at 85 - 91 °C for approximately 35 min and allowed to reach room temperature to obtain benzenesulfonic acid 4-chlorocarbonyl-phenyl ester in practically quantitative yield.

### Methanesulfonic acid 4-chlorocarbonyl-phenyl ester

A flask was charged with 4-Methanesulfonyloxy-benzoic acid (10.0 g, 46.2 mmol), toluene (30 mL), and dimethyl formamide (0.02 mL). The mixture was heated to 62 °C with stirring and SOCl₂ (3.4 mL, 47.1 mmol) was added slowly (0.5 h). Stirring was continued for 5 h at approximately 60 °C to complete the reaction. The solvent was removed under reduced pressure at 50 °C. The residue was dissolved in toluene (10 mL) and the solvent was distilled off in vacuum at 50 °C to give the title compound. Yield: 10.61 g (45.2 mmol, 97.9 %). **Purity**: 95.41 % (GC). **Mp**.: 72.6 - 73.8 °C. **DSC**: peak at 74.31 °C. ¹**H NMR** (300 MHz, DMSO-d₆, ppm): δ 8.04 (d, 2 H, arom.), 7.46 (d, 2H, arom.), 3.43 (s, 3 H, -CH₃).
**¹³C-{H}-NMR** (75 MHz, DMSO-d₆, ppm): δ66.19 (>C=O), 152.27 (R₂COSO₂CH₃), 131.44 (=CH-), 129.69 (=C<-), 122.31 (=CH-), 37.78 (-CH₃). **IR** (KBr, cm⁻¹): 3033, 1733, 1597, 1496, 1411, 1365, 1207, 1182, 1157, 1014, 971, 871, 788, 709, 657, 631, 553, 523. **GC/MS** (El, RT; 17.058 min): m/z = 234 [M]⁺. **XRPD**: the X-ray diffractogram exhibits signals at the following 2θ values:

| **2theta[°]** | **I/I₀** |
|---|---|
| 8.62 | 87.74 |
| 15.75 | 522.96 |
| 17.34 | 321.79 |
| 19.07 | 729.08 |
| 19.96 | 375.87 |
| 20.1 | 254.83 |
| 21.76 | 171.23 |
| 21.97 | 438.63 |
| 23.03 | 1000 |
| 24.2 | 409.67 |
| 24.47 | 201.94 |
| 25.04 | 187.7 |
| 26.61 | 173.19 |
| 27.42 | 336.67 |
| 28.28 | 242.92 |
| 28.83 | 46.68 |
| 30.63 | 173.43 |
| 30.82 | 90.03 |
| 31.84 | 72.59 |
| 32.46 | 126.9 |
| 33.04 | 46.99 |
| 33.77 | 99.68 |
| 34.1 | 140.73 |
| 34.48 | 110.86 |
| 34.7 | 87.28 |
| 35.12 | 202.5 |
| 38.41 | 66.08 |
| 42.55 | 71.8 |
| 46.63 | 62.36 |
| 53.82 | 62.07 |

### Toluenesulfonic acid 4-chlorocarbonyl-phenyl ester

A flask was charged with 4-toluene-4-sulfonyloxy-benzoic acid (15.0 g, 51.3 mmol), toluene (45 mL), and dimethyl formamide (0.02 mL). The mixture was heated to 72 °C and SOCl₂ (3.9 mL, 53.9 mmol) was added slowly (0.5 h) at 63-72 °C with stirring. Stirring was continued for 5 h and the solvent was distilled off at 45 °C under reduced pressure. The residue was dissolved in toluene (15 mL) and the solution was taken to dryness at 50 °C in vacuo to obtain the title compound. Yield: 15.73 g (50.6 mmol, 98.7%). **Purity**: 97.33 % (GC).
**Mp.:** 75.0 -76.4 °C. **DSC**: 77.46 °C.**¹H NMR** (300 MHz, DMSO-d₆, ppm): δ7.93 (d, 2 H, arom.), 7.74 (d, 2 H, arom.), 7.45 (d, 2 H, arom.), 7.15 (d, 2 H, arom.), 2.40 (s, 3 H, -CH₃)₋ **¹³C-{H}-NMR** (75 MHz, DMSO-d₆, ppm): δ166.05 (>C=O), 152.12 (R₂COSO₂R), 146.01 (=C<), 131.28 (=CH-), 130.27 (=CH-), 129.70 (=C<), 128.18 (=CH-), 122.45 (=C<) 122.13 (=CH-), 21.15 (-CH₃). **IR** (KBr, cm⁻¹): 3070, 1778, 1748, 1597, 1497, 1412, 1382, 1298, 1202, 1176, 1162, 1092, 1018, 889, 864, 836, 817, 751, 721, 672, 651, 642, 618, 571, 551, 517. **GC/MS** (El, RT; 23.0 min): m/z = 310 [M]⁺. **XRPD**: the X-ray diffractogram exhibits signals at the following 2θ values:

| **2theta[°]** | **I/I₀** |
|---|---|
| 11.57 | 731.9 |
| 15.81 | 829.06 |
| 15.98 | 941.36 |
| 16.27 | 186.02 |
| 16.78 | 166.09 |
| 17.71 | 469.67 |
| 18.03 | 254.45 |
| 18.54 | 458.06 |
| 18.71 | 1000 |
| 18.84 | 272.79 |
| 19.41 | 819.05 |
| 19.67 | 699.35 |
| 20.51 | 566.26 |
| 21.98 | 179.07 |
| 22.7 | 741.37 |
| 23.28 | 255.92 |
| 23.68 | 912.37 |
| 24.21 | 747.61 |
| 25.36 | 440.32 |
| 25.7 | 141.67 |
| 27.13 | 381.23 |
| 27.63 | 481.15 |
| 27.97 | 621.58 |
| 29.57 | 212.4 |
| 30:69 | 165.92 |
| 30.74 | 156.07 |
| 30.81 | 146.35 |
| 30.87 | 173.63 |
| 37.91 | 174.83 |
| 38.23 | 165.32 |

### Example 9: Preparation of compounds of formula VII'

### Benzenesulfonicacid-4-[6-methoxy-2-(4-methoxy-phenyl)-benzo[b]thiophene-3-carbonyl]-phenyl ester

### a) Preparation by using Fe powder / Fe₂(SO₄)₃ x 5 H₂O as the catalyst and toluene as the solvent:

A mixture of 68.1 g of a solution of benzenesulfonic acid 4-chlorocarbonyl-phenyl ester (19.3 g, 65 mmol, calcd.) in toluene, prepared according to example 8 item a, toluene (107 mL), 6-methoxy-2-(4-methoxyphenyl)- benzothiophene (14.1 g, 52 mmol), iron (0.242 g, 4 mmol) and Fe₂(SO₄)₃ x 5 H₂O (1.898 g, 4 mmol) was heated to 106 °C to initiate the reaction. The dark coloured mixture was subsequently treated with 6-methoxy-2-(4-methoxy-phenyl)benzothiophene (126.48 g, 468 mmol) and toluene (107 mL). 612.7 g of a solution of benzenesulfonic acid 4-chlorocarbonyl-phenyl ester in toluene (173.57 g, 585 mmol, calcd.) were added slowly (5.5 h) at 100 -111 °C with stirring. The mixture was then cooled to 98 °C and flushed with nitrogen for 11 h at 99 - 104 °C. The reaction mixture was cooled to 62 - 66 °C, diluted with toluene (637 ml), treated with sodium hydroxide (aq., 1 N, 328 mL), and SiO₂ (11.57 g) with stirring. Stirring was continued for 30 min at 62 - 66 °C and insolubles were filtered off. The filter residue was rinsed with hot toluene (40 mL). The combined biphasic filtrates were separated. The aqueous phase was disposed. The organic phase was alkalized with sodium hydroxide (aq., 1 N, 328 mL) and heated at 60 - 64 °C for approximately 40 min with vigorous stirring. The phases were separated and the aqueous phase was disposed. The organic phase was washed with water (3 x 328 mL) and the solvent was distilled off at 62 - 66 °C in vacuo to yield 301.6 g of benzenesulfonic acid-4-[6-methoxy-2-(4-methoxy-phenyl)-benzo[b]thiophene-3-carbonyl]-phenyl ester.

For an optional purification, the residue was treated with acetone (240 mL) and heated to reflux with stirring. After 10 min the clear solution was cooled to 1 - 4 °C and stirring was continued for further 60 min. The suspension was filtered and the solid washed with cold acetone (3 x 40 mL) to give the title compound as crystalline solid. Yield: 183.2 g (66.4 %, calcd. dry). **Purity**: 96.74 % (HPLC). **XRPD** the XRD diffractorgram exhibitssignals at the following 28 values:

| **2theta[°]** | **I/I₀** |
|---|---|
| 8.81 | 208.7 |
| 11.15 | 182.15 |
| 12.93 | 585.91 |
| 13.77 | 600.06 |
| 14.42 | 210.08 |
| 14.84 | 77.14 |
| 16.16 | 691.36 |
| 17.07 | 112.41 |
| 18.73 | 239.91 |
| 19.51 | 124.75 |
| 19.63 | 119.23 |
| 20.09 | 156.51 |
| 20.22 | 130.03 |
| 21.46 | 307.24 |
| 22.26 | 1000 |
| 23.11 | 513.89 |
| 23.87 | 241.67 |
| 24.29 | 68.5 |
| 24.61 | 199.52 |
| 24.88 | 209.9 |
| 25.76 | 55.71 |
| 26.03 | 125.04 |
| 26.19 | 65.87 |
| 26.74 | 193.14 |
| 27.67 | 136.14 |
| 27.79 | 173.01 |
| 28.09 | 83.15 |
| 29.11 | 67.1 |
| 30.39 | 92.73 |
| 34.17 | 81.95 |

### b) Preparation by using Fe powder / Fe₂(SO₄)₃ × 5 H₂O as the catalyst and chlorobenzene as the solvent (0.32 eq):

Benzenesulfonicacid-4-[6-methoxy-2-(4-methoxy-phenyl)-benzo[b]thiophene-3-carbonyl]-phenyl ester was also obtained in a similar manner as described under example 9 item a), employing 0.32 eq. of iron ions as catalyst and chlorobenzene as the solvent.

### c) Preparation by using Fe powder / Fe₂(SO₄)₃ x 5 H₂O as the catalyst and toluene as the solvent employing 0.005 eq. of iron ions

Benzenesulfonicacid-4-[6-methoxy-2-(4-methoxy-phenyl)-benzo[b]thiophene-3-carbonyl]-phenyl ester was also obtained in a similar manner as described under example 9 item a), employing 0.005 eq. of iron ions as catalyst and toluene as the solvent.

### d) Preparation by using FeCl₃ as the catalyst and toluene as the solvent:

A mixture of benzenesulfonic acid 4-chlorocarbonyl-phenyl ester (26.7 g, 0.090 mol) prepared according to example 8 item b, toluene (135 mL), 6-methoxy-2-(4-methoxyphenyl)benzothiophene (19.5 g, 0.072 mol), and FeCl₃ (0.292 g, 0.002 mol) was stirred for 1.5 h at 23 - 24 °C and for 4.5 h at 56 - 63 °C. The mixture was treated with sodium hydroxide (aq., 1 N, 50 mL) and toluene (60 mL) with stirring. The mixture was filtered and the phases separated. The aqueos phase was discarded. The organic phase was washed with water (3 x 70 mL) and concentrated at 40 °C under reduced pressure. Methanol (75 mL) was added and the suspension was stirred for 0.5 h at 0 - 5 °C. The solid was separated, washed a mixture of toluene and methanol (1:1 v/v, 2 × 10 mL) and dried at 50 °C in vacuo. **DSC**: peak at 128.1 °C.

For optional purification, a sample (26.4 g) of the product obtained above was dissolved in hot toluene (45 mL). The turbid solution was filtered and the filter washed with toluene (3 mL). The combined filtrates were cooled to 4 °C with stirring and filtered. The solid was washed with toluene (2 × 10 mL) and dried at 50 °C under reduced pressure.

A sample (15.0 g) of the re-crystallized product was dissolved in hot acetone (35 mL). The clear solution was allowed to reach approx. 30 °C. The resulting suspension was cooled to 2-3 °C and stirring was continued for further 60 min. The solid was isolated, washed acetone (2 x 4 mL) and dried at 40 °C under reduced pressure. Yield: 41.4 % (calcd.). **Purity:** 99.86% (HPLC). **DSC**: peak at 134.2 °C. **¹H NMR** (300 MHz, CDCl₃, ppm): δ7.66 (m, 3 H, arom.), 7.60 (m, 3 H, arom.), 7.46 (m, 2 H, arom.), 7.27 (s, 1H, arom.), 7.17 (d, 2 H, arom.), 6.96 (d, 1 H, arom.), 6.76 (d, 2 H, arom.), 6.68 (d, 2 H, arom.), 3.83 (s, 3 H, -OCH₃), 3.71 (s, 3 H, -OCH₃); **¹³C-{H}-NMR** (75 MHz, CDCl₃, ppm): δ 192.46 (>C=O), 160.05, 157.90, 152.51, 145.49, 140.11, 136.53, 135.21 (=C<), 134.35 (=CH-), 133.58 (=C<), 131.41, 130.67 (=CH-), 129.66 (=C<), 129.16, 128.37 (=CH-), 125.73 (=C<), 124.21, 122.11, 115.05, 113.98, 104.57 (=CH-), 55.64, 55.27 (-OCH₃). **IR** (KBr, cm⁻¹): 3068, 3006, 2961, 2907, 2836, 1641 (v_{c=o}), 1606 (vₐᵣₒₘ.), 1530, 1493, 1477, 1450, 1438, 1409, 1376, 1355, 1327, 1290, 1253, 1218, 1204, 1154, 1132, 1093, 1046, 1030, 1015, 892, 863, 841, 785, 750, 730, 711, 687, 617, 581. **GC/MS** (El, RT; 47.138 min): m/z = 530 [M]⁺.

### e) Preparation by using Fe / Fe₂(SO₄)₃ as the catalyst and chlorobenzene as the solvent employing 0.02 eq. of iron ions

A mixture of benzenesulfonic acid 4-chlorocarbonyl-phenyl ester (26.7 g, 0.090 mol), chlorobenzene (133 mL), 6-methoxy-2-(4-methoxyphenyl)benzothiophene (19.5 g, 0.072 mol), iron (0.0335 g, 0.0006 mol), and Fe₂(SO₄)₃ (0.263 g, 0.0013 mol) was stirred for 4 h at 56 - 63 °C. The organic phase was washed with sodium hydroxide (aq., 1 N) and water and taken to dryness at 65 °C under reduced pressure.

### f) Preparation by using Fe / Fe₂(SO₄)₃ as the catalyst and chlorobenzene as the solvent employing 0.05 eq. of iron ions

Benzenesulfonicacid-4-[6-methoxy-2-(4-methoxy-phenyl)-benzo[b]thiophene-3-carbonyl]-phenyl ester was also obtained in a similar manner as described under example 9 item a), employing 0.05 eq. of iron ions as catalyst.

### g) Preparation by using Fe / Fe₂(SO₄)₃ as the catalyst and chlorobenzene as the solvent employing 0.005 eq. of iron ions:

Benzenesulfonicacid-4-[6-methoxy-2-(4-methoxy-phenyl)-benzo[b]thiophene-3-carbonyl]-phenyl ester was obtained in a similar manner as described under example 9 item a), employing 0.005 eq. of iron ions as catalyst.

### h) Preparation by using ZnCl₂ as the catalyst and chlorobenzene as the solvent:

Benzenesulfonicacid-4-[6-methoxy-2-(4-methoxy-phenyl)-benzo[b]thiophene-3-carbonyl]-phenyl ester was also obtained in a similar manner as described under example 9 item a), employing ZnCl₂ (0.05 eq.) as catalyst.

### i) Preparation by using AlCl₃ as the catalyst and chlorobenzene as the solvent:

Benzenesulfonicacid-4-[6-methoxy-2-(4-methoxy-phenyl)-benzo[b]thiophene-3-carbonyl]-phenyl ester was also obtained in a similar manner as described under example 9 item a), employing AlCl₃ (0.05 eq.) as catalyst.

### j) Preparation by using Zinc trifluoromethanesulfonate as the catalyst and chlorobenzene as the solvent:

Benzenesulfonicacid-4-[6-methoxy-2-(4-methoxy-phenyl)-benzo[b]thiophene-3-carbonyl]-phenyl ester was also obtained in a similar manner as described under example 9 item a), employing Zinc trifluoromethanesulfonate (0.05 eq.) as catalyst.

### Methanesulfonic acid-4-[6-methoxy-2-(4-methoxy-phenyl)-benzo[b]thiophene-3-carbonyl]-phenyl ester

A mixture of methanesulfonic acid 4-chlorocarbonyl-phenyl ester (7.5 g, 31.96 mmol), toluene (30 mL), 6-methoxy-2-(4-methoxyphenyl)benzothiophene (6.9 g, 25.5 mmol), iron (0.012 g, 0.22 mmol), and Fe₂(SO₄)₃ x 5 H₂O (0.093 g, 0.22 mmol) was heated at reflux for 9 h with stirring. The mixture was then flushed with nitrogen for 7.5 h at 108 -110 °C, cooled to 70 °C and treated with toluene (30 mL), sodium hydroxide (aq., 1 N, 16 mL), and SiO₂ (0.6 g). Stirring was continued for 50 min at 70 - 72 °C and the suspension was filtered. The filter residue was washed with hot toluene (5 mL) and the filtrates were combined. The phases were separated. The aqueous phase was disposed. The organic phase was treated with sodium hydroxide (aq., 1 N, 16 mL) and the mixture was stirred for 30 min at 75 °C. The phases were separated and the organic phase was washed with water (3 × 16 mL) at 90 °C. The organic phase was cooled to 1 °C and stirring was continued for 40 min. The solid was filtered off, washed with cold toluene (3x3mL) and dried at 45 °C under reduced pressure. Yield: 5.26 g (11.2 mmol, 44.05 %). **Purity**: 95.01 % (HPLC).

A sample (4.84 g, 10.3 mmol) of the above obtained product was suspended in acetone (55 mL) and heated to reflux. The resulting solution was cooled to 52 °C and seeded. The turbid solution was cooled to 1 - 3 °C and stirring was continued for 1 h. The solid was isolated, washed with cold acetone (3x1mL) and dried at 45 °C under reduced pressure. Yield: 3.88 g (8.3 mmol, 80.0 %). **Purity**: 98.99 % (HPLC). **DSC**: peak at 163.08 °C.
**¹H NMR** (300 MHz, DMSO-d₆, ppm): δ7.75 (d, 2 H, arom.), 7.67 (d, 1 H, arom.), 7.58 (d, 1 H, arom.), 7.37 - 7.18 (m, 4 H, arom.), 7.06 (dd, 1 H, arom.), 6.83 (d, 2 H, arom.), 3.85 (s, 3 H, -OCH₃), 3.69 (s, 3 H, -OCH₃), 3.33 (s, 3 H, -OSO₂CH₃).
**¹³C-{H)-NMR** (75 MHz, DMSO-d₆, ppm): δ92.07 (>C=O), 159.66 (=C<), 157.47(=C<), 152.21(=C<), 144.37(=C<), 139.45(=C<), 135.89(=C<), 132.84(=C<), 131.46 (=CH-), 130.24 (=CH-), 129.18(=C<), 124.93(=C<), 123.59 (=CH-), 122.14 (=CH-), 115.18 (=CH-), 114.13 (=CH-), 105.11 (=CH-), 55.54 (-OCH₃), 55.14 (-OCH₃), 37.41 (-CH₃). **IR** (KBr, cm⁻¹): 3015, 2939, 2837, 1640 (v_{c=o}), 1606, 1527, 1493, 1473, 1440, 1362, 1298, 1250, 1222, 1172, 1148, 1127, 1047, 1028, 959, 892, 871, 862, 840, 786, 769, 717, 633; 615, 556, 527, 517. **GC/MS** (El, RT; 39.900 min): m/z = 468 [M]⁺. **XRPD**: the X-ray diffractogram exhibits signals at the following 2θ values:

| **2theta[°]** | **I/I₀** |
|---|---|
| 6.21 | 71.16 |
| 11.44 | 144.14 |
| 12.25 | 62.4 |
| 12.97 | 99.06 |
| 14.51 | 86.7 |
| 15.31 | 59.98 |
| 16.31 | 57.5 |
| 17.86 | 222.06 |
| 18.77 | 479.76 |
| 18.93 | 408.11 |
| 19.07 | 753.68 |
| 19.23 | 122.2 |
| 19.39 | 145.69 |
| 19.77 | 1000 |
| 21.25 | 70.77 |
| 22.21 | 108.58 |
| 23 | 148.5 |
| 23.84 | 140.58 |
| 24.64 | 262.97 |
| 25.44 | 193.37 |
| 25.57 | 130.08 |
| 26.11 | 251.36 |
| 28.26 | 377.94 |
| 29.76 | 65.39 |
| 29.92 | 136.45 |
| 30.3 | 112.15 |
| 39.06 | 50.3 |

### Toluenesulfonic acid-4-[6-methoxy-2-(4-methoxy-phenyl)-benzo[b]thiophene-3-carbonyl]-phenyl ester

A mixture of toluenesulfonic acid 4-chlorocarbonyl-phenyl ester (10.0 g, 32.18 mmol), toluene (40 mL), 6-methoxy-2-(4-methoxyphenyl)benzothiophene (6.96 g, 25.7 mmol), iron (0.012 g, 0.22 mmol), and Fe₂(SO₄)₃ × H₂O (0.094 g, 0.22 mmol) was heated at reflux for 10.5 h with stirring. The mixture was cooled to approx. 70 °C, diluted with toluene (40 mL), treated with sodium hydroxide (aq., 1 N, 16 mL) and SiO₂ (0.6 g) and stirring was continued for 30 min at 65 - 68 °C. The mixture was filtered and the residue washed with hot toluene (5 mL). The filtrates were combined and the phases separated. The aqueous phase was disposed. The organic phase was treated with sodium hydroxide (aq., 1 N, 16 mL) and stirring was continued for 15 min at 60 - 68 °C. The phases were separated and the organic phase was washed with water (3 × 16mL) at ambient temperature, concentrated at 45 °C under reduced pressure, and cooled to 1 °C with stirring. After 60 min the resulting solid was isolated, washed with cold toluene (2 x 1 mL) and dried at 45 °C under reduced pressure. Yield 8.17 g (15.0 mmol, 58.4 %). Purity: 84.37 % (HPLC).

A sample (7.56 g,13.88 mmol) of the material obtained above was dissolved in toluene (19 mL) at approx. 100 °C. The solution was cooled to 5 °C with seeding and stirring was continued for 1.5 h. The resulting solid was filtered off, washed with cold toluene (2 x 2 mL), and dried at 50 °C under reduced pressure. Yield 4.72 g (8.7 mmol, 62.4 %). The re-crystallized material (4.55 g, 8.35 mmol) was suspended in acetone (13 mL) and heated to reflux. The resulting solution was cooled to 1 °C with seeding and stirring was continued for 45 min. The solid was isolated, washed with cold acetone (2 x 0.75 mL) and dried at 65 °C to give the title compound as crystalline solid. Yield: 3.49 g (6.4 mmol,
76.7 %). **Purity:** 92.33 % (HPLC). **Mp**.: 132.9 - 134.0 °C. **DSC**: peak at 133.14 °C. **¹H NMR** (300 MHz, DMSO-d₆, ppm): δ 7.70 - 7.50 (m, 6 H , arom.), 7.44 (m, 2 H, arom.), 7.22 (d, 2 H, arom.), 7.04 (dd, 1 H, arom.), 6.93 (d, 2 H, arom.), 6.85 (d, 2 H9, arom.), 3.84 (s, 3 H, - OCH₃), 3.72 (s, 3 H, -OCH₃) 2.41 (s, 3H, -CH₃). **¹³C-{H}-NMR** (75 MHz, DMSO-d₆, ppm): δ191.87 (>C=O), 159.68 (=C<), 157.46 (=C<), 152.00 (=C<), 145.98 (=C<), 144.46 (=C<), 139.47 (=C<), 135.90 (=C<), 132.82 (=C<), 131.28 (=CH-), 131.05 (=C<), 130.23 (=CH-), 130.11 (=CH-), 128.98 (=C<), 128.02 (=CH-), 124.90 (=C<), 123.54 (=CH-), 122.13 (=CH-), 115.14 (=CH-), 114.14 (=CH-), 105.08 (=CH-), 55.52 (-OCH₃), 55.18 (-OCH₃), 21.9 (-CH₃). **IR** (KBr, cm⁻¹): 2947, 2844, 1649 (v_{c=o}), 1598, 1527, 1497, 1476, 1442, 1376, 1356, 1294, 1251, 1215, 1201, 1177, 1154, 1092, 1045, 1022, 893, 862, 838, 739; 726, 668, 617, 573, 553. **GC/MS** (El, RT; 71.400 min): m/z = 544 [M]⁺. **XRPD:** the X-ray diffractogram exhibits signals at the following 2θ values:

| **2theta[°]** | **I/I₀** |
|---|---|
| 6.04 | 247.1 |
| 7.54 | 202.01 |
| 10.84 | 177.43 |
| 12.09 | 595.56 |
| 13.05 | 240.93 |
| 14.38 | 118.19 |
| 14.81 | 125.76 |
| 16.05 | 487.7 |
| 17.59 | 1000 |
| 17.86 | 333.83 |
| 18.01 | 176.09 |
| 18.97 | 486.46 |
| 19.18 | 83.42 |
| 19.41 | 86.52 |
| 20.4 | 267.28 |
| 20.53 | 299.19 |
| 21.05 | 522.54 |
| 21.3 | 200.27 |
| 22.57 | 289.73 |
| 23.03 | 623.01 |
| 24.17 | 82.34 |
| 24.37 | 128.02 |
| 25.3 | 148.52 |
| 26.08 | 87.7 |
| 26.44 | 584.05 |
| 26.85 | 262.88 |
| 27.97 | 92.12 |
| 29.07 | 95.11 |
| 31.1 | 96.61 |
| 32.46 | 85.66 |

### Example 10: Preparation of compound of formula VIII'

### (4-Hydroxy-phenyl)-[6-methoxy-2-(4-methoxy-phenyl)-benzo[b]thiophen-3-yl]-methanone

### a) Preparation by using moist benzenesulfonic acid-4-[6-methoxy-2-(4-methoxyphenyl)-benzo[b]thiophene-3-carbonyl]-phenyl ester, TBAB as the catalyst, NaOH as the base and toluene/water as the solvent:

A 2 L glass flask was charged with benzenesulfonic acid-4-[6-methoxy-2-(4-methoxy-phenyl)-benzo[b]thiophene-3-carbonyl]-phenyl ester (182.5 g, 344 mmol, calcd. dry), prepared as described under example 9 item a, TBAB (2.2 g, 7 mmol), and toluene (365 mL). The contents were heated to 106 - 108 °C and 22 mL of solvents were distilled off. The mixture was cooled to 80 °C and treated with water (160 mL) and aq. sodium hydroxide (92 mL, 1720 mmol). The biphasic mixture was heated to reflux with stirring. After 4 h water (365 mL) was added and stirring was continued for 20 min at 88 °C. The phases were separated and the organic phase was disposed. The aqueous phase was vigorously washed with toluene (290 mL) at approx. 90 °C, cooled to 12 °C, and treated with methylene chloride (670 mL) and hydrochloric acid (aq., 175 mL, 1800 mmol) in sequence. Stirring was continued for 25 min at 12 - 20 °C until a pH of 1 was observed. The phases were separated and the organic phase was washed with water (3 x 165 mL) to yield a solution of (4-hydroxy-phenyl)-[6-methoxy-2-(4-methoxy-phenyl)-benzo[b]thiophen-3-yl]-methanone in methylene chloride (1002 g). **Purity**: 97.62 % (HPLC).

### b) Preparation by using TBAB as the catalyst, NaOH as the base and toluene/water as the solvent:

A flask was charged with benzenesulfonic acid-4-[6-methoxy-2-(4-methoxy-phenyl)-benzo[b]thiophene-3-carbonyl]-phenyl ester (27.91 g, 0.053 mol), TBAB (0.34 g, 0.0011 mol), and toluene (170 mL). Aq. sodium hydroxide (cc., 16.9 mL, 0.318 mol) and water (25 mL) were added at ambient temperature and the mixture was heated to reflux with stirring. After 6 h the mixture was treated with water (68 mL). The aqueous phase was separated and washed with toluene (45 mL) at 80 - 86 °C. The aqueous phase was subsequently treated with methylene chloride (100 mL) and aq. hydrochloric acid (cc., 32 mL, 0.331 mol) at room temperature with stirring. The organic phase was separated, washed with water (4 x 25 mL), and evaporated to dryness. A mixture of MTBE (12 mL), toluene (110 mL), and activated charcoal (1.0 g) was added after which the suspension was heated to reflux. The suspension was filtered and the residue washed with a hot mixture of MTBE and toluene (1:9 v:v, 10 mL). The combined filtrates were cooled to 2 °C with stirring. The resulting precipitate was isolated, washed with a mixture of MTBE and toluene (1:9 v/v, 2 x 5 mL) and dried at 40 °C under reduced pressure. The product was dissolved in methanol (100 mL) and evaporated to dryness. This procedure was repeated twice. The residue was dried under reduced pressure to yield the title compound as an amorphous solid. Yield: 80.9 %. **Purity**: 99.21 % (HPLC). **Mp.**: 79.8 - 83.2 °C. **DSC**: first peak at 69.8 °C, second peak at 368.6 °C.**¹H NMR** (300 MHz, DMSO-d₆, ppm): δ10.47 (b, 1 H, -OH), 7.62 (m, 3 H, arom.), 7.33 (m, 3 H, arom.), 6.98 (m, 1 H, arom.), 6.88 (d, 2 H, arom.), 6.74 (d, 2 H, arom.), 3.83 (s, 3 H, -OCH₃), 3.71 (s, 3 H, -OCH₃); **¹³C-{H}-NMR** (75 MHz, DMSO-d₆, ppm): δ 92.03 (>C=O), 162.61, 159.33, 157.24, 140.20, 139.24, 133.21 (=C<), 132.00 (=CH-), 130.33 (=C<), 129.50 (=CH-), 128.19, 125.21 (=C<), 123.21, 115.35, 114.81, 114.21, 105.00 (=CH-), 55.41, 55.04 (-OCH₃). IR (KBr, cm⁻¹): 3277, 2935, 2834, 1633 (v_{c=o}), 1603 (vₐᵣₒₘ.), 1574, 1535, 1499, 1474, 1438, 1349, 1254, 1178, 1162, 1076, 1046, 1030, 893, 829, 783, 616. **LC/MS** (ESl(+), RT; 17.02 min): m/z = 391 [MH]⁺.

### c) Preparation by using TBAB as the catalyst, KOH and toluene/water as the solvent:

(4-Hydroxy-phenyl)-[6-methoxy-2-(4-methoxy-phenyl)-benzo[b]thiophen-3-yl]-methanone was also obtained in a similar manner as described under example 10 item b, employing aqueous potassium hydroxide solution (6.0 eq.) as base. **Purity**: 97.13 % (HPLC).

### Example 11: Preparation of sodium and potassium salts of compound of formula VIII'

### Sodium 4-[6-methoxy-2-(4-methoxy-phenyl)-benzo[b]thiophene-3-carbonyl]-phenolate

### a) Using sodium methylate as the base and methanol as the solvent:

(4-Hydroxy-phenyl)-[6-methoxy-2-(4-methoxy-phenyl)-benzo[b]thiophen-3-yl]-methanone (14.61 g, 0.024 mol), prepared as described under example 10 item b, was dissolved in methanol (14.5 mL) at ambient temperature. MTBE (44 mL) and a solution of sodium methylate in methanol (7.8 mL, 1.1 eq.) were added to the clear solution. After addition of MTBE (27 mL), the mixture was cooled and stirred for 4 h at -16 to -17 °C. The amorphous solid was separated, washed with a mixture of methanol and MTBE (40:10 v/v, 2 x 5 mL) and dried at 45 °C under reduced pressure. **Purity**: 99.28 % (HPLC). **DSC**: peak at 96.8 °C.¹H NMR (300 MHz, DMSO-d₆, ppm): δ7.58 (d, 1 H, arom.), 7.48 - 7.19 (m, 5 H, arom.) 6.95 (dd, 1 H, arom.) 6.90 (d, 2 H, arom.), 6.19 (b, 2 H, arom.), 3.83 (s, 3 H, -OCH₃), 3.72 (s, 3 H, -OCH₃)- **IR** (KBr, cm-¹): 2938, 2835, 1608 (v_{c=o}), 1569, 1498, 1473, 1438, 1344, 1280, 1258, 1215, 1180, 1157, 1075, 1045, 1025, 894, 830, 785, 737, 644, 618, 595, 554. **LC/MS** (ESI(+), RT; 19.88): m/z = 390.9 [MH-Na]⁺.

### b) Sodium 4-[6-methoxy-2-(4-methoxy-phenyl)-benzo[b]thiophene-3-carbonyl]-phenolate using 2-piperidin-1-yl-ethanolate (formed in situ) as the base and toluene as the solvent:

A flask was charged with a solution of toluene (5 mL), sodium (0.64 g, 0.0277 mol), and 2-piperidin-1-yl-ethanol (4.6 mL, 0.0342 mol) and benzenesulfonic acid-4-[6-methoxy-2-(4-methoxy-phenyl)-benzo[b]thiophene-3-carbonyl]-phenyl ester (7.0 g, 0.0132 mol) in toluene (55 mL) was added within 20 min at 18 - 23 °C. After stirring for 1 h at ambient temperature, the mixture was heated at 47-50 °C for 0.5 h with stirring. The resulting solid was separated by filtration, washed with toluene (3 × 10 mL) and dried to give the title compound as its sodium salt.

### Potassium 4-[6-methoxy-2-(4-methoxy-phenyl)-benzo[b]thiophene-3-carbonyl]-phenolate

### a) Potassium 4-[6-methoxy-2-(4-methoxy-phenyl)-benzo[b]thiophene-3-carbonyl]-phenolate by using 2-piperidin-1-yl-ethanolate (formed in situ) as the base and toluene as the solvent:

A corresponding potassium salt was obtained in a similar manner as described under example 11 item b, employing potassium for forming 2-piperidin-1-yl-ethanolate as base.

### b) Potassium 4-[6-methoxy-2-(4-methoxy-phenyl)-benzo[b]thiophene-3-carbonyl]-phenolate using K₂CO₃ as the base:

A mixture of benzenesulfonic acid-4-[6-methoxy-2-(4-methoxy-phenyl)-benzo[b]thiophene-3-carbonyl]-phenyl ester (3.0 g, 0.0056 mol), 2-piperidin-1-yl-ethanol (9 mL, 67 mmol), and K₂CO₃ (1.88 g, 2.4 eq.) was stirred for 6.5 h at 80 - 83 °C. The mixture was cooled and extracted with a mixture of 45 mL of toluene (45 mL) and water (25 mL). The organic phase was extracted with water (2 x 25 mL) and evaporated to dryness. Yield: 85 %.

### Examples 12 and 13: Preparations of compounds of formula X'

### Example-12

### 1-(2-{4-[6-Methoxy-2-(4-methoxy-phenyl)-benzo[b]thiophene-3-carbonyl]-phenoxy}-ethyl)-piperidine

### a) Preparation by using NaOH as the base and water/methylene chloride as the solvent:

A solution (830.5 g) of (4-hydroxy-phenyl)-[6-methoxy-2-(4-methoxy-phenyl)-benzo[b]thiophen-3-yl]-methanone (118.4 g, 0.303 mol, calcd. dry) in methylene chloride, prepared as described under example 10 item a, was treated with water (45 mL), 1-(2-chloroethyl)-piperidinium chloride (58.57 g, 0.318 mol), and methylene chloride (175 mL). Aq. sodium hydroxide (cc., 40.6 mL, 0.758 mol) was added slowly (15 min) at 16 - 20 °C with stirring. The mixture was heated to reflux and stirring was continued for 6.5 h. The contents were cooled to 24 °C and water (765 mL) was added with stirring. The phases were separated. The organic phase was washed with water (3 x 260 mL) and concentrated at 40 - 42 °C. The solution was diluted with methanol (510 mL) and concentrated at approx. 60 °C to give 478 g of a solution of [6-Methoxy-2-(4-methoxy-phenyl)-benzo[b]thiophen-3-yl]-[4-(2-piperidin-1-yl-ethoxy)-phenyl]-methanone in methanol. Yield: quantitative. **Purity:** 97.0 % (HPLC).

### b) Preparation by using polyethylene glycol 600 as the catalyst, K₂CO₃ as the base and toluene/MTBE as the solvent:

A mixture of (4-hydroxy-phenyl)-[6-methoxy-2-(4-methoxy-phenyl)-benzo[b]thio-phen-3-yl]-methanone (3.9 g, 0.010 mol), toluene (25 mL), MTBE (5 mL), 1-(2-chloro-ethyl)-piperidine hydrochloride (2.03 g, 0.011 mol), K₂CO₃ (4.42 g, 0.032 mol), and PEG 600 (0.6 g, 0.001 mol) was heated at 62 - 68 °C for 16 h with stirring. The mixture was cooled and washed with water (5 x 20 mL) at ambient temperature. The organic phase was dried over Na₂SO₄ and evaporated to dryness. Yield: 88.0 %. **Purity**: 91.0 % (HPLC).

### c) Synthesis from 1-(2-{4-[6-Methoxy-2-(4-methoxy-phenyl)-benzo[b]thiophene-3-carbonyl]-phenaxy}-ethyl)-piperidinium chloride

A flask was charged with 1-(2-{4-[6-Methoxy-2-(4-methoxy-phenyl)-benzo-[b]thiophene-3-carbonyl]-phenoxy}-ethyl)-piperidinium chloride (145.0 g, 269.5 mmol), methylene chloride (750 mL), and water (250 mL). Aq. sodium hydroxide (18.86 N, 17.1 mL) was added dropwise at 18 °C with stirring. Stirring was continued for 10 min and the phases were separated. The organic phase was washed with water (2 x 250 mL) to give a solution, (795 mL) of the title compound in methylene chloride. A sample (70 mL) was evaporated to dryness at 45°C under reduced pressure to give 1-(2-{4-[6-Methoxy-2-(4-methoxy-phenyl)-benzo[b]thiophene-3-carbonyl]-phenoxy}-ethyl)-piperidin. Yield: 11.58 g (23.08 mmol, 97.3 %, calcd.). **Purity**: 97.42 (HPLC). ¹**H NMR** (300 MHz, DMSO-d₆, ppm): δ 7.67 (d, 2 H, arom.), 7.64 (d, 1 H, arom.), 7.37 - 7.26 (m, 3 H, arom.), 6.98 (dd, 1 H, arom.), 6.94 (m, 4 H, arom.), 4.05 (t, 2 H, -OCH₂CH₂NR₂), 3.83 (s, 3 H, -OCH₃), 3.70 (s, 3 H, -OCH₃), 2.58 (-OCH₂CH₂NR₂), 2.36 (bt, 4 H, piperidine), 1.50-1.27 (m, 6 H, piperidine). **¹³C-{H}-NMR** (75 MHz, DMSO-d₆, ppm): δ 192.26 (R₂CO), 162.88 (=C<), 159.45 (=C<), 157.35 (=C<), 140.7 (=C<), 139.33 (=C<), 133.18 (=C<), 131.73 (=CH-), 130.16 (=C<), 129.6 (=CH-), 129.52 (=C<), 125.17(=C<), 123.24 (=CH-), 114.93 (=CH-), 114.52 (=CH-), 114.31 (=CH-), 105.12 (=CH-), 65.98 (-CH₂-), 57.01 (-CH₂-), 55.5 (-OCH₃), 55.12 (-OCH₃), 54.25 (-CH₂-), 25.48 (-CH₂-), 23.81 (-CH₂-). **lR** (KBr, cm⁻¹): 2933, 1647 (v_{c=o}), 1598, 1572, 1535, 1500, 1475, 1294, 1253, 1164, 1031, 892, 828, 782, 620. **LC/MS** (ESl(+), RT; 14.17 min): m/z = 501.9 [MH]⁺.

### 1-(2-{4-[6-Methoxy-2-(4-methoxy-phenyl)-benzo[b]thiophene-3-carbonyl]-phenoxy}-ethyl)-piperidinium chloride

### d) Preparation by using TBAB as the catalyst, NaOH as the base and water/methylene chloride as the solvent:

A flask was charged with (4-hydroxy-phenyl)-[6-methoxy-2-(4-methoxy-phenyl)-benzo[b]thiophen-3-yl]-methanone (17.6 g, 0.045 mol), prepared according to example 10 item c, and methylene chloride (140 mL). The stirred mixture was treated with aq. sodium hydroxide (cc., 5.9 mL, 0.113 mol), water (6 mL), TBAB (0.73 g, 0.0023 mol), and 1-(2-chloroethyl)-piperidine hydrochloride (9.13 g, 0.050 mol) at ambient temperature. Stirring was continued for 16 h and the phases were separated. The organic phase was washed with aq. hydrochloric acid (1N, 3 x 50 mL), water (55 mL), aq. sodium hydroxide (2 N, 55 mL), and with water (5 x 50 mL) in sequence. The organic phase was dried over Na₂SO₄ and evaporated to dryness. The residue (21.8 g) was dissolved in methanol (65 mL) and aq. hydrochloric acid (cc., 3.8 mL, 0.046 mol) was added at 51 °C. The suspension was cooled to 2 °C and stirring was continued for 0.75 h. The solid was isolated, washed with methanol (2 x 7.5 mL) and dried at 70 °C. Yield: 18.36 g (78.4 %). **Purity:** 98.64 % (HPLC).

A sample (17.1 g, 31.78 mmol) of the material obtained above was dissolved in hot methanol (128 mL) and the solution was filtered. The clear filtrate was cooled to 1 °C with stirring. The resulting solid was isolated, washed with methanol (3 x 4.5 mL), and dried at 40 °C under reduced pressure. Yield: 14.61 g (85.4 %). **Purity**: 99.03 % (HPLC). **Mp**.: 217.8 °C; DSC: first peak at 220.7 °C, second peak at 314 °C. **¹H NMR** (300 MHz, DMSO-d₆, ppm): δ 10.55 (b, 1 H, -NH), 7.71 (m, 3 H, arom.), 7.32 (m, 3 H, arom.), 7.00 (d, 3 H, arom.), 6.90 (d, 2 H, arom.), 4.44 (t, 2 H, -OCH₂CH₂NH-), 3.84 (s, 3 H, -OCH₃), 3.72 (s, 3 H, -OCH₃), 3.43 (m, 4 H, -NHCH₂CH₂-), 2.95 (b, 2 H, -OCH₂CH₂NH-), 1.77 (m, 4 H, -CH₂CH₂CH₂NR₂), 1.66 - 1.36 (m, 2H, -CH₂CH₂CH₂-); **¹³C-{H}-NMR** (75 MHz, DMSO-d₆, ppm) δ192.22 (>C=O), 161.66 (=C<), 159.37 (=C<), 157.26 (=C<), 140.69 (=C<), 139.23 (=C<), 132.98 (=C<), 131.64 (=CH-), 130.11 (=C<), 129.94 (=C<), 129.50 (=CH-), 125.02 (=C<), 123.06 (=CH-), 114.87 (=CH-), 114.61 (=CH-), 114.27 (=CH-), 105.09 (=CH-), 62.40 (-CH₂-), 55.43 (-OCH₃), 55.08 (-OCH₃), 54.30 (-CH₂-), 52.41 (-CH₂-), 22.08 (-CH₂-), 20.95 (-CH₂-). **lR** (KBr, cm⁻¹): 3413, 2941, 2453, 1643 (vc=o), 1599 (vₐᵣₒₘ.), 1574, 1535, 1498, 1475, 1438, 1353, 1307, 1295, 1251, 1179, 1043, 1026, 949, 893, 830, 783, 645, 621. **LC/MS** (ESI(+), RT; 11.90 min): m/z = 502.1 [MHr. **XRPD:** the XRD diffragtogram exhibits signals at the following 28 values:

| **2theta[°]** | **I/I₀** |
|---|---|
| 6.41 | 84.31 |
| 11.41 | 259.94 |
| 12.56 | 215.27 |
| 13.07 | 303.03 |
| 14.82 | 198.13 |
| 15.45 | 144.45 |
| 15.86 | 109.53 |
| 16.74 | 421.69 |
| 17.1 | 119.28 |
| 18.33 | 90.15 |
| 18.95 | 197.08 |
| 19.27 | 1000 |
| 19.39 | 321.4 |
| 19.79 | 66.13 |
| 20.09 | 233.2 |
| 21.56 | 278.71 |
| 22.32 | 118.66 |
| 22.92 | 114.72 |
| 23.42 | 995.98 |
| 24 | 247.55 |
| 24.63 | 780.62 |
| 24.77 | 96.24 |
| 25.53 | 236.05 |
| 25.89 | 136.45 |
| 26.05 | 320.65 |
| 26.83 | 121,67 |
| 29.35 | 147.82 |
| 29.49 | 128.56 |
| 30.45 | 67.32 |
| 42.03 | 66.09 |

### e) Preparation by using NaOH as the base and water/methylene chloride as the solvent:

A flask was charged with a solution (735 g) of (4-hydroxy-phenyl)-[6-methoxy-2-(4-methoxyphenyl)-benzo[b]thiophen-3-yl]-methanone (96.0 g, 0.246 mol, calcd. dry) in methylene chloride, prepared as described under example 10 item a, water (35 mL), 1-(2-chloro-ethyl)-piperidine hydrochloride (49.82 g, 0.271 mol), and methylene chloride (96 mL). Aq. sodium hydroxide (cc., 32.9 mL, 0.622 mol) was added slowly (20 min) at 15 -18 °C with stirring. The mixture was allowed to reach room temperature and stirring was continued for a further 60 min after which the reactor contents were heated at reflux for approx. 2.25 h. The resulting clear solution was allowed to reach room and treated with water (620 mL). Stirring was continued for a further 10 min and the phases were separated. The organic phase was washed with water (4 x 210 mL), concentrated at max. 48 °C and dissolved in methanol (385 mL). The resulting clear solution was concentrated at normal pressure and aq. hydrochloric acid (cc., 21.5 mL.) was added at approx. 50 °C with stirring. The solution was seeded with appropriate material. Stirring was continued for approx. 45 min and the resulting suspension was cooled to approx. -10 °C. Stirring was continued for a further 60 min at -6 to -9 °C after which the precipitate was isolated, washed with cold methanol (3 x 30 mL) and dried under reduced pressure (5 h, 50 °C) to give the title compound. Yield: 99.19 g (75.0 %). **Purity:** 97.16 % (HPLC).

For an optional purification, a sample (98.3 g, 182.7 mmol) was suspended in methanol (675 mL) and heated to reflux. The resulting clear solution was seeded with appropriate material with stirring. The resulting suspension was cooled to 2 °C and stirring was continued for 1.5 h. The solid was isolated, washed with cold methanol (2 x 30 mL) and dried at 50 °C under reduced pressure. Yield: 85.7 g (159.3 mmol, 87.2 %). Purity: 99.33 % (HPLC).

### f) Preparation from 1-(2-{4-[6-Methoxy-2-(4-methoxy-pheny))-benzo[b]thiophene-3-carbonyl]-phenoxy}-ethyl)-piperidinium bromide as the starting material

1-(2-{4-[6-Methoxy-2-(4-methoxy-phenyl)-benzo[b]thiophene-3-carbonyl]-phenoxy}-ethyl)-piperidinium bromide (122.3 g, 209.9 mmol), prepared according to example 13 item c was added to mixture of methylene chloride (630 mL) and water (210 mL) with stirring. Aq. sodium hydroxide (18.86 N, 14.5 mL) was added over a period of 5 min at 17 °C and stirring was continued for 10 min. The phases were separated and the organic phase was washed with water (2 x 210 mL). Aqueous phases were disposed and the organic phase was concentrated at 45 °C under reduced pressure. Methanol (700 mL) was added to the residue and the mixture was heated to 50 °C. Hydrochloric acid (aq., 12 N, 21 mL, 252 mmol) was added dropwise to reach a pH of 1.5. The clear solution was seeded with appropriate material and, after crystallization was observed, the suspension was cooled to approx. -10 °C and stirring was continued for 2.5 h. The solid was isolated, washed with cold methanol (3 x 30 mL) and dried at 50 °C under reduced pressure to give the title compound. Yield: 105.76 g (196.5 mmol, 93.6 %). Purity: 99.69 % (HPLC).

### Example 13

### 1-(2-{4-[6-Methoxy-2-(4-methoxy-phenyl)-benzo[b]thiophene-3-carbonyl]-phenoxy}-ethyl)-piperidinium bromide

### a) Preparation by using methanol as the solvent:

A solution of [6-Methoxy-2-(4-methoxy-phenyl)-benzo[b]thiophen-3-yl]-[4-(2-piperidin-1-yl-ethoxy)-phenyl]-methanone (75.0. g, 0.150 mol) in methanol (195 mL), prepared as described under example 12 item a, was heated to 50 - 58 °C and aq. hydrobromic acid (47 %, 25.9 g, 0.150.mol) was added dropwise to adjust a pH of 1. The resulting suspension was cooled to - 6 °C and stirring was continued for 0.75 h. The crystalline solid was separated, washed with cold methanol (3 x 20 mL) and dried at 50 °C under reduced pressure. Yield: 79.01 g (135.4 mmol, 89.5 %). Purity: 98.34 % (HPLC). DSC: peak at 232 °C; peak at 330 °C.

**lR** (KBr, cm⁻¹): 2940, 2838, 2627, 2517, 1642 (v_{c=o}), 1598, 1573, 1535, 1498, 1475, 1455, 1435, 1354, 1307, 1294, 1251, 1217, 1178, 1128, 1083, 1043, 1026, 1007, 970, 947, 893, 829,783,765,644,620,512.
For an optional purification, a sample (77.4 g, 132.6 mmol) of the product as obtained above was dissolved in a mixture of 1-propanol and water (75:25 v/v, 500 mL) at reflux temperature. The solution was allowed to cool to 70 °C, seeded with appropriate material, and cooled to approximately 5 °C. Stirring was continued for a further 60 min and the solid was separated.
The crystalline material was washed with a cold mixture of 1-propanol and water (75:25 v/v, 2 x 25 mL) and dried under reduced pressure (6 h, 50 °C). Yield: 70.79 g (121.3 mmol, 91.5 %); **Purity**: 99.18 % (HPLC). **Mp**.: 230.8 °C; **DSC**: first peak at 233.1 °C, second peak at 330.6 °C. **¹H NMR** (300 MHz, DMSO-d₆, ppm): δ 9.58 (bs, 1 H, - NH), 7.73 (d, 2 H, arom.), 7.66 (dd, 1 H, arom.), 7.32 (m, 3 H, arom.), 7.00 (m, 3 H, arom.), 6.89 (d, 2 H, arom.), 4.42 (t, 2 H, -O**CH**₂CH₂NH-), 3.84 (s, 3 H, -OCH₃), 3.72 (s, 3 H, -OCH₃), 3.50 (b, 4 H, -OCH₂**CH**₂NH-), 3.02 (m, 2 H, piperidine), 1.96 - 1.17 (m, 6 H, piperidine).
¹³**C-{H}-NMR** (75 MHz, DMSO-d₆, ppm): 6 192.36 (>C=_{O}), 161.78 (=C<), 159.46 (=C<), 157.36 (=C<), 140.81 (=C<), 139.36 (=C<), 133.10 (=C<), 131.76 (=CH-), 130.20 (=C<), 130.01 (=C<), 129.62 (=CH-), 125.13 (=C<), 123.17 (=CH-), 115.01 (=CH-), 114.76 (=CH-), 114.38 (=CH-), 105.18 (=CH-), 62.42 (-CH₂-), 55.55 (-OCH₃), 55.21 (-OCH₃), 54.56 (-CH₂-), 52.63 (-CH₂-), 22.27 (-CH₂-), 20.99 (-CH₂-). **IR** (KBr, cm⁻¹): 2940, 2628, 2517, 1642 (v_{c}=ₒ), 1598, 1573, 1535, 1498, 1475, 1454, 1435, 1354, 1307, 1294, 1251, 1217, 1173, 1127, 1043, 1026, 1007, 970, 946, 893, 829, 783, 765, 644, 620, 542, 512. **LC/MS** (ESI(+), RT; 13.99 min): m/z = 502.1 [MH]⁺. **XRPD:** the X-ray diffractogram exhibits signals at the following 2θ values:

| **2theta[°]** | **I/I₀** |
|---|---|
| 7.41 | 236.8 |
| 11.38 | 97.77 |
| 13.05 | 432.67 |
| 14.57 | 130.76 |
| 15.28 | 130.66 |
| 16.64 | 196.79 |
| 17.1 | 104.55 |
| 19.07 | 328.62 |
| 19.22 | 370.8 |
| 19.56 | 103.49 |
| 19.71 | 111.49 |
| 20.04 | 158.16 |
| 21.45 | 455.47 |
| 22.35 | 129.8 |
| 22.84 | 119.53 |
| 23.31 | 492.39 |
| 23.81 | 127.93 |
| 24.34 | 1000 |
| 25.45 | 145.39 |
| 25.81 | 606.94 |
| 25.96 | 126.15 |
| 26.12 | 101.11 |
| 26.43 | 89.73 |
| 26.62 | 213.81 |
| 27.3 | 163.65 |
| 29.3 | 120.27 |
| 30.33 | 124.47 |
| 33.65 | 100.22 |
| 33.95 | 103.18 |
| 41.36 | 102.63 |

### b) Preparation by using 2-propanol as the solvent:

1-(2-{4-[6-Methoxy-2-(4-methoxy-phenyl)-benzo[b]thiophene-3-carbonyl]-phenoxy}-ethyl)-piperidinium bromide was also obtained in a similar manner as described under example 13 item a, employing 2-propanol as solvent. Yield: 5.81 g (9.96 mmol, 83.4 %). Purity: 96.0 % (HPLC). **Mp**..: 225.8 °C; DSC: first peak at 230.5 °C, second peak at 328.7 °C. **lR** (KBr, cm⁻¹): 2941, 2838, 2628, 2517, 1642 (v_{c}=ₒ), 1598, 1573, 1535, 1498, 1475, 1455, 1435, 1354, 1307, 1294, 1251, 1217, 1173, 1128, 1083, 1043, 1026, 1007, 970, 947, 893, 829, 783, 644, 621, 512. **XRPD**: X-ray diffractogramdata correspond to those given for Example 13 a).

### c) Preparation by using n-propanollwater as the solvent:

A solution of [6-Methoxy-2-(4-methoxy-phenyl)-benzo[b]thiophen-3-yl]-[4-(2-piperidin-1-yl-ethoxy)-phenyl]-methanone (189.09 g, 376.9 mmol) in a mixture of n-propanol and water (75:25 v:v, 570 mL) prepared as described under example 12 item a, was heated to 60 - 70 °C and aq. hydrobromic acid (47 %, 42 mL, 377 mmol) was added slowly (30 min) to adjust a pH of 1.5 - 2. The resulting suspension was cooled to 2 - 5 °C and stirring was continued for 1.5 h. The solid was isolated and washed three times with a cold mixture of n-propanol/water (75:25 v:v, 3 x 40 mL). Yield: 227.2 g (172.8 g, 296.6 mmol, 86.8 %, calcd. dry). **Purity**: 98.18 % (HPLC).

### d) Preparation from 1-(2-{4-[6-Methoxy-2-(4-methoxy-phenyl)-benzo[b]thiophene-3-carbonyl]-phenoxy)-ethyl)-piperidinium chloride as the starting material

1-(2-{4-[6-Methoxy-2-(4-methoxy-phenyl)-benzo[b]thiophene-3-carbonyl]-phenoxy}-ethyl)-piperidinium chloride (145.0 g, 269.5 mmol), prepared according example 12 item e was added to a mixture of methylene chloride (750 mL) and water (250 mL) with stirring. Aq. sodium hydroxide (18.86 N, 17.1 mL) was added dropwise over a period of 5 min at 18 °C and stirring was continued for 10 min. The phases were separated and the organic phase was washed water (2 x 250 mL). Yield: 795 mL of a solution of 1-(2-{4-[6-Methoxy-2-(4-methoxyphenyl)-benzo[b]thiophene-3-carbonyl]-phenoxy}-ethyl)-piperidine in methylene chloride. A sample (655 mL) was evaporated at 45 °C under reduced pressure and treated with a mixture of 1-propanol (525 mL) and water (160 mL). The mixture was heated to 70 °C and aq. hydrobromic acid (48%, 29 mL, 258 mmol) was added dropwise over a period of 8 min to adjust a pH of 1.5. The solution was allowed to cool to 77 °C and seeded with appropriate material. After crystallization was observed the suspension was cooled to 0 - 5 °C and stirring was continued for 2 h. The solid was isolated, washed with a cold mixture of 1-propanol and water (75:25 v/v, 3 x 40 mL) and dried at 50 °C under reduced pressure. Yield: 121.24 g (208.1 mmol, 77.2 %). **Purity**: 99.3 % (HPLC). **IR** (KBr, cm-¹): 2940, 2627, 2517, 1642 (v_{c}=ₒ), 1598, 1573, 1534, 1498, 1475, 1454, 1435, 1354, 1307, 1294, ,1251, 1217, 1173, 1127, 1083, 1043, 1026, 1007, 969, 946, 893, 829, 783, 764, 644, 620, 533, 512.

### Example 14 to 17: Preparation of compound of formula Xl'

### Example 14

### Solvates of 1-(2-{4-[6-Hydroxy-2-(4-hydroxy-phenyl)-benzo[b]thiophene-3-carbonyl]-phenoxy)-ethyl)-piperidinium chloride

### a) 1-(2-{4-[6-Hydroxy-2-(4-hydroxy-phenyl)-benzo[b]thiophene₋3-carbonyl]-phenoxy}-ethyl)-piperidinium chloride. 0.28 methanol. 0.53 methylene chloride:

A solution of boron trichloride in methylene chloride (1 M, 446 mL, 446 mmol) was added dropwise to a mixture of 1-(2-{4-[6-Methoxy-2-(4-methoxy-phenyl)-benzo[b]thiophene-3-carbonyl]-phenoxyl-ethyl)-piperidinium chloride (80.0 g, 0.149 mol), prepared as described under example 12 item e, and methylene chloride (400 mL) over a period of 3 h at 0 - 2 °C with stirring. The mixture was stirred for 1 h at 2 - 6 °C and heated to ambient temperature over a period of 2 h. Stirring was continued for approx. 18 h after which the suspension was cooled to 4 °C and boron trichloride in methylene chloride (1 M, 149 mL, 149 mmol) was added within 1.5 h, not exceeding an inner temperature of 7 °C. The suspension was heated at reflux for 6.6 h and cooled to room temperature. Boron trichloride in methylene chloride (1 M, 149 mL, 149 mmol) was added over a period of 1 h at 22 °C after which the mixture was heated at reflux for 5 h with stirring. The mixture was cooled to 15 °C and methanol (240 mL) was added slowly (0.75 h), not exceeding an inner temperature of 27 °C. The resulting suspension was heated at reflux for 0.5 h and allowed to reach room temperature. Stirring was continued for a further 60 min and the precipitate was filtered off. The crystalline solid was washed with methanol (6 x 75 mL). A sample (79.1 g) was dried at 50 °C under reduced pressure (40 mbar) to give 66.78 g of 1-(2-{4-[6-Hydroxy-2-(4-hydroxy-phenyl)-benzo[b]thiophene-3-carbonyl]-phenoxy}-ethyl)-piperidinium chloride 0.28 methanol 0.53 methylene chloride. Yield: 92.6 % (138 mmol, calcd. dry).
Purity: 98.18 % (HPLC). **Methanol:** 1.6 % by weight, corresponding to approx. 0.28 molar equivalents (GC). **Methylen** chloride: 8.0 % by weight, corresponding to approx. 0.53 molar equivalents (GC). **Mp.**: 225.8 °C; DSC: first peak at 201.4 °C, second peak at 225.3 °C, third peak at 262.9 °C, fourth peak at 338.5 °C; ¹H **NMR** (300 MHz, DMSO-d₆, ppm): δ 10.68 (bs, 1 H, -NH), 9.88 (bs, 2 H, -OH), 7.70 (d, 2 H, arom.), 7.38 (m, 1 H, arom.), 7.27 (d, 1 H, arom.), 7.17 (d, 2 H, arom.), 6.97 (d, 2 H, arom.), 6.89 (m, 1 H, arom.), 6.71 (d, 2 H, arom. ) 5.75 (CH₂C1₂), 4.45 (m, 2H, -OCH₂CH₂NH-), 3.51 - 3.37 (m, 4 H, -OCH₂CH₂NH-, piperidine), 3.17 (CH₃0H), 2.95 (m, 2 H, piperidine,) 1.95 -1.22 (m, 6 H, piperidine).
¹³C-{H}-NMR (75 MHz, DMSO-d₆, ppm): δ 192.54 (>C=O), 161.60 (=C<), 157.89 (=C<), 155.50 (=C<), 140.59 (=C<), 139.20 (=C<), 132.16 (=C<), 131.73 (=CH-), 130.35 (=C<), 129.64 (=CH-), 129.55 (=C<), 123.68 (=C<), 123.19 (=CH-), 115.70 (=CH-), 115.23 (=CH-), 114.61 (=CH-), 107.11 (=CH-), 62.51 (-CH₂-), 54.45 (-CH₂-), 52.55 (-CH₂-), 22.23 (-CH₂-), 21.08 (-CH₂-). IR (KBr, cm-¹): 3203, 2958, 2722, 1653 (v_{c}=ₒ), 1597, 1548, 1501, 1467, 1437, 1419, 1343, 1309; 1250, 1166, 1065, 1037, 1021, 953, 939, 907, 840, 817, 808, 786, 738, 645, 635, 627, 594, 524. **LC/MS** (ESI(+), RT; 10.31 min): m/z = 473.9 [M-Cl⁻]⁺. **XRPD:** the X-ray diffractogram exhibits signals at the following 2θ values:

| **2theta[°]** | **I/I₀** |
|---|---|
| 11.01 | 290.35 |
| 13.31 | 167.25 |
| 13.46 | 631.03 |
| 15.88 | 208.55 |
| 16.45 | 1000 |
| 17.56 | 861.31 |
| 18.76 | 994.61 |
| 18.9 | 439.77 |
| 19.1 | 781.29 |
| 19.53 | 949.56 |
| 19.76 | 612.2 |
| 20.75 | 505.2 |
| 20.98 | 478.97 |
| 21.26 | 615.03 |
| 21.61 | 180 |
| 23.4 | 498.84 |
| 23.57 | 774.29 |
| 24.4 | 465.6 |
| 24.76 | 328.79 |
| 25.32 | 396.14 |
| 25.42 | 182.71 |
| 25.84 | 375.3 |
| 25.96 | 449.3 |
| 28.69 | 208.41 |
| 28.73 | 204.75 |
| 28.78 | 210.11 |
| 29.39 | 287.22 |
| 31.1 | 176.01 |
| 31.17 | 182.21 |
| 32.74 | 166.22 |

### b) 1-(2-(4-[6-Hydroxy-2-(4-hydroxy-phenyl)-benzo[b]thiophene-3-carbonyl]-phenoxy)-ethyl)-piperidinium chloride 0.37 methanol - 0.51 methylene chloride:

Boron trichloride in methylene chloride (1 M, 149 mL, 149 mmol) was cooled to 2 - 5 °C and 1-(2-{4-[6-Methoxy-2-(4-methoxy-phenyl)-benzo[b]thio-phene-3-carbony)]-phenoxy}-ethy))-piperidinium chloride (20.0 g, 37.2 mmol) was added in portions over a period of 70 min with stirring. The mixture was stirred for 35 min at 3 - 4 °C and allowed to warm to ambient temperature within 90 min. Stirring was continued for 49 h after which the mixture was cooled to 12 °C. Methanol (60 mL) was then added slowly (approx. 20 min) and the resulting suspension was heated at reflux for 0.75 h. The solution was allowed to reach 20 °C and the solid was filtered off. The crystalline residue was with methanol (6 x 10 mL) and dried at 45°C under reduced pressure to give 1-(2-{4-[6-Methoxy-2-(4-methoxy-phenyl)-benzo[bjthiophene-3-carbonyl]-phenoxy)-ethyl)-piperidinium chloride 0.37 methanol 0.51 methylene chloride. Yield: 20.14 g (34.9 mmol, 95.0 %, calcd.). Purity: HPLC: 98.01 % (HPLC). Methanol: 2.1 % by weight, corresponding to approx. 0.37 molar equivalents (GC). **Methylene chloride:** 7.7 % by weight, corresponding to approx. 0.51 molar equivalents (GC). **Assay:** 101.7 % (AgN0₃). **Mp.:** 213 - 221 °C. DSC: Peak at 197.47 °C, Peak at 221.81 °C, Peak at 334.13 °C. **lR** (KI3r, cm⁻¹): 3205, 2958, 2722_{;} 2558, 1653 (v_{c}=ₒ), 1597, 1547, 1500, 1467, 1437, 1418, 1343, 1309, 1250, 1166, 1065, 1037, 1020, 953, 938, 907, 840, 816, 808, 785, 738, 645, 635, 627, 594, 524. **XRPD_{.}** XRD data correspond to those given for Example 14 item a).

### c) 1-(2-(4-[6-Hydroxy-2-(4-hydroxy-phenyl)-benzo[b]thiophene-3-carbonyl]-phenoxy)-ethyl)-piperidinium chloride - 0.34 methanol - 0.62 methylene chloride from non-solvated crystalline 1-(2-{4-[6-Hydroxy-2-(4-hydroxy-phenyl)-benzo[b]thiaphene-3-carbonyl]-phenoxy)-ethyl)-piperldinium chloride:

A mixture of 1-(2-{4-[6-Hydroxy-2-(4-hydroxy-phenyl)-benzo[b]-thiophene-3-carbonyl]-phenoxy)-ethyl)-piperidinium chloride (12.1 g, 0.024 mol), prepared as described under example 15 item a, methanol (350 mL) and methylene chloride (230 mL) were heated to reflux. The resulting clear solution was allowed to cool to 45 °C and concentrated under reduced pressure. The resulting suspension was cooled to 2 °C with stirring. The solid was isolated, washed with a mixture of methanol and methylene chloride (60:40 v:v, 3 x 4 mL) and dried at 45 °C under reduced pressure (40 mbar) to give 1-(2-{4-[6-Hydroxy-2-(4-hydroxyphenyl)-benzo[b]thiophene-3-carbonyl]-phenoxy}-ethyl)-piperidinium chloride . 0.34 methanol 0.62 methylene chloride. Yield: 66 %. **Purity**: 99.10 % (HPLC). **Methanol:** 1.9 % by weight, corresponding to approx. 0.34 molar equivalents (GC). **Methylene chloride**: 9.2 % by weight, corresponding to approx. 0.62 molar equivalents (GC). **Mp..:** 255 - 259.1 °C **DSC**: first peak at 206.2 °C, second peak at 223.3 °C, third peak at 233.1 °C, fourth peak at 268.8 °C, fifth peak at 343.3 °C. **lR** (KBr, cm⁻¹): 3202, 2958, 2722, 1653 (v_{c}=ₒ), 1596, 1549, 1500, 1467, 1437, 1418, 1343, 1309, 1270, 1227, 1166, 1065, 1036, 1020, 953, 939, 907, 839, 816, 808, 785, 738, 645, 635, 627, 594, 524. XRPD . XRD data correspond to those given for Example 14 item a).

### d) 1-(2-(4-[6-Hydroxy-2-(4-hydroxy-phenyl)-benzo[b]thiophene-3-carbonyl]-phenoxy)-ethyl)-piperidinium chloride - 0.08 methanol - 0.29 toluene:

1-(2-{4-[6-Hydroxy-2-(4-hydroxy-phenyl)-benzo[b]thiophene-3-carbonyl]-phenoxy}-ethyl)-piperidinium chloride - 0.28 methanol 0.53 methylene chloride (60.0 g, 0.118 mol), prepared as described under example 14 item a was dissolved in a mixture of water (540 mL) and aq. sodium hydroxide (cc., 20 mL, 0.38 mol) and washed with toluene (4 x 500 mL). The aqueous phase was treated with methanol (550 mL) and hydrochloric acid (aq., cc., 37 mL, 444 mmol) was added dropwise at approx. 15 °C to adjust a pH of 2 with stirring. The resulting suspension was heated to reflux and treated with a mixture of methanol and water (50:50 v/v, 300 mL) and cooled to 2 °C with stirring. The solid was collected by filtration, washed with cold aqueous methanol (50:50 v/v, 2 x 25 mL), water (2 x 25 mL), and cold aqueous methanol (50:50 v/v, 2 x 25 mL). A sample (3.21 g) was dried at 50 °C under reduced pressure (40 mbar) to give 1-(2-{4-[6-hydroxy-2-(4-hydroxy-phenyl)-benzo[b]thiophene-3-carbonyl]-phenoxy}-ethyl)-piperidinium chloride - 0.08 methanol - 0.29 toluene. Yield: 51.2 g (100.4 mmol, 94.5 %, calc. dry). **Purity**: 98.94 % (HPLC). **Methanol:** 0.5 % by weight, corresponding to approx. 0.08 molar equivalents (GC). **Toluene:** 4.9 % by weight, corresponding to approx. 0.29 molar equivalents (GC). **Mp.:** 260.6 °C; DSC: first peak at 211.7 °C, second peak at 247.4, third peak at 267.0 °C, fourth peak at 332.9 °C. **IR** (KBr, cm⁻¹): 3203, 2958, 2722, 1653 (v_{c}=ₒ), 1597, 1548, 1501, 1467, 1437, 1419, 1343, 1309, 1250, 1166, 1065, 1037, 1021, 953, 939, 907, 840, 817, 808, 786, 738, 645, 635, 627, 594, 524. **XRPD:** the X-ray diffractogram exhibits signals at the following 2θ values:

| **2theta[°]** | **I/I₀** |
|---|---|
| 11.12 | 294.09 |
| 13.45 | 740.54 |
| 14.43 | 669.08 |
| 15.75 | 413.53 |
| 16.38 | 773.84 |
| 17.52 | 491.55 |
| 18.34 | 861.17 |
| 18.61 | 484.9 |
| 19.11 | 739.17 |
| 19.4 | 372.71 |
| 19.74 | 745.12 |
| 20.38 | 599.01 |
| 20.64 | 404.98 |
| 20.95 | 558.87 |
| 21.18 | 769.79 |
| 21.37 | 391.36 |
| 22.66 | 1000 |
| 23 | 268.21 |
| 23.47 | 419.87 |
| 23.72 | 667.65 |
| 24.1 | 315.09 |
| 24.17 | 356.16 |
| 24.36 | 305.51 |
| 25.08 | 513.78 |
| 25.51 | 217.57 |
| 25.64 | 477.11 |
| 25.89 | 257.51 |
| 28 | 268.99 |
| 29.02 | 239.51 |
| 29.28 | 222.56 |

### e) 1 -(2-(4-[6-Hydroxy-2-(4-hydroxy-phenyl)-benzo[b]thiophene-3-carbonyl]-phenoxy)-ethyl)-piperidinium chloride -0.35 methanol -0.67 methylene chloride

1-(2-{4-[6-Hydroxy-2-(4-hydroxy-phenyl)-benzo[b]thiophene-3-carbonyl]-phenoxy}-ethyl)-piperidinium chloride -0.35 methanol -0.67 methylene chloride was obtained in a similar manner as described under example 14 item a), employing 1-(2-{4-[6-Methoxy-2-(4-methoxyphenyl)-benzo[b]thiophene-3-carbonyl]-phenoxy)-ethyl)-piperidinium bromide instead of 1-(2-{4-[6-Methoxy-2-(4-methoxy-phenyl)-benzo[b]thiophene-3-carbonyl]-phenoxy}-ethyl)-piperidinium chloride.
**Methanol:** 1.93 % by weight, corresponding to approx. 0.35 molar equivalents (GC). **Methylene chloride**: 9.8 % by weight, corresponding to approx. 0.67 molar equivalents (GC).

### Example 15 .

### Non-solvated crystalline 1-(2-{4-[6-Hydroxy-2-(4-hydroxy-phenyl)-benzo[b]thiophene-3-carbonyl]-phenoxy)-ethyl)-piperidinium chloride

### a) Preparation by using a mixture of methanol/water (70:30 v/v) as the solvent:

1-(2-{4-[6-hydroxy-2-(4-hydroxy-phenyl)-benzo[b]thiophene-3-carbonyl]-phenoxy}-ethyl)-piperidinium chloride 0.34 methanol - 0.62 toluene, (44.14 g, 0.0865 mol, calcd. dry), prepared as described under example 14 item c, was dissolved in hot aqueous methanol (70:30 v/v, 610 mL). Approx. 50 mL of methanol was distilled off at 70 °C and a mixture of activated charcoal (3 g) and aqueous methanol (70:30 v/v, 50 mL) was added. The suspension was diluted with methanol (50 mL) and heated reflux to reflux. Insolubles were filtered off and the residue was washed with hot aqueous methanol (70:30 v/v, 10 mL). The combined filtrates were cooled 2 °C and stirring was continued for 2.3 h at 2 - 8 °C. The resulting solid was isolated and washed with aqueous methanol (70:30 v/v, 3 x 20 mL) to give 48.23 g of 1-(2-{4-[6-hydroxy-2-(4-hydroxy-phenyl)-benzo[b]thiophene-3-carbonyl]-phenoxy}-ethyl)-piperidinium chloride. Yield: 31.22 g (70.8 %, calcd. dry). **Purity**: 99.59 % (HPLC).

A sample (46.5 g) of the product as obtained above (30.1 g, 0.059 mol, calcd. dry) was dissolved in hot aqueous methanol (70:30 v/v, 330 mL) and a mixture of activated charcoal (3.0 g) in aqueous methanol (70:30 v/v, 30 mL) was added. The mixture was heated to reflux and insolubles were filtered off. The residue was washed with aqueous methanol (70:30 v/v, 10 mL). The combined filtrates were cooled to 2 °C and stirring was continued for a further 60 min at 2 - 5 °C. The solid was isolated, washed with cold aqueous methanol (70:30 v/v, 3 x 15 mL) and dried under reduced pressure for (50 ° C, 7 h). Yield: 22.93 g (44.96 mmol, 76.1 %). **Purity:** 99.70 %; all individual impurities: nmt 0.10 % (HPLC). **Water:** 0.16 % (KFT). **Assay** (NaOH): 99.9 %. **Assay (AgN0₃):** 99.8 %. **Sulphated ash**: 0.02 %. **Heavy metals:** nmt 20 ppm. **Residual solvents**: methanol (707 ppm).
Mp.: 260.0 °C. DSC: first peak at 266.1 °C, second peak at 331.7 °C.
¹**H NMR** (300 MHz, DMSO-d₆, ppm): 8 10.68 (b, 1 H, -NH⁺), 9.87 (m, 2 H, -OH), 7.70 (d, ³J = 9.0 Hz, 2 H, arom.), 7.38 (d, ⁴J = 2.2 Hz, 1 H, arom.), 7.26 (d, ³J = 8.8 Hz, 1 H, arom.), 7.17 (d,³J = 8.7 Hz, 2 H, arom.), 6.96 (d, ³J = 9.0 Hz, 2 H, arom.), 6.88 (dd, ³J = 8.8 Hz, ⁴J = 2.3 Hz, 1 H, arom.), 6.71 (d, ³J = 8.7 Hz, 2 H, arom.), 4.45 (m, 2 H, -OCH₂CH_{Z}NH-), 3.42 (m, 4 H, -NHCH₂CH₂CH₂-, -OCH₂CH₂NH-), 2.97 (m, 2 H, -NHCH₂CH₂CH₂-), 1.87 -.1.28 (m, 6 H, -CH₂CH₂CH₂-NR₂, -CH₂CH₂CH₂-);
¹³**C-{H}-NMR** (75 MHz, DMSO-d₆, ppm): δ 192.57 (>C=O), 161.64 (=C<), 157.93 (=C<), 155.53 (=C<), 140.61 (=C<), 139.22 (=C<)_{;} 132.19 (=C<), 131.77 (=CH-), 130.36 (=C<), 129.67 (=CH-), 129.57 (=C<), 123.70 (=C<), 123.22 (=CH-), 115.74 (=CH-), 115.27 (=CH-), 114.65 (=CH-), 107.14 (=CH-), 62.54 (-CH₂-), 54.47 (-CH₂-), 52.57 (-CH₂-), 22.26 (-CH₂-), 21.12 (-CH₂-)- **IR** (KBr, cm⁻¹): 3146, 2945, 2694, 1643 (v_{c}=ₒ), 1597 (vₐᵣₒₘ.)₋ 1540, 1500, 1468 (vₐᵣₒₘ.), 1358, 1259_{;} 1170, 1038, 1002, 906, 840, 807, 623. **XRPD**: the X-ray diffractogram exhibits signals at the following 28 values:

| **2theta[º]** | **I/I₀** |
|---|---|
| 12.78 | 150.93 |
| 14.45 | 696.72 |
| 15.75 | 372.19 |
| 16.26 | 158 |
| 18.61 | 140.55 |
| 19.13 | 332.59 |
| 19.62 | 95.86 |
| 19.78 | 226.51 |
| 20.41 | 155.52 |
| 21 | 344.2 |
| 21.18 | 715.36 |
| 21.38 | 488.6 |
| 21.65 | 139.21 |
| 21.87 | 131.02 |
| 22.48 | 206.29 |
| 22.67 | 1000 |
| 22.88 | 136.09 |
| 23.02 | 331.41 |
| 23.82 | 100.83 |
| 24.06 | 222.51 |
| 24.4 | 171.37 |
| 25.09 | 114.33 |
| 25.47 | 137.89 |
| 25.79 | 208.69 |
| 25.9 | 174.26 |
| 27.63 | 142.62 |
| 27.98 | 107.44 |
| 29.02 | 222.22 |
| 31.28 | 158.82 |
| 31.9 | 99.36 |

### b) Preparation by using a mixture of 1-propanol/water (75:25 v/v) as the solvent:

1-(2-{4-[6-Hydroxy-2-(4-hydroxy-phenyl)-benzo[b]thiophene-3-carbonyl]-phenoxy}-ethyl)-piperidinium chloride was also obtained in a similar manner as described under example 15 item a, employing 1-(2-{4-[6-Hydroxy-2-(4-hydroxy-phenyl)-benzo[b]thiophene-3-carbonyl]-phenoxy}-ethyl)-piperidinium chloride 0.28 methanol - 0.53 methylene chloride, obtained according to example 15 item a, and a mixture of 1-propanol and water (75:25 v/v) as solvent. Yield: 34 %. **Purity**: 99.28 % (HPLC). **DSC**: first peak at 266.3 °C, second peak at 335.4°C). **IR** (KBr, cm⁻¹): 3146, 2945, 2692, 1643 (v_{c}=ₒ), 1597, 1541, 1500, 1468, 1431, 1357, 1311, 1259, 1169, 1125, 1078, 1048, 1038, 1002, 952, 906, 840, 807, 762, 724, 706, 643, 623, 588, 529.

### Example 16

### Solvates of 1-(2-{4-[6-Hydroxy-2-(4-hydroxy-phenyl)-benzo[b]thiophene-3-carbonyl-phenoxy)-ethyl)-piperidinium bromide

### a) 1-(2-(4-[6-Hydroxy-2-(4-hydroxy-phenyl)-benzo[b]thlophene-3-carbonyl]-phenoxy)-ethyl)-piperidinium bromide 0.25 methanol - 0.52 methylene chloride:

Boron tribromide (51.6 g, 0.206.mol) in methylene chloride (20 mL) was added to a mixture of of 1-(2-{4-[6-Methoxy-2-(4-methoxy-phenyl)-benzo[b]thiophene-3-carbonyl]-phenoxy)-ethyl)-piperidinium bromide (40.0 g, 0.0687 mol), prepared as described under example 13 item a, and methylene chloride (560 mL) over a period of 2 h at 37 - 39 °C with stirring. Stirring was continued for 0.5 h at 39 °C and the mixture was cooled to 17 °C. Methanol (111 mL) was added slowly (0.5 h) at 17 - 32 °C and the resulting suspension was heated at reflux for 0.25 h. The suspension was allowed to cool to 15 - 20 °C and stirring was continued for 15 min. The solid was filtered off, washed with methanol (4 x 35 mL) and dried under reduced pressure (45 °C, 30 mbar, 3 h) to give 1-(2-{4-[6-Hydroxy-2-(4-hydroxy-phenyl)-benzo[b]thiophehe-3-carbonyl]-phen-oxy)-ethyl)-piperidinium bromide 0.25 methanol - 0.52 methylene chloride. Yield: 33.49 g (55.17 mmol, 80.4 %).
**Purity:** 97.29 % (HPLC). **Methanol:** 1.3 % by weight, corresponding to approx. 0.25 molar equivalents (GC). **Methylene chloride:** 7.3 % by weight, corresponding to approx. 0.52 molar equivalents (GC). **Mp.**: 250.4 °C. **Water**: 0.36 % (KFT). **Assay** (AgNO₃): 99.4 % (calcd. on non-solvated substance) **DSC:** first peak at 182.6 °C, second peak at 197.9 °C, third peak at 254.4 °C, fourth peak at 352.2 °C. **IR** (KBr, cm⁻¹): 3247, 2945, 2742, 1653 (v_{c}=ₒ), 1597, 1546, 1500, 1467, 1433, 1418, 1341, 1305, 1250, 1226, 1167, 1064, 1037, 1019, 952, 907, 839, 817, 808, 784, 738, 627, 523. **XRPD:** the X-ray diffractogram exhibits signals at the following 2θ values:

| **2theta[°]** | **I/I₀** |
|---|---|
| 10.82 | 183.94 |
| 13.47 | 450.42 |
| 14.11 | 305.45 |
| 15.92 | 415.8 |
| 16.43 | 524.47 |
| 17.11 | 198.42 |
| 17.57 | 955.66 |
| 18.76 | 750.64 |
| 18.96 | 592.19 |
| 19.06 | 805.32 |
| 19.52 | 300.68 |
| 19.75 | 280.67 |
| 20 | 186.3 |
| 20.73 | 251.74 |
| 20.98 | 665.41 |
| 21.18 | 984.83 |
| 23.06 | 224.76 |
| 23.39 | 503.31 |
| 23.54 | 999.33 |
| 24.37 | 476.01 |
| 24.53 | 219.79 |
| 25.28 | 541.05 |
| 25.88 | 411.61 |
| 28.55 | 158.07 |
| 28.64 | 179.27 |
| 28.74 | 186.54 |
| 29.4 | 249.82 |
| 31.07 | 257.25 |
| 31.71 | 218.43 |
| 32.68 | 153.25 |

### b) 1-(2-{4-[6-Hydroxy-2-(4-hydroxy-phenyl)-benzo[b]th iophene-3-carbonyl]-phenoxy}-ethyl)-piperidinium bromide 0.08 methanol 0.51 methylene chloride:

A flask was charged with non-solvated 1-(2-{4-[6-Hydroxy-2-(4-hydroxy-phenyl)-benzo[b]thiophene-3-carbonyl]-phenoxy}-ethyl)-piperidinium bromide (4.5 g, 0.008 mol) and a mixture of methanol and methylene chloride (1:1 v/v, 115 mL). The mixture was heated to reflux. The resulting clear solution was allowed to cool to 45 °C. The suspension was concentrated under reduced pressure (870 mbar) at 45 °C and cooled to 2 - 3 °C with stirring. The solid was isolated, washed a mixture of methanol and methylene chloride (1:1 v/v, 3 x 3 mL), and dried at 50 °C under reduced pressure to to give
1-(2-{4-[6-Hydroxy-2-(4-hydroxy-phenyl)-benzo[b]thiophene-3-carbonyl]-phen-oxy)-ethyl)-piperidinium bromide 0.08 methanol . 0.51 methylene chloride. Yield: 3.39 g (5.6 mmol, 70 %).
**Purity:** 99.36 % (HPLC). **Water:** 0.41 % (KFT). **Methanol:** 0.4 % by weight, corresponding to approx. 0.08 molar equivalents (GC). **Methylene chloride**: 7.3 % by weight, corresponding to approx. 0.51 molar equivalents (GC). **Mp.**: 256.5 - 258.4 °C; DSC: first peak at 180.1 °C, second peak at 199.6 °C, third peak at 219.7 °C, fourth peak at 263.9 °C, fifth peak at 359.2. **IR** (KBr, cm⁻¹): 3246, 2946, 2744, 2549, 1653 (v_{c}=ₒ), 1597, 1547, 1500, 1467, 1434, 1418, 1339, 1305, 1249, 1226, 1167, 1064, 1037, 1019, 952, 937, 907, 852, 839, 818, 808, 785, 739, 660, 645, 635, 627, 594, 524. **XRPD:** XRD data correspond to those given for Example 16 item a).

### c) 1-(2-(4-[6-Hydroxy-2-(4-hydroxy-phenyl)-benzo[b]thiophene-3-carbonyl]-phenoxy)-ethyl)-piperidinium bromide 0.16 methanol 0.64 chlorobenzene

Boron tribromide (21.5 mL, 85.8 mmol) in chlorobenzene (20 mL) was added slowly (40 min) to a mixture of 1-(2-{4-[6-Methoxy-2-(4-methoxy-phenyl)-benzo[b]thiophene-3-carbonyl]-phenoxy)-ethyl)-piperidinium bromide (10.0 g, 17.2 mmol) and chlorobenzene (80 mL) at 2 - 3 °C with stirring. The mixture was allowed to reach room temperature and stirring was continued for 44.5 h. Methanol (30 mL) was added slowly at 10 °C and the resulting suspension was allowed to reach room temperature. Stirring was continued for a further 60 min and the suspension was filtered. The solid was washed with methanol (6 x.5 mL) and dried at 45 °C under reduced pressure to give the title compound.
Yield: 7.63 g (13.75 mmol, 80.5 %, calcd. dry). Purity: 95.71 %. Methanol: 0.83 % by weight, corresponding to approx. 0.16 molar equivalents (GC). Chlorobenzene: 11.41 % by weight, corresponding to approx. 0.64 molar equivalents (GC).

### Example 17

### Non-solvated crystalline 1-(2-{4-[6-Hydroxy-2-(4-hydroxy-phenyl)-benzo[b]thiophene-3-carbonyl]-phenoxy)-ethyl)-piperidinium bromide

### Synthesis via 1-(2-{4-[6-Hydroxy-2-(4-hydroxy-phenyl)-benzo[b]thiophene-3-carbonyl]-phenoxy)-ethyl)-piperidinium bromide 0.16 methanol - 0.64 chlorobenzene

A sample (6.49 g, 11.7 mmol, calcd. dry) of the material obtained according to example 16 item c was dissolved in methanol (130 mL) and treated with activated charcoal (0.39 g). The mixture was heated to reflux, filtered, and the residue was washed with hot methanol (3 mL). The combined filtrates were cooled, seeded with appropriate material, and stored at 2 °C for 16.5 h. The solid was isolated, washed with 2 mL cold methanol (3 x 2 mL) and dried at 50°C under reduced pressure. Yield: 3.94 g (7.1 mmol, 60.7 %).
**Purity**: 99.25 % (HPLC).

A sample of the material (3.13 g, 5.64 mmol) was re-crystallized from methanol (69 ml) and stirred for 1.75 h at -6 to -10 °C. The solid was isolated, washed with cold methanol (3 x 1 mL) and dried at 50°C under reduced pressure. Yield: 2.13 g (3.84 mmol, 68.3 %).
**Purity**: 99.69 % (HPLC). **XRPD:** the X-ray diffractogram exhibits signals at the following 2θ values:

| **2theta[°]** | **I/I₀** |
|---|---|
| 12.32 | 179.64 |
| 14 | 450.33 |
| 14.68 | 124.93 |
| 15.02 | 334.5 |
| 18.31 | 139.86 |
| 19.14 | 467.48 |
| 19.28 | 319.2 |
| 19.7 | 259.65 |
| 20.93 | 828.37 |
| 21.22 | 262.49 |
| 21.91 | 125.76 |
| 22.47 | 339.99 |
| 22.6 | 1000 |
| 23.59 | 192 |
| 23.68 | 170.4 |
| 23.96 | 363.43 |
| 24.45 | 481.64 |
| 24.79 | 426.65 |
| 25.15 | 123.16 |
| 26.1 | 225.47 |
| 27.45 | 352.33 |
| 27.73 | 406.18 |
| 28.22 | 150.52 |
| 28.59 | 208.75 |
| 28.79 | 122.45 |
| 29.07 | 223.4 |
| 30.23 | 139.53 |
| 30.73 | 150.13 |
| 33.17 | 169.3 |
| 36.83 | 123.26 |

## Claims

1. A process for preparing a compound of formula VII' 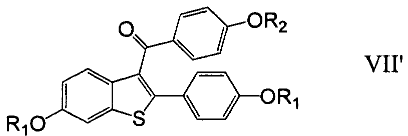 wherein R₁ and R₂ represent different hydroxy protecting groups which are independently from each other selectively removable, with the proviso that both -OR₁, and -OR₂ are substantially not cleavable in the presence of a Lewis acid at Friedel-Crafts acylation conditions,
wherein R₁ is selected from the group consisting of linear or branched alkyl, linear or branched arylalkyl and linear or branched alkenyl, and R₂ is selected from the group consisting of linear or branched alkylsulfonyl, arylsulfonyl and linear or branched alkylarylsulfonyl,
wherein a compound of formula VI' 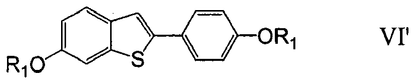 is reacted with a compound of formula V' 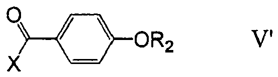 wherein R₁ and R₂ are defined as above, and
X represents Cl, Br, I or 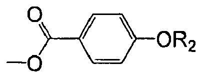 wherein R₂ is defined as above,
in the presence of a Lewis acid and a solvent.

2. The process according to claim 1, wherein a compound of formula VIII 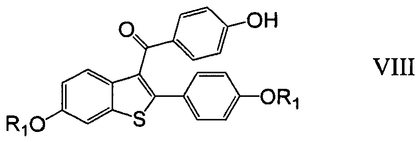 is prepared from the compound of formula VII' 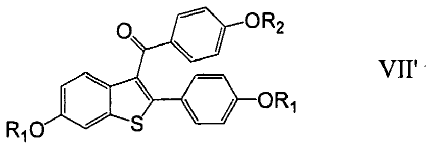 wherein R₁ is selected from the group consisting of linear or branched alkyl, linear or branched arylalkyl and linear or branched alkenyl, and R₂ is selected from the group consisting of linear or branched alkylsulfonyl, arylsulfonyl and linear or branched alkylarylsulfonyl,
the process comprising selective deprotection of the -OR₂ group.

3. The process according to claim 2, wherein a compound of formula X 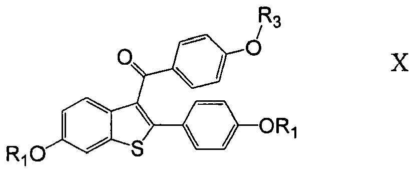 wherein R₁ is selected from the group consisting of linear or branched alkyl, linear or branched arylalkyl and linear or branched alkenyl, and
wherein R₃ is substituted or unsubstituted alkyl, substituted or unsubstituted aryl, substituted or unsubstituted arylalkyl, with the proviso that -OR₃ is substantially not cleavable at deprotection conditions for removal of R₁,
is prepared from the compound of formula VIII 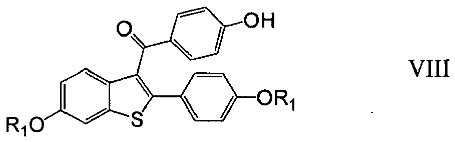 by O-alkylation with a compound of formula IX
Y-R₃ IX
wherein R₃ is defined as above
and Y is Cl, Br, I.

4. The process according to claim 3, wherein R₃ is 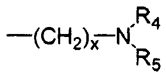 , wherein x = 1 to 20, preferably 1 to 15, more preferably 2 to 10, and in particular 2, and R₄ and R₅ independently from each other represent substituted or unsubstituted alkyl, substituted or unsubstituted aryl, substituted or unsubstituted arylalkyl, or R₄ and R₅ cooperatively with the N-atom form a part of a 5- to 10-membered ring, preferably a 5- to 7-membered ring, more preferably a 5- or 6-membered ring, in particular a 6-membered ring.

5. The process according to claim 3 or 4, wherein a compound of formula XI or a salt thereof 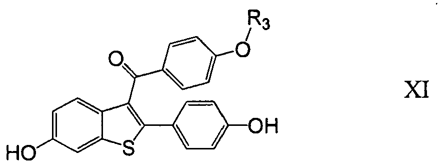 is prepared from the compound of formula X 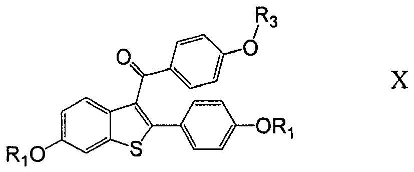 wherein R₁ is selected from the group consisting of linear or branched alkyl, linear or branched arylalkyl and linear or branched alkenyl, and
wherein R₃ is 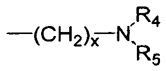 , wherein x = 1 to 20, preferably 1 to 15, more preferably 2 to 10, and in particular 2, and R₄ and R₅ independently from each other represent substituted or unsubstituted alkyl, substituted or unsubstituted aryl, substituted or unsubstituted arylalkyl, or R₄ and R₅ cooperatively with the N-atom form a part of a 5- to 10-membered ring, preferably a 5- to 7-membered ring, more preferably a 5- or 6-membered ring, in particular a 6-membered ring.
by deprotection of the -OR₁ groups.

6. A compound of formula VII" 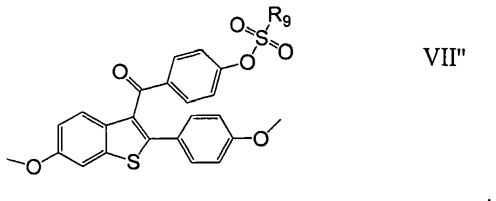 wherein R₉ is methyl, para-toluene or phenyl.

7. A process for preparing raloxifene or a pharmaceutical composition containing raloxifene with the formula 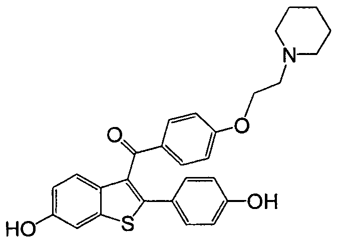 or a pharmaceutically acceptable salt thereof,
comprising the steps of:
a) providing a compound of formula VII' by a process according to claim 1,
b) converting said compound of formula VII' to compound of formula VIII by a process according to claim 2,
c) converting said compound of formula VIII to compound of formula X or optionally a salt thereof, wherein R₃ is 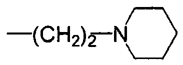 by a process according to claim 4, and
d) converting said compound of formula X or its salt to compound of formula XI according to claim 5 in order to obtain raloxifene or a pharmaceutically acceptable salt thereof,
e) optionally mixing said raloxifene or a pharmaceutically acceptable salt thereof with at least one pharmaceutically acceptable excipient.

8. Use of compounds of formula VII" as defined in claim 6 in a process for preparing raloxifene or a pharmaceutically acceptable salt thereof.

9. A process for preparing a crystalline solvate form of a salt of a compound of formula XI 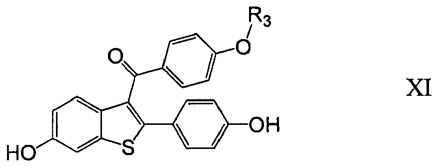 wherein R₃ is 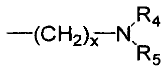 , wherein x = 1 to 20,
and R₄ and R₅ independently from each other represent substituted or unsubstituted alkyl, substituted or unsubstituted aryl, substituted or unsubstituted arylalkyl, or R₄ and R₅ cooperatively with the N-atom form a part of a 5- to 10-membered ring,
wherein a first solvent and a second solvent different from said first solvent are incorporated in said solvate form of the salt of the compound of formula XI with the proviso that a common solvate is formed,
wherein said process comprises the steps of:
a) carrying out a process according to claim 5 wherein a free base or a salt of a compound of formula XI is provided,
b) dissolving said compound provided in step a) in a mixture of said first solvent and said second solvent,
c) optionally distilling off a part of the volume of the mixture of first solvent and second solvent,
d) optionally seeding the mixture resulting from step b) or c) in order to induce crystallisation,
e) allowing a solvate form of a salt of a compound of formula XI to precipitate or to crystallize, optionally under agitation, and
f) separating the crystalline solvate form of a salt of a compound of formula XI.

10. A process for preparing a crystalline solvate form of a salt of a compound of formula XI 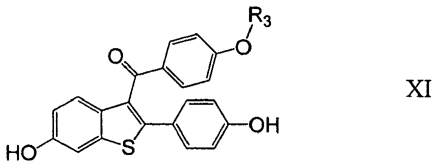 wherein R₃ is as defined in claim 9,
wherein a first solvent and a second solvent different from the first solvent is incorporated in said solvate form of the salt of the compound of formula XI with the proviso that a common solvate is formed,
wherein said process comprises the steps of:
i) carrying out a process according to claim 3 wherein via said process, a salt or a free base of a compound of formula X is
provided 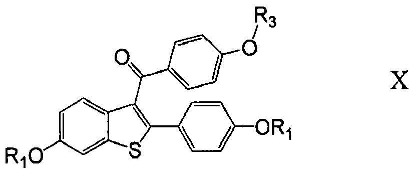 wherein R₁ is selected from the group consisting of linear or branched alkyl, linear or branched arylalkyl and linear or branched alkenyl, and
wherein R₃ is 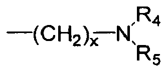 , wherein x = 1 to 20,
and R₄ and R₅ independently from each other represent substituted or unsubstituted alkyl, substituted or unsubstituted aryl, substituted or unsubstituted arylalkyl, or R₄ and R₅ cooperatively with the N-atom form a part of a 5- to 10-membered ring,
ii) deprotecting the -OR₁ groups of said salt of a compound of formula X in the presence of a second solvent in order to convert compound of formula X into compound of formula XI,
iii) adding a first solvent to a reaction mixture resulting from step ii),
iv) heating the reaction mixture resulting from step iii),
v) cooling and optionally agitating the reaction mixture resulting from step iv), and
vi) separating the solvate form of a salt of a compound of formula XI.

11. The process according to claim 10, wherein in step i) a compound of formula X' is provided 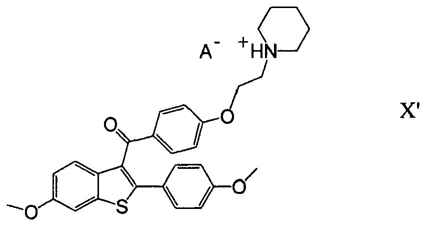 wherein A⁻ is Cl⁻ or Br.

12. The process according to claim 11, wherein the compound of formula X' is provided in crystalline form,
preferably a compound of formula X' wherein A- is Cl⁻ and whose X-ray diffraction (XRD) pattern has at least signals at the following 2θ values (±0.5° respectively):
| | | | | | | | | | | |
|---|---|---|---|---|---|---|---|---|---|---|
| **2theta[°]** | 11.41 | 13.07 | 16.74 | 19.27 | 19.39 | 21.56 | 23.42 | 24 | 24.63 | 26.05 |
; or a compound of formula X' wherein A⁻ is Br⁻ and whose X-ray diffraction (XRD) pattern has at least signals at the following 2θ values (±0.5° respectively):
| | | | | | | | | | | |
|---|---|---|---|---|---|---|---|---|---|---|
| **2theta[°]** | 7.41 | 13.05 | 16.64 | 19.07 | 19.22 | 21.45 | 23.31 | 24.34 | 25.81 | 26.62 |

13. The process according to any one of claims 9 to 12, wherein the crystalline solvate is **characterized by** one or more of the following features (x) to (z):
(x) the salt is derived from an organic or inorganic acidselected from the group consisting of oxalic acid, succinic acid, lactic acid, malonic acid, HCl, HBr and H₂SO₄, more preferably HCl, HBr and H₂SO₄, even more preferably HCl and HBr, and in particular HBr;
(y) said first solvent is incorporated into the salt of compound of formula XI in an amount of percent by weight which is lower than the amount of percent by weight of the second solvent; and/or wherein the crystalline solvate form of a salt of compound of formula XI comprises said first solvent in an amount of about 0.1 to 8 percent by weight and said second solvent in an amount of about 3 to 15 percent by weight relative to the solvate form of a salt of compound of formula XI;
(z) said first solvent is an alcohol and said second solvent is a halocarbon, preferably said first solvent is a C₁-C₄-alcohol and said second solvent is a C₁-C₁₀-halocarbon optionally comprising a benzene ring, more preferably said first solvent is methanol or ethanol and said second solvent is methylene chloride, chloroform or chlorobenzene, in particular said first solvent is methanol and said second solvent is methylene chloride.

14. A process for preparing a non-solvated pharmaceutically acceptable salt of a compound of formula XI 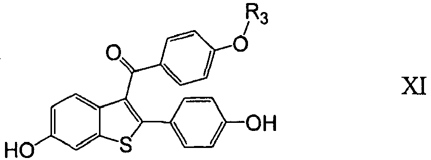 wherein R₃ is 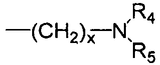 wherein x = 1 to 20,
and R₄ and R₅ independently from each other represent substituted or unsubstituted alkyl, substituted or unsubstituted aryl, substituted or unsubstituted arylalkyl, or R₄ and R₅ cooperatively with the N-atom form a part of a 5- to 10-membered ring,
comprising the steps of:
i) carrying out a process according to any one of claims 9 to 13 to provide a crystalline solvate form of a pharmaceutically acceptable salt of a compound of formula XI
ii) dissolving said crystalline solvate form provided in step i) in an organic solvent, wherein said organic solvent forms an azeotrope with at least one solvent incorporated in said solvate form,
iii) optionally seeding the mixture resulting from step ii) in order to induce crystallisation,
iv) allowing the pharmaceutically acceptable salt of compound of formula XI to precipitate or to crystallize, and
v) separating the pharmaceutically acceptable salt of a compound of formula XI.

15. The process according to claim 14, wherein the crystalline solvate form is **characterized by** one or more of the following features (a) to (c):
(a) said crystalline solvate form comprises a first solvent and a second solvent different from the first solvent, wherein said first solvent and said second solvent are incorporated in the pharmaceutically acceptable salt of a compound of formula XI;
(b) said first solvent is incorporated into the salt of compound of formula XI in an amount of percent by weight which is lower than the amount of percent by weight of the second solvent; and/or wherein the crystalline solvate form of a salt of compound of formula XI comprises said first solvent in an amount of about 0.1 to 8 percent by weight and said second solvent in an amount of about 3 to 15 percent by weight relative to the solvate form of a salt of compound of formula XI;
(c) said first solvent is an alcohol and said second solvent is a hydrocarbon or halocarbon, preferably said first organic solvent is a C₁-C₄-alcohol and said second organic solvent is a C₆-C₁₀-hydrocarbon comprising a benzene moiety or a C₁-C₁₀-halocarbon optionally comprising a benzene moiety, more preferably said first solvent is methanol or ethanol and said second solvent is toluene, methylene chloride, chloroform or chlorobenzene, even more preferably said first solvent is methanol and said second solvent is toluene, methylene chloride or chloroform, and in particular, said first solvent is methanol and said second solvent is methylene chloride.

16. The process according to claim 14 or 15, **characterized by** either one or a combination of the following procedural features (d) to (f):
(d) the azeotrope formed in step ii) forms under normal or reduced pressure, preferably under reduced pressure;
(e) in step ii), the resulting mixture is heated prior to step iii), preferably heating is under reflux conditions;
(f) solvent(s) incorporated into said solvate form of a pharmaceutically acceptable salt of a compound of formula XI is/are removed by azeotropic distillation of the mixture resulting from step ii), preferably said azeotropic distillation is carried out at reduced pressure.

17. The process according to any one of claims 14 to 16, **characterized by** either one or a combination of the following procedural features (g) to (i):
(g) said organic solvent of step ii) is an alcohol, preferably a water-miscible alcohol, more preferably a C₁-C₄-alcohol, even more preferably methanol or ethanol, and in particular methanol;
(h) said organic solvent of step ii) comprises a predetermined amount of water, preferably said amount of water is predetermined by a volume ratio of water and organic solvent of 0 to 0.5, preferably 0.24 to 0.44, more preferably 0.27 to 0.40, and in particular 0.29 to 0.38;
(i) said water used in step ii) is potable water, preferably deionized water, more preferably reverse osmosis water.

18. The process according to any one of claims 14 to 17, **characterized by** either one or a combination of the following features (j) and (k):
(j) the acid forming the pharmaceutically acceptable salt of a compound of formula XI whereupon said solvate form is based is selected from the group of organic or inorganic acids, preferably from the group consisting of oxalic acid, succinic acid, lactic acid, malonic acid, HCl, HBr and H₂SO₄, more preferably HCl, HBr and H₂SO₄, even more preferably HCl and HBr, and in particular HBr;
(k) said pharmaceutically acceptable salt of a compound of formula XI obtained in step v) has a purity of > 98% measured by HPLC, more preferably > 99%, even more preferably > 99.2%, yet even more preferably > 99.4%, and in particular > 99.6%.

## Patentansprüche

1. Ein Verfahren zur Herstellung einer Verbindung der Formel VII' 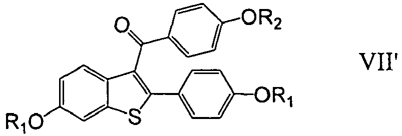 worin R₁ und R₂ verschiedene Hydroxyschutzgruppen darstellen, welche unabhängig voneinander selektiv entfernbar sind, mit der Maßgabe, dass sowohl -OR₁ als auch -OR₂ in Gegenwart von einer Lewis-Säure unter Friedel-Crafts Acylierungsbedingungen im Wesentlichen nicht spaltbar sind,
wobei R₁ aus der aus linearem oder verzweigtem Alkyl, linearem oder verzweigtem Arylalkyl und linearem oder verzweigtem Alkenyl bestehenden Gruppe ausgewählt wird, und R₂ aus der aus linearem oder verzweigtem Alkylsulfonyl, Arylsulfonyl und linearem oder verzeigtem Alkylarylsulfonyl bestehenden Gruppe ausgewählt wird,
wobei eine Verbindung der Formel VI' 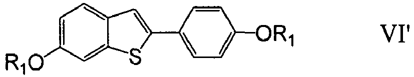 mit einer Verbindung der Formel V' 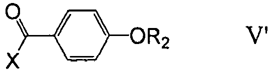 , worin R₁ und R₂ wie oben definiert sind, und
X Cl, Br, I oder 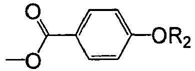 darstellt, wobei R₂ wie oben definiert ist,
in der Gegenwart einer Lewis-Säure und einem Lösungsmittel reagiert wird.

2. Verfahren gemäß Anspruch 1, wobei eine Verbindung der Formel VIII 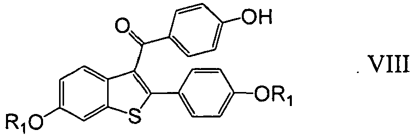 aus der Verbindung der Formel VII' 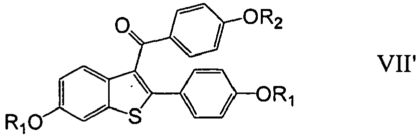 , worin R₁ aus der aus linearem oder verzweigtem Alkyl, linearem oder verzweigtem Arylalkyl und linearem oder verzweigtem Alkenyl bestehenden Gruppe ausgewählt wird, und R₂ aus der aus linearem oder verzweigtem Alkylsulfonyl, Arylsulfonyl und linearem oder verzeigtem Alkylarylsulfonyl bestehenden Gruppe ausgewählt wird,
hergestellt wird, wobei das Verfahren selektive Entschützung der -OR₂ Gruppe umfasst.

3. Verfahren gemäß Anspruch 2, wobei eine Verbindung der Formel X 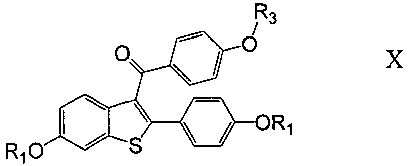 , worin R₁ aus der aus linearem oder verzweigtem Alkyl, linearem oder verzweigtem Arylalkyl und linearem oder verzweigtem Alkenyl bestehenden Gruppe ausgewählt wird, und worin R₃ substituiertes oder unsubstituiertes Alkyl, substituiertes oder unsubstituiertes Aryl oder substituiertes oder unsubstituiertes Arylalkyl darstellen, mit der Maßgabe, dass -OR₃ unter Entschützungsbedingungen zur Entfernung von R₁ im Wesentlichen nicht spaltbar ist,
aus der Verbindung der Formel VIII 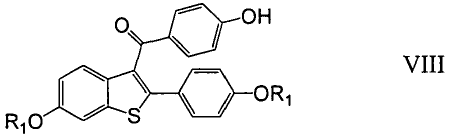 durch O-Alkylierung mit einer Verbindung der Formel IX
Y-R₃ IX
, worin R₃ wie oben definiert ist,
und Y gleich Cl, Br, I ist, hergestellt wird.

4. Verfahren gemäß Anspruch 3, wobei R₃ 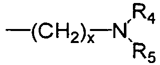 ist, wobei x = 1 bis 20, bevorzugt 1 bis 15, weiter bevorzugt 2 bis 10, und insbesondere 2,
und R₄ und R₅ unabhängig voneinander substituiertes oder unsubstituiertes Alkyl, substituiertes oder unsubstituiertes Aryl oder substituiertes oder unsubstituiertes Arylalkyl darstellen, oder R₄ und R₅ bilden zusammen mit dem N-Atom einen Teil eines 5- bis 10-gliedrigen Rings, bevorzugt eines 5- bis 7-gliedrigen Rings, weiter bevorzugt eines 5- oder 6-gliedrigen Rings, insbesondere eines 6-gliedrigen Rings.

5. Verfahren gemäß Anspruch 3 oder 4, wobei eine Verbindung der Formel XI 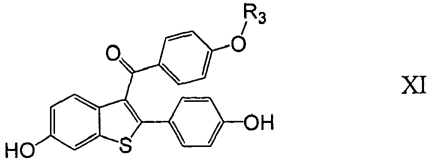 oder ein Salz davon
aus der Verbindung der Formel X 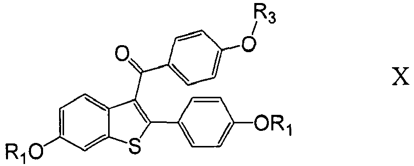 , worin R₁ aus der aus linearem oder verzweigtem Alkyl, linearem oder verzweigtem Arylalkyl und linearem oder verzweigtem Alkenyl bestehenden Gruppe ausgewählt wird, und
worin R₃ 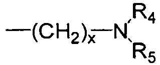 ist, wobei x = 1 bis 20, bevorzugt 1 bis 15, weiter bevorzugt 2 bis 10, und insbesondere 2,
und R₄ und R₅ unabhängig voneinander substituiertes oder unsubstituiertes Alkyl, substituiertes oder unsubstituiertes Aryl oder substituiertes oder unsubstituiertes Arylalkyl darstellen, oder R₄ und R₅ bilden zusammen mit dem N-Atom einen Teil eines 5- bis 10-gliedrigen Rings, bevorzugt eines 5- bis 7-gliedrigen Rings, weiter bevorzugt eines 5- oder 6-gliedrigen Rings, insbesondere eines 6-gliedrigen Rings,
durch Entschützen der -OR₁ Gruppen hergestellt wird.

6. Eine Verbindung der Formel VII" 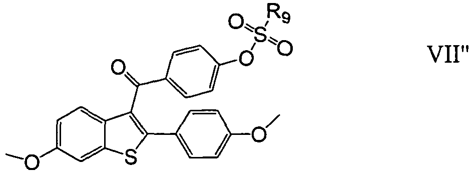 worin R₉ gleich Methyl, para-Toluol oder Phenyl ist.

7. Ein Verfahren zur Herstellung von Raloxifen oder einer pharmazeutischen Zusammensetzung, die Raloxifen mit der Formel 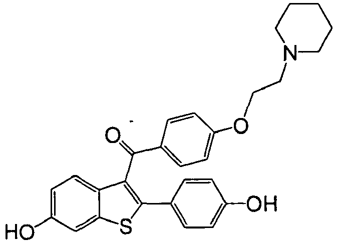 oder ein pharmazeutisch akzeptables Salz davon enthält,
umfassend die Schritte:
a) Bereitstellen einer Verbindung der Formel VII' durch ein Verfahren gemäß Anspruch 1,
b) Überführen der Verbindung der Formel VII' in die Verbindung der Formel VIII durch ein Verfahren gemäß Anspruch 2,
c) Überführen der Verbindung der Formel VIII in die Verbindung der Formel X oder optional in ein Salz davon, worin R₃ 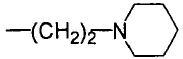 ist, durch ein Verfahren gemäß Anspruch 4, und
d) Überführen der Verbindung der Formel X oder dessen Salz in die Verbindung der Formel XI gemäß Anspruch 5, um Raloxifen oder ein pharmazeutisch akzeptables Salz davon zu erhalten,
e) optional Mischen des Raloxifens oder eines pharmazeutisch akzeptablen Salzes davon mit mindestens einem pharmazeutisch akzeptablen Hilfsstoff.

8. Verwendung der wie in Anspruch 6 definierten Verbindung der Formel VII" in einem Verfahren zur Herstellung von Raloxifen oder eines pharmazeutisch akzeptablen Salzes davon.

9. Ein Verfahren zur Herstellung einer kristallinen Solvatform eines Salzes einer Verbindung der Formel XI 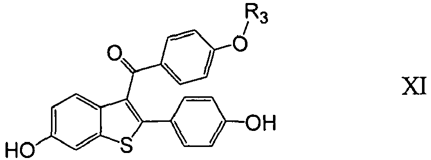 worin R₃ 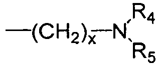 ist, wobei x = 1 bis 20,
und R₄ und R₅ unabhängig voneinander substituiertes oder unsubstituiertes Alkyl, substituiertes oder unsubstituiertes Aryl oder substituiertes oder unsubstituiertes Arylalkyl darstellen, oder R₄ und R₅ bilden zusammen mit dem N-Atom einen Teil eines 5- bis 10-gliedrigen Rings,
wobei ein erstes Lösungsmittel und ein zweites, vom ersten Lösungsmittel verschiedenes Lösungsmittel in die Solvatform der Verbindung der Formel XI inkorporiert wird, mit der Maßgabe, dass ein gemeinsames Solvat gebildet wird,
wobei das Verfahren die Schritte umfasst:
a) Ausführen eines Verfahrens gemäß Anspruch 5, wobei eine freie Base oder ein Salz einer Verbindung der Formel XI bereitgestellt wird,
b) Lösen der im Schritt a) bereitgestellten Verbindung in einer Mischung eines ersten Lösungsmittels und eines zweiten Lösungsmittels,
c) optional Abdestillieren eines Teils des Volumens der Mischung aus erstem Lösungsmittel und zweiten Lösungsmittel,
d) optional Keimbildung der aus Schritt b) oder c) resultierenden Mischung, um Kristallisation zu induzieren,
e) Präzipitieren oder Kristallisieren lassen einer Solvatform eines Salzes einer Verbindung der Formel XI, optional unter Agitation, und
f) Abtrennen der kristallisierten Solvatform eines Salzes der Verbindung der Formel XI.

10. Ein Verfahren zur Herstellung einer kristallinen Solvatform eines Salzes einer Verbindung der Formel XI 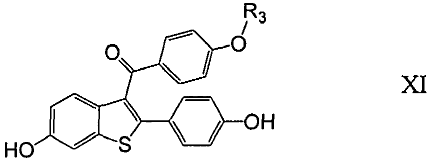 worin R₃ wie in Anspruch 9 definiert ist,
wobei ein erstes Lösungsmittel und ein zweites, vom ersten Lösungsmittel verschiedenes Lösungsmittel in die Solvatform des Salzes der Verbindung der Formel XI inkorporiert wird, mit der Maßgabe, dass ein gemeinsames Salz gebildet wird,
wobei das Verfahren die Schritte umfasst:
i) Ausführen eines Verfahrens gemäß Anspruch 3, wobei über das Verfahren ein Salz oder eine freie Base einer Verbindung der Formel X 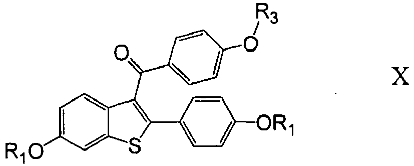 bereitgestellt wird,
worin R₁ aus der aus linearem oder verzweigtem Alkyl, linearem oder verzweigtem Arylalkyl und linearem oder verzweigtem Alkenyl bestehenden Gruppe ausgewählt wird, und
worin R₃ 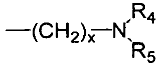 ist, wobei x = 1 bis 20,
und R₄ und R₅ unabhängig voneinander substituiertes oder unsubstituiertes Alkyl, substituiertes oder unsubstituiertes Aryl oder substituiertes oder unsubstituiertes Arylalkyl darstellen, oder R₄ und R₅ bilden zusammen mit dem N-Atom einen Teil eines 5- bis 10-gliedrigen Rings,
ii) Entschützen der -OR₁ Gruppen des Salzes der Verbindung der Formel X in der Gegenwart eines zweiten Lösungsmittels, um die Verbindung der Formel X in die Verbindung der Formel XI zu überführen,
iii) Zugabe eines ersten Lösungsmittels zu einer aus Schritt ii) resultierenden Reaktionsmischung,
iv) Erwärmen der aus Schritt iii) resultierenden Reaktionsmischung,
v) Abkühlen und optional Agitieren der aus Schritt iv) resultierenden Reaktionsmischung, und
vi) Abtrennen der Solvatform eines Salzes einer Verbindung der Formel XI.

11. Verfahren gemäß Anspruch 10, wobei in Schritt i) eine Verbindung der Formel X' 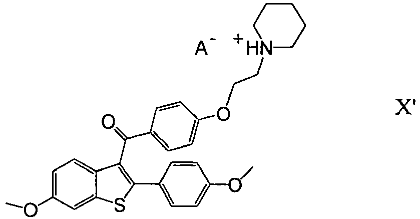 bereitgestellt wird, worin A⁻ gleich Cl⁻ oder Br⁻ ist.

12. Verfahren gemäß Anspruch 11, wobei die Verbindung der Formel X' in kristalliner Form bereitgestellt wird,
bevorzugt eine Verbindung der Formel X' worin A⁻ gleich Cl⁻ ist und deren Röntgenbeugungs (XRD)-Muster mindestens Signale bei den folgenden 2θ Werten (jeweils ±0.5°) aufweist:
| | | | | | | | | | | |
|---|---|---|---|---|---|---|---|---|---|---|
| **2theta[°]** | 11.41 | 13.07 | 16.74 | 19.27 | 19.39 | 21.56 | 23.42 | 24 | 24.63 | 26.05 |
; oder eine Verbindung der Formel X' worin A⁻ gleich Br⁻ ist und deren Röntgenbeugungs (XRD)-Muster mindestens Signale bei den folgenden 2θ Werten (jeweils ±0.5°) aufweist:
| | | | | | | | | | | |
|---|---|---|---|---|---|---|---|---|---|---|
| **2theta[°]** | 7.41 | 13.05 | 16.64 | 19.07 | 19.22 | 21.45 | 23.31 | 24.34 | 25.81 | 26.62 |

13. Verfahren gemäß irgendeinem der Ansprüche 9 bis 12, wobei das kristalline Solvat durch eines oder mehrere der folgenden Merkmale (x) bis (z) gekennzeichnet ist:
(x) das Salz ist abgeleitet von einer organischen oder anorganischen Säure, die aus der aus Oxalsäure, Bernsteinsäure, Malonsäure, HCl, HBr und H₂SO₄ bestehenden Gruppe, weiter bevorzugt HCl, HBr und H₂SO₄, noch weiter bevorzugt HCl und HBr, und insbesondere HBr ausgewählt ist;
(y) das erste Lösungsmittel wird in das Salz der Verbindung der Formel XI zu einem Gewichtsprozentgehalt inkorporiert, der geringer als der Gewichtsprozentgehalt des zweiten Lösungsmittels ist; und/oder wobei die kristalline Solvatform eines Salzes der Verbindung der Formel XI das erste Lösungsmittel in einer Menge von etwa 0,1 bis 8 Gew.-% und das zweite Lösungsmittel in einer Menge von etwa 3 bis 15 Gew.-% bezogen auf die Solvatform eines Salzes der Verbindung der Formel XI umfasst;
(z) das erste Lösungsmittel ist ein Alkohol und das zweite Lösungsmittel ist ein Halogenkohlenwasserstoff, bevorzugt ist das erste Lösungsmittel ein C₁-C₄-Alkohol und das zweite Lösungsmittel ist ein optional einen Benzolring umfassender C₁-C₁₀- Halogenkohlenwasserstoff, weiter bevorzugt ist das erste Lösungsmittel Methanol oder Ethanol und das zweite Lösungsmittel ist Methylenchlorid, Chloroform oder Chlorbenzol, insbesondere ist das erste Lösungsmittel Methanol und das zweite Lösungsmittel ist Methylenchlorid.

14. Ein Verfahren zur Herstellung eines nicht-solvatisierten pharmazeutisch akzeptablen Salzes einer Verbindung der Formel XI 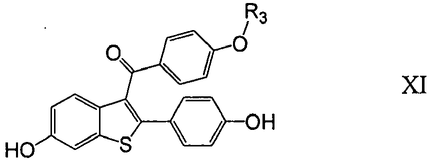 worin R₃ 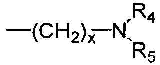 ist, wobei x = 1 bis 20,
und R₄ und R₅ unabhängig voneinander substituiertes oder unsubstituiertes Alkyl, substituiertes oder unsubstituiertes Aryl oder substituiertes oder unsubstituiertes Arylalkyl darstellen, oder R₄ und R₅ bilden zusammen mit dem N-Atom einen Teil eines 5- bis 10-gliedrigen Rings,
umfassend die Schritte:
i) Ausführen eines Verfahrens gemäß irgendeinem der Ansprüche 9 bis 13, um eine kristalline Solvatform eines pharmazeutisch akzeptablen Salzes einer Verbindung der Formel XI bereitzustellen,
ii) Lösen der im Schritt i) bereitgestellten kristallinen Solvatform in einem organischen Lösungsmittel, wobei das organische Lösungsmittel mit mindestens einem in der Solvatform inkorporierten Lösungsmittel ein Azeotrop bildet,
iii) optional Keimbildung der aus Schritt ii) resultierenden Mischung, um Kristallisation zu induzieren,
iv) Präzipitieren oder Kristallisieren lassen des pharmazeutisch akzeptablen Salzes der Verbindung der Formel XI, und
v) Abtrennen des pharmazeutisch akzeptablen Salzes einer Verbindung der Formel XI.

15. Verfahren gemäß Anspruch 14, wobei die kristalline Solvatform durch eines oder mehrere der folgenden Merkmale (a) bis (c) gekennzeichnet ist:
(a) die kristalline Solvatform umfasst ein erstes Lösungsmittel und ein zweites, vom ersten Lösungsmittel verschiedenes Lösungsmittel, wobei das erste Lösungsmittel und das zweite Lösungsmittel in das pharmazeutisch akzeptable Salz einer Verbindung der Formel XI inkorporiert werden;
(b) das erste Lösungsmittel wird in das Salz der Verbindung der Formel XI zu einem Gewichtsprozentgehalt inkorporiert, die geringer als der Gewichtsprozentgehalt des zweiten Lösungsmittels ist; und/oder wobei die kristalline Solvatform eines Salzes der Verbindung der Formel XI das erste Lösungsmittel in einer Menge von etwa 0,1 bis 8 Gew.-% und das zweite Lösungsmittel in einer Menge von etwa 3 bis 15 Gew.-% bezogen auf die Solvatform eines Salzes der Verbindung der Formel XI umfasst;
(c) das erste Lösungsmittel ist ein Alkohol und das zweite Lösungsmittel ist ein Halogenkohlenwasserstoff, bevorzugt ist das erste Lösungsmittel ein C₁-C₄-Alkohol und das zweite Lösungsmittel ist ein optional einen Benzolring umfassender C₁-C₁₀- Halogenkohlenwasserstoff, weiter bevorzugt ist das erste Lösungsmittel Methanol oder Ethanol und das zweite Lösungsmittel ist Toluol, Methylenchlorid, Chloroform oder Chlorbenzol, noch weiter bevorzugt ist das erste Lösungsmittel Methanol und das zweite Lösungsmittel Toluol, Methylenchlorid oder Chloroform, und insbesondere ist das erste Lösungsmittel Methanol und das zweite Lösungsmittel ist Methylenchlorid.

16. Verfahren gemäß Anspruch 14 oder 15, **gekennzeichnet durch** entweder eines oder eine Kombination der folgenden Verfahrensmerkmale (d) bis (f):
(d) das in Schritt ii) gebildete Azeotrop bildet sich unter normalem oder reduziertem Druck, bevorzugt unter reduziertem Druck;
(e) in Schritt ii) wird die resultierende Mischung vor Schritt iii) erwärmt, bevorzugt findet Erwärmung unter Rückflussbedingungen statt;
(f) in die Solvatform eines pharmazeutisch akzeptablen Salzes einer Verbindung der Formel XI inkorporierte(s) Lösungsmittel wird/werden **durch** azeotrope Abdestillation von der aus Schritt ii) resultierenden Mischung entfernt, bevorzugt wird die azeotrope Abdestillation bei reduziertem Druck durchgeführt.

17. Verfahren gemäß irgendeinem der Ansprüche 14 bis 16, **gekennzeichnet durch** entweder eines oder eine Kombination der folgenden Verfahrensmerkmale (g) bis (i):
(g) das organische Lösungsmittel des Schritts ii) ist ein Alkohol, bevorzugt ein wassermischbarer Alkohol, weiter bevorzugt ein C₁-C₄-Alkohol, noch weiter bevorzugt Methanol oder Ethanol, und insbesondere Methanol;
(h) das organische Lösungsmittel des Schritts ii) umfasst eine vorgegebene Wassermenge, bevorzugt wird die Wassermenge **durch** eine Volumenverhältnis von Wasser und organischem Lösungsmittel von 0 bis 0,5, bevorzugt 0,24 bis 0,44, weiter bevorzugt 0,27 bis 0,40, und insbesondere 0,29 bis 0,38 vorgegeben;
(i) das in Schritt ii) verwendete Wasser ist Trinkwasser, bevorzugt entionisiertes Wasser, weiter bevorzugt Umkehrosmosewasser.

18. Verfahren gemäß irgendeinem der Ansprüche 14 bis 17, **gekennzeichnet durch** entweder eines oder eine Kombination der folgenden Merkmale (j) und (k):
(j) die Säure, die das pharmazeutisch akzeptable Salz einer Verbindung der Formel XI worauf die Solvatform basiert, bildet, wird aus der Gruppe aus organischen oder anorganischen Säuren, bevorzugt aus der aus Oxalsäure, Bernsteinsäure, Milchsäure, Malonsäure, HCI, HBr und H₂SO₄ bestehenden Gruppe, weiter bevorzugt HCl, HBr und H₂SO₄, noch weiter bevorzugt HCl und HBr, und insbesondere HBr ausgewählt;
(k) das in Schritt v) erhaltene pharmazeutisch akzeptable Salz einer Verbindung der Formel XI weist eine mittels HPLC gemessene Reinheit > 98% auf, weiter bevorzugt > 99%, noch weiter bevorzugt > 99,2%, besonders bevorzugt > 99,4%, und insbesondere > 99,6%.

## Revendications

1. Procédé de préparation d'un composé de formule VII' 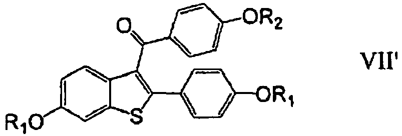 dans laquelle R₁ et R₂ représentent différents groupes protecteurs hydroxyle qui sont indépendamment l'une de l'autre éliminables sélectivement, à la condition que les deux éléments -OR₁ et -OR₂ soient sensiblement non clivables en présence d'un acide de Lewis dans les conditions d'acylation de Friedel-Craft,
dans laquelle R₁ est choisi dans le groupe constitué par un alkyle linéaire ou ramifié, un arylalkyle linéaire ou ramifié et un alcényle linéaire ou ramifié, et R₂ est choisi dans le groupe constitué par un alkylsulfonyle linéaire ou ramifié, un arylsulfonyle, et un alkylarylsulfonyle linéaire ou ramifié,
dans laquelle l'on fait réagir un composé de formule VI' 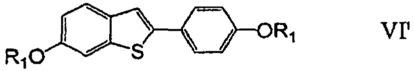 avec un composé de formule V' 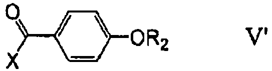 dans laquelle R₁ et R₂ sont tels que définis ci-dessus, et
X représente CI, Br, I ou 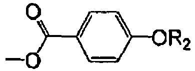 où R₂ est tel que défini ci-dessus,
en présence d'un acide de Lewis et d'un solvant.

2. Procédé selon la revendication 1, dans lequel un composé de formule VIII 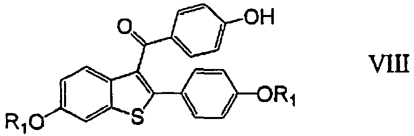 est préparé à partir du composé de formule VII' 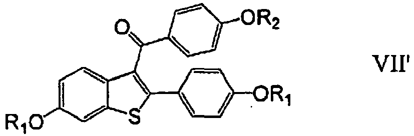 dans laquelle R₁ est choisi dans le groupe constitué par un alkyle linéaire ou ramifié, un arylalkyle linéaire ou ramifié et un alcényle linéaire ou ramifié, et R₂ est choisi dans le groupe constitué par un alkylsulfonyle linéaire ou ramifié, un arylsulfonyle, et un alkylarylsulfonyle linéaire ou ramifié,
ledit procédé comprenant une déprotection sélective du groupe -OR₂.

3. Procédé selon la revendication 2, dans lequel un composé de formule X 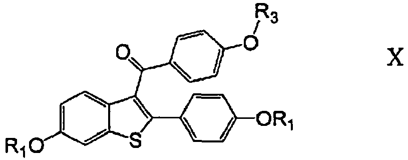 dans laquelle R₁ est choisi dans le groupe constitué par un alkyle linéaire ou ramifié, un arylalkyle linéaire ou ramifié et un alcényle linéaire ou ramifié, et
dans laquelle R₃ est un alkyle substitué ou non substitué, un aryle substitué ou non substitué, un arylalkyle substitué ou non substitué, à la condition que -OR₃ soit sensiblement non clivable dans les conditions de déprotection pour l'élimination de R₁,
est préparé à partir du composé de formule VIII 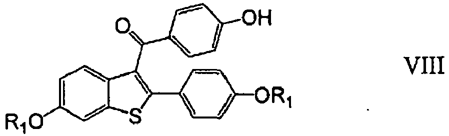 par O-alkylation avec un composé de formule IX
Y-R₃ IX
dans laquelle R₃ est tel que défini ci-dessus
et Y est CI, Br, I.

4. Procédé selon la revendication 3, dans lequel R₃ est 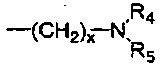 où x = 1 à 20, de préférence 1 à 15, plus préférentiellement 2 à 10, et en particulier 2,
et R₄ et R₅, indépendamment l'un de l'autre, représentent un alkyle substitué ou non substitué, un aryle substitué ou non substitué, un arylalkyle substitué ou non substitué, ou R₄ et R₅ forment, coopérativement avec l'atome de N, une partie d'un cycle de 5 à 10 éléments, de préférence un cycle de 5 à 7 éléments, plus préférentiellement un cycle de 5 ou 6 éléments, en particulier un cycle à 6 éléments.

5. Procédé selon la revendication 3 ou 4, dans lequel un composé de formule XI ou un sel de celui-ci 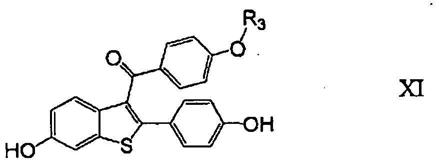 est préparé à partir du composé de formule X 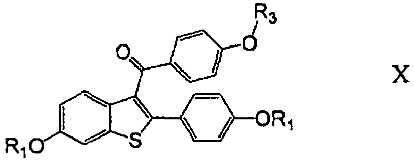 dans laquelle R₁ est choisi dans le groupe constitué par un alkyle linéaire ou ramifié, un arylalkyle linéaire ou ramifié et un alcényle linéaire ou ramifié, et
dans laquelle R₃ est 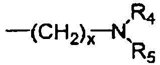 où x = 1 à 20, de préférence 1 à 15, plus préférentiellement 2 à 10, et en particulier 2, et R₄ et R₅, indépendamment l'un de l'autre, représentent un alkyle substitué ou non substitué, un aryle substitué ou non substitué, un arylalkyle substitué ou non substitué, ou R₄ et R₅ forment, coopérativement avec l'atome de N, une partie d'un cycle de 5 à 10 éléments, de préférence un cycle de 5 à 7 éléments, plus préférentiellement un cycle de 5 ou 6 éléments, en particulier un cycle à 6 éléments,
par déprotection des groupes -OR₁.

6. Composé de formule VII" 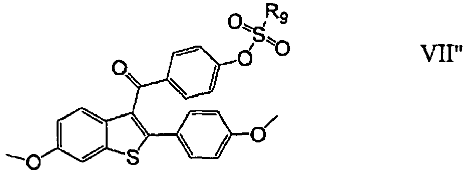 dans laquelle R₉ est un groupe méthyle, para-toluène ou phényle.

7. Procédé de préparation du raloxifène ou d'une composition pharmaceutique contenant du raloxifène de formule 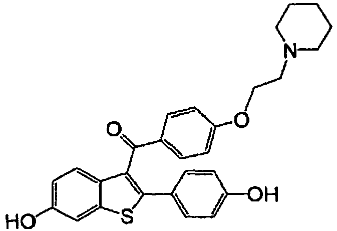 ou un sel pharmaceutiquement acceptable de celui-ci,
comprenant les étapes consistant à :
a) fournir un composé de formule VII' par un procédé selon la revendication 1,
b) convertir ledit composé de formule VII' en composé de formule VIII par un procédé selon la revendication 2,
c) convertir ledit composé de formule VIII en composé de formule X ou éventuellement un sel de celui-ci, dans laquelle R₃ est 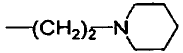
par un procédé selon la revendication 4, et
d) convertir ledit composé de formule X ou son sel en composé de formule XI selon la revendication 5 afin d'obtenir du raloxifène ou un sel pharmaceutiquement acceptable de celui-ci,
e) éventuellement mélanger ledit raloxifène ou un sel pharmaceutiquement acceptable de celui-ci avec au moins un excipient pharmaceutiquement acceptable.

8. Utilisation de composés de formule VII" tels que définis dans la revendication 6 dans un procédé de préparation du raloxifène ou d'un sel pharmaceutiquement acceptable de celui-ci.

9. Procédé de préparation d'une forme de solvate cristallin d'un sel d'un composé de formule XI 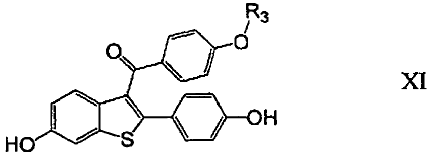 dans laquelle R₃ est 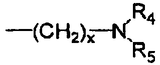 où x = 1 à 20,
et R₄ et R₅, indépendamment l'un de l'autre, représentent un alkyle substitué ou non substitué, un aryle substitué ou non substitué, un arylalkyle substitué ou non substitué, ou R₄ et R₅ forment, coopérativement avec l'atome de N, une partie d'un cycle de 5 à 10 éléments,
dans lequel un premier solvant et un second solvant différent dudit premier solvant sont incorporés dans ladite forme de solvate du sel du composé de formule XI à la condition qu'un solvate commun soit formé,
dans laquelle ledit procédé comprend les étapes consistant à :
a) réaliser un procédé selon la revendication 5, dans lequel une base libre ou un sel d'un composé de formule XI est fourni(e),
b) dissoudre ledit composé fourni à l'étape a) dans un mélange dudit premier solvant et dudit second solvant,
c) éventuellement distiller une partie du volume du mélange du premier solvant et du second solvant,
d) éventuellement ensemencer le mélange résultant de l'étape b) ou c) afin d'induire la cristallisation,
e) permettre à une forme de solvate d'un sel d'un composé de formule XI de se précipiter ou de se cristalliser, éventuellement sous agitation, et
f) séparer la forme de solvate cristallin d'un sel d'un composé de formule XI.

10. Procédé de préparation d'une forme de solvate cristallin d'un sel d'un composé de formule XI 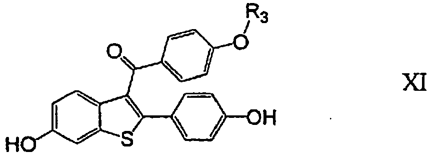 dans laquelle R₃ est tel que défini dans la revendication 9,
dans laquelle un premier solvant et un second solvant différent du premier solvant sont incorporés dans ladite forme de solvate du sel du composé de formule XI à la condition qu'un solvate commun soit formé,
dans laquelle ledit procédé comprend les étapes consistant à :
i) réaliser un procédé selon la revendication 3, dans laquelle, par l'intermédiaire dudit procédé, un sel ou une base libre d'un composé de formule X est fourni(e) 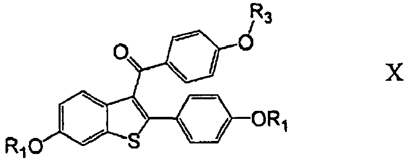 dans laquelle R₁ est choisi dans le groupe constitué par un alkyle linéaire ou ramifié, un arylalkyle linéaire ou ramifié et un alcényle linéaire ou ramifié, et
dans laquelle R₃ est 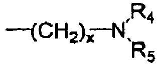 où x = 1 à 20,
et R₄ et R₅, indépendamment l'un de l'autre, représentent un alkyle substitué ou non substitué, un aryle substitué ou non substitué, un arylalkyle substitué ou non substitué, ou R₄ et R₅ forment, coopérativement avec l'atome de N, une partie d'un cycle de 5 à 10 éléments,
ii) déprotéger les groupes -OR₁ dudit sel d'un composé de formule X en présence d'un second solvant afin de convertir le composé de formule X en composé de formule XI,
iii) ajouter un premier solvant à un mélange de réaction résultant de l'étape ii),
iv) chauffer le mélange de réaction résultant de l'étape iii),
v) refroidir et éventuellement agiter le mélange de réaction résultant de l'étape iv), et
vi) séparer la forme de solvate d'un sel d'un composé de formule XI.

11. Procédé selon la revendication 10, dans laquelle, à l'étape i), un composé de formule X' est fourni 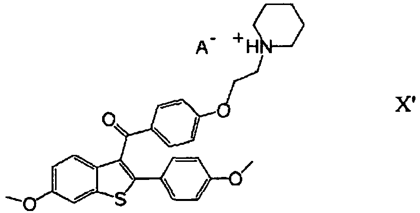 dans laquelle A⁻ est Cl⁻ ou Br⁻.

12. Procédé selon la revendication 11, dans laquelle le composé de formule X' est fourni sous une forme cristalline,
de préférence un composé de formule X', dans laquelle A⁻ est Cl⁻ et dont le diagramme de diffraction aux rayons X (XRD) a au moins des signaux aux valeurs 2θ suivantes (± 0,5° respectivement) :
| | | | | | | | | | | |
|---|---|---|---|---|---|---|---|---|---|---|
| **2thêta[°]** | 11,41 | 13,07 | 16,74 | 19,27 | 19,39 | 21,56 | 23,42 | 24 | 24,63 | 26,05 |
ou un composé de formule X', dans laquelle A⁻ est Br⁻ et dont le diagramme de diffraction aux rayons X (XRD) a au moins des signaux aux valeurs 2θ suivantes (± 0,5° respectivement) :
| | | | | | | | | | | |
|---|---|---|---|---|---|---|---|---|---|---|
| **2thêta[°]** | 7,41 | 13,05 | 16,64 | 19,07 | 19,22 | 21,45 | 23,31 | 24,34 | 25,81 | 26,62 |

13. Procédé selon l'une quelconque des revendications 9 à 12, dans laquelle le solvate cristallin est **caractérisé par** une ou plusieurs des caractéristiques suivantes (x) à (z) :
(x) le sel est dérivé d'un acide organique ou inorganique choisi dans le groupe constitué par l'acide oxalique, l'acide succinique, l'acide lactique, l'acide malonique, le HCl, le HBr et le H₂SO₄, plus préférentiellement le HCl, le HBr et le H₂SO₄, encore plus préférentiellement le HCl et le HBr et en particulier le HBr ;
(y) ledit premier solvant est incorporé dans le sel du composé de formule XI en une quantité en pourcentage en poids qui est inférieure à la quantité en pourcentage en poids du second solvant ; et/ou dans laquelle la forme du solvate cristallin d'un sel du composé de formule XI comprend ledit premier solvant en une quantité d'environ 0,1 à 8 pourcent en poids et ledit second solvant en une quantité d'environ 3 à 15 pourcent en poids par rapport à la forme solvate d'un sel du composé de formule XI ;
(z) ledit premier solvant est un alcool et ledit second solvant est un halocarbure, de préférence ledit premier solvant est un alcool en C₁-C₄ et ledit second solvant est un halocarbure en C₁ à C₁₀, comprenant éventuellement un cycle benzénique, plus préférentiellement ledit premier solvant est le méthanol ou l'éthanol et ledit second solvant est le chlorure de méthylène, le chloroforme ou le chlorobenzène, en particulier ledit premier solvant est le méthanol et ledit second solvant est le chlorure de méthylène.

14. Procédé de préparation d'un sel pharmaceutiquement acceptable non solvaté d'un composé de formule XI 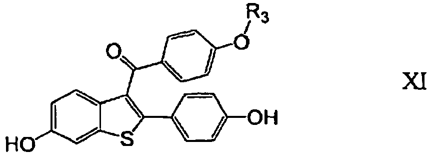 dans laquelle R₃ est 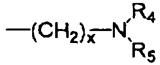 où x = 1 à 20,
et R₄ et R₅, indépendamment l'un de l'autre, représentent un alkyle substitué ou non substitué, un aryle substitué ou non substitué, un arylalkyle substitué ou non substitué, ou R₄ et R₅ forment, coopérativement avec l'atome de N, une partie d'un cycle de 5 à 10 éléments,
comprenant les étapes consistant à :
i) réaliser un procédé selon l'une quelconque des revendications 9 à 13, pour donner une forme de solvate cristallin d'un sel pharmaceutiquement acceptable d'un composé de formule XI
ii) dissoudre ladite forme de solvate cristallin fourni à l'étape i) dans un solvant organique, dans laquelle ledit solvant organique forme un azéotrope avec au moins un solvant incorporé dans ladite forme de solvate,
iii) éventuellement ensemencer le mélange résultant de l'étape ii) afin d'induire la cristallisation,
iv) permettre au sel pharmaceutiquement acceptable d'un composé de formule XI de se précipiter ou de se cristalliser, et
v) séparer le sel pharmaceutiquement acceptable d'un composé de formule XI.

15. Procédé selon la revendication 14, dans laquelle la forme de solvate cristallin est **caractérisée par** une ou plusieurs des caractéristiques suivantes (a) à (c) :
(a) ladite forme de solvate cristallin comprend un premier solvant et un second solvant différent du premier solvant, dans laquelle ledit premier solvant et ledit second solvant sont incorporés dans le sel pharmaceutiquement acceptable d'un composé de formule XI;
(b) ledit premier solvant est incorporé dans le sel du composé de formule XI en une quantité en pourcentage en poids qui est inférieure à la quantité en pourcentage en poids du second solvant ; et/ou dans laquelle la forme du solvate cristallin d'un sel du composé de formule XI comprend ledit premier solvant en une quantité d'environ 0,1 à 8 pourcent en poids et ledit second solvant en une quantité d'environ 3 à 15 pourcent en poids par rapport à la forme solvate d'un sel du composé de formule XI;
(c) ledit premier solvant est un alcool et ledit second solvant est un hydrocarbure ou halocarbure, de préférence ledit premier solvant organique est un alcool en C₁-C₄ et ledit second solvant organique est un hydrocarbure en C₆-C₁₀ comprenant une fraction benzénique ou un halocarbure en C₁-C₁₀, comprenant éventuellement une fraction benzénique, plus préférentiellement ledit premier solvant est le méthanol ou l'éthanol et ledit second solvant est le toluène, le chlorure de méthylène, le chloroforme ou le chlorobenzène, même plus préférentiellement ledit premier solvant est le méthanol et ledit second solvant est le toluène, le chlorure de méthylène ou le chloroforme, et en particulier, ledit premier solvant est le méthanol et ledit second solvant est le chlorure de méthylène.

16. Procédé selon la revendication 14 ou 15, **caractérisé par** l'une ou l'autre combinaison de caractéristiques de procédure suivantes (d) à (f) :
(d) l'azéotrope formé à l'étape ii) se forme sous pression normale ou réduite, de préférence sous pression réduite ;
(e) à l'étape ii), le mélange résultant est chauffé avant l'étape iii), de préférence le chauffage est réalisé dans des conditions de reflux ;
(f) le ou les solvants incorporés dans ladite forme de solvate d'un sel pharmaceutiquement acceptable d'un composé de formule XI est/sont retirés par distillation azéotrope du mélange résultant de l'étape ii), de préférence ladite distillation azéotrope est réalisée sous pression réduite.

17. Procédé selon l'une quelconque des revendications 14 à 16, **caractérisé par** l'une ou l'autre combinaison de caractéristiques de procédure suivantes (g) à (i) :
(g) ledit solvant organique de l'étape ii) est un alcool, de préférence un alcool miscible dans l'eau, plus préférentiellement un alcool en C₁-C₄, même plus préférentiellement le méthanol ou l'éthanol et en particulier le méthanol.
(h) ledit solvant organique de l'étape ii) comprend une quantité prédéterminée d'eau, de préférence ladite quantité d'eau est prédéterminée par un rapport volumique de l'eau et du solvant organique de 0 à 0,5, de préférence de 0,24 à 0,44, plus préférentiellement de 0,27 à 0,40 et en particulier de 0,29 à 0,38 ;
(i) ladite eau utilisée à l'étape ii) est de l'eau potable, de préférence de l'eau déminéralisée, plus préférentiellement de l'eau ayant subi une osmose inverse.

18. Procédé selon l'une quelconque des revendications 14 à 17, **caractérisé par** l'une ou l'autre combinaison de caractéristiques de procédure suivantes (j) et (k) :
(j) l'acide formant le sel pharmaceutiquement acceptable d'un composé de formule XI sur lequel est basée ladite forme de solvate est choisi dans le groupe constitué par les acides organiques ou les acides inorganiques, de préférence dans le groupe constitué par l'acide oxalique, l'acide succinique, l'acide lactique, l'acide malonique, le HCl, le HBr et le H₂SO₄, plus préférentiellement le HCl, le HBr et le H₂SO₄, encore plus préférentiellement le HCl et le HBr, et en particulier le HBr;
(k) ledit sel pharmaceutiquement acceptable d'un composé de formule XI obtenu à l'étape v) a une pureté > 98 %, mesurée par HPLC, plus préférentiellement > 99 %, même plus préférentiellement > 99,2 %, encore plus préférentiellement > 99,4 % et en particulier > 99,6 %.
